(19) Europäisches Patentamt
European Patent Office
Office européen des brevets

(11) EP 2 280 282 A1

(12) EUROPEAN PATENT APPLICATION

(43) Date of publication:
**02.02.2011 Bulletin 2011/05**

(21) Application number: **10185947.8**

(22) Date of filing: **03.02.2005**

(51) Int Cl.:
***G01N 33/53** (2006.01)*
***G01N 33/573** (2006.01)*
***C07K 16/00** (2006.01)*

(84) Designated Contracting States:
**AT BE BG CH CY CZ DE DK EE ES FI FR GB GR HU IE IS IT LI LT LU MC NL PL PT RO SE SI SK TR**

(30) Priority: **03.02.2004 US 541583 P**

(62) Document number(s) of the earlier application(s) in accordance with Art. 76 EPC:
**05712595.7 / 1 718 967**

(71) Applicant: **Diadexus, Inc.**
**South San Francisco, CA 94080 (US)**

(72) Inventors:
- **Wolfert, Robert**
  **Palo ALto, CA CA 94303 (US)**
- **Kim, Nam**
  **Santa Clara, CA CA 95054 (US)**
- **Duan, Xiaozhu**
  **Almeda, CA CA 94502 (US)**

(74) Representative: **Ablett, Graham Keith et al**
**Ablett & Stebbing**
**Caparo House**
**101-103 Baker Street**
**London, W1U 6FQ (GB)**

Remarks:
This application was filed on 01-10-2010 as a divisional application to the application mentioned under INID code 62.

(54) **Methods of detecting Lp-PLA2 activity**

(57) This invention relates to a method for measuring enzymatically active Lipoprotein-associated Phospholipase A2 (Lp-PLA2) in a sample. Further, this invention relates to a Hybrid Immunocapture method for measuring enzymatically active Lp-PLA2 in a sample. Specifically, this invention relates to a Hybrid Immunocapture method for measuring enzymatically active Lp-PLA2 in a sample utilizing an enzymatically active Lp-PLA2 standard. In addition, this invention relates to a kit for measuring enzymatically active Lp-PLA2 in a sample. Specifically, this invention relates to a kit for measuring enzymatically active Lp-PLA2 in a sample containing an enzymatically active Lp-PLA2 standard.

FIG. 1A and FIG. 1B
Hybrid ImmunoCapture (HIC) Assay

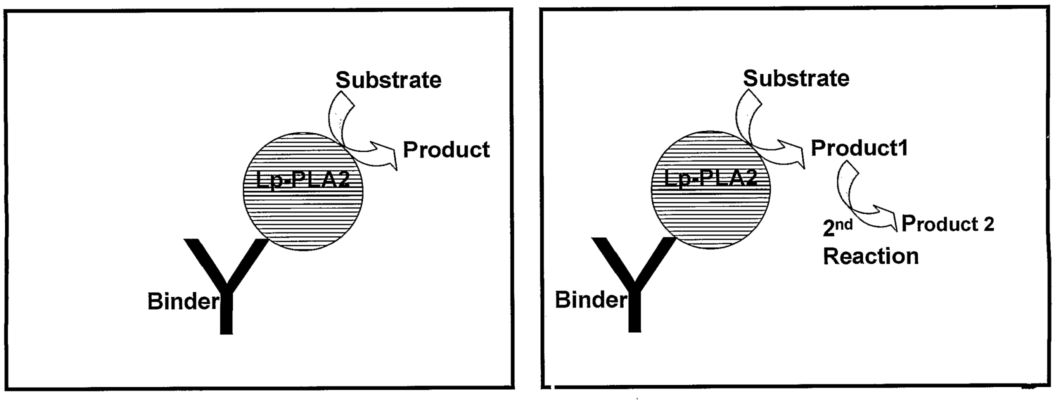


Fig. 1A

Fig. 1B

EP 2 280 282 A1

## Description

This patent application claims the benefit of priority from U.S. Provisional Patent Application Serial No. 60/541,583, filed February 3, 2004, which is herein incorporated by reference in its entirety.

## FIELD OF THE INVENTION

This invention relates to methods for determining the activity of Lipoprotein-associated Phospholipase A2 (Lp-PLA2). Specifically, it relates to determining the activity of Lp-PLA2 by use of Lp-PLA2-specific binders and/or substrates capable of being converted into a detectable product in various formats. Furthermore, this invention relates to a hybrid-immunocapture activity assay for specifically determining the activity of Lp-PLA2.

## BACKGROUND OF THE INVENTION

### Introduction

Lipoprotein-associated Phospholipase A2 (Lp-PLA2) is an enzymatically active 50 kD protein. Lp-PLA2 is a member of the phospholipase A2 family, and unlike most phospholipases, is $Ca^{2+}$ independent. Lp-PLA2 has been previously identified and characterized in the literature by Tew et al. (1996) Arterioscler. Thromb. Vasc. Biol. 16:591-599, Tjoelker, et al. (1995) Nature 374(6522):549-53), and Caslake et al. (2000) Atherosclerosis 150(2): 413-9. In addition, the protein and immunoassays have been described in the patent literature WO 95/00649-A1, U.S. Patents 5,981,252; 5,968,818; and 6,177,257 (to SmithKline Beecham) and WO 00/24910-A1, U.S. Patents 5,532,152; 5,605,801; 5,641,669; 5,656,431; 5,698,403; 5,977,308; and 5,847,088 (to ICOS Corporation) the contents of which are hereby incorporated by reference in their entirety. Lp-PLA2 is expressed by macrophages, with increased expression in atherosclerotic lesions (Hakkinin (1999) Arterioscler Thromb Vasc Biol 19(12): 2909-17). Lp-PLA2 circulates bound mainly to LDL, co-purifies with LDL, and is responsible for >95% of the phospholipase activity associated with LDL (Caslake 2000).

In recent studies, lipoprotein-associated phospholipase A2 (Lp-PLA2) levels have been shown to be significantly correlated in men with angiographically-proven Coronary Heart Disease (CHD) (Caslake 2000) and associated with cardiac events in men with hypercholesterolemia (Packard (2000) N Engl J Med 343(16): 1148-55).

Coronary heart disease (CHD) is the single most prevalent fatal disease in the United States. In the year 2003, an estimated 1.1 million Americans are predicted to have a new or recurrent coronary attack (see the American Heart Association web site, americanheart with the extension .org of the world wide web). Approximately 60% of these individuals have no previously known risk factors. It is apparent that there is a great need to diagnose individuals at risk of developing CHD, selecting patients suitable for therapy and monitor response to therapies directed and reducing the individual's risk.

Various methods for detecting Lp-PLA2 protein have been reported which include immunoassays (Caslake, 2000)., activity assays (PAF Acetylhydrolase Assay Kit, Cat#760901 product brochure, Cayman Chemical, Ann Arbor, MI, 12/18/97 (caymanchem with the extension. com of the world wide web); Azwell Auto PAF-AH kit available from the Nesco Company, Azwell Inc., 2-24-3 Sho, Ibaraki, Osaka, Japan or Karlan Chemicals, Cottonwood, Arizona, see also Kosaka (2000)), spectrophotometric assays for serum platelet activating factor acetylhydrolase activity (Clin Chem Acta 296: 151-161, WO 00/32808 (to Azwell)). Other published methods to detect Lp-PLA2 include WO 03/048172 (to SIRS-LAB) and WO 2005/001416 (to Glaxo). The contents of the published applications are hereby incorporated by reference in their entirety. None of these published papers or applications offer a method to measure both Lp-PLA2 mass and activity.

Recently, the United States Food and Drug Administration (FDA) has granted clearance for an ELISA test for the quantitative determination of Lp-PLA2 in human plasma to be used as a predictor of risk for coronary heart disease (CHD) ((2003) Sep-Oct; New test predicts heart risk. FDA Consum. 37(5):6.).

These assay formats have limitations. For example, assays which measure only enzymatic activity suffer from competitive activity for the substrate by other enzymes or substances present in the test sample. For instance, many members of the phospholipase A2 family show enzymatic activity toward oxidized phosphatidylcholine. Additionally, the Cayman activity assay suffers from background signal due to substances in serum which convert the substrate independent of Lp-PLA2 activity. Specifically, the Cayman kit relies on the detection of free thiol as part of the methodology. While the Cayman assay may work well in a laboratory setting, detecting free thiols makes the Cayman kit ill-suited for use to measure Lp-PLA2 (or PAF-AH) in human samples because of the abundant free thiols in human tissue, plasma or serum samples. In addition, existing assays may detect erroneously high activity due to the lack of specificity. False measurements of activity in a clinical setting may lead to improper diagnosis of disease, or a patient's response to a therapy intended to reduce enzymatic activity.

In contrast, standard antibody based immunoassays are highly specific and capable of detecting and quantifying the amount of a target of interest amongst other closely related proteins. However, they are not capable of determining

the level of enzymatic activity of the target. While this assay format ensures only the protein of interest is being measured, this limitation precludes such assays from being useful tools in monitoring a response to an enzyme inhibitor.

Accordingly, there is a great need for an assay capable of specifically selecting Lp-PLA2 from amongst other PLA2 family members which is further able to measure the enzymatic activity of Lp-PLA2.

## Coronary Heart Disease

Coronary vascular disease (CVD) encompasses all diseases of the vasculature, including high blood pressure, coronary heart disease (CHD), stroke, congenital cardiovascular defects and congestive heart failure. Studies have shown that CHD is responsible for the majority of the CVD. The prevalence of CHD increases markedly as a function of age, with men having a higher prevalence than women within most age groups.

The current standard of care used to identify individuals at risk for heart disease is the measurement of a lipid panel, including triglycerides, total cholesterol, low density lipoprotein (LDL)-cholesterol, and high density lipoprotein (HDL)-cholesterol (Adult Treatment Panel III). Executive Summary of The Third Report of The National Cholesterol Education Program (NCEP) Expert Panel on Detection, Evaluation, And Treatment of High Blood Cholesterol In Adults (Adult Treatment Panel III). JAMA (2001) 285(19): 2486-97. According to the recent Adult Treatment Panel III (ATP III) guidelines (2001), depending on the risk factor score, individuals with LDL-cholesterol levels from ≥100 to ≤130 mg/dL are recommended to initiate therapeutic lifestyle changes. Adults with LDL-cholesterol >130 mg/dL are recommended for intensive lifestyle therapy and an LDL-cholesterol-lowering drug therapy to achieve an LDL-cholesterol goal of <100 mg/dL. Patients with LDL levels >160 mg/dL should be considered for therapies with lipid-lowering drugs. The American Heart Association has estimated that over 100 million adults in the US exceed the optimal level of total cholesterol. See the website americanheart with the extension .org of the world wide web.

While research continues to link elevated LDL-cholesterol levels with CHD risk, it is well understood that a significant number of individuals with normal LDL-cholesterol levels experience a cardiac event, suggesting that other factors not currently recognized may be involved (Eaton (1998) J Am Board Fam Pract 11(3): 180-6). In the search for new risk factors, significant attention has been focused in recent years on markers of inflammation, as a growing body of basic and clinical research emerges regarding the role of inflammation in atherogenesis (Lusis (2000) Atherosclerosis. Nature 407(6801): 233-41; Lindahl (2000) N Engl J Med 343(16): 1139-47). Some of the inflammatory markers under investigation include cell adhesion molecules, CD-40 ligand, interleukin 6 and C-reactive protein (CRP, measured by the high sensitivity method, or hsCRP). CRP, a non-specific acute phase inflammatory marker, has recently received significant attention as a potential risk indicator for CHD (Ridker (2002) N Engl J Med 347(20): 1557-65; Blake (2002)); J Intern Med 252(4): 283-94). CRP, however, is well known to be responsive to many sources of inflammation, which justifies further investigations to identify more specific markers of arterial involvement.

The pathogenesis of atherosclerosis leading to the formation of unstable plaque has been recognized as one of the major causes of CHD (Lusis 2000). Recently, new understanding of the pathogenesis of atherosclerosis has placed emphasis on the inflammatory process as a key contributor to the formation of unstable plaque. The instability of the atherosclerotic plaque, rather than the degree of stenosis, is considered to be the primary culprit in the majority of myocardial infarctions (MI). This realization has led to the investigation of plaque biology and recognition that markers of inflammation may be useful as predictors of cardiovascular risk. Among the various candidate markers of inflammation, CRP (measured by high sensitivity method, hs-CRP), a non-specific acute phase inflammatory marker, has received the most attention as a predictor of CHD (Ridker 2002).

## The Molecular Basis for Disease

Oxidation of LDL in the endothelial space of the artery is considered a critical step in the development of atherosclerosis. Oxidized LDL, unlike native LDL, has been shown to be associated with a host of pro-inflammatory and pro-atherogenic activities, which can ultimately lead to atherosclerotic plaque formation (Glass (2001) Cell 104(4): 503-16; Witztum (1994) Lancet 344(8925): 793-5). Increasing evidence from basic research suggests that atherosclerosis has an inflammatory component and represents much more than simple accumulation of lipids in the vessel wall. The earliest manifestation of a lesion is the fatty streak, largely composed of lipid-laden macrophages known as foam cells. The precursors of these cells are circulating monocytes. The ensuing inflammatory response can further stimulate migration and proliferation of smooth muscle cells and monocytes to the site of injury, to form an intermediate lesion. As layers of macrophages and smooth muscle cells accumulate, a fibrous plaque is formed, which is characterized by a necrotic core composed of cellular debris, lipids, cholesterol, calcium salts and a fibrous cap of smooth muscle, collagen and proteoglycans. Gradual growth of this advanced lesion may eventually project into the arterial lumen, impeding the flow of blood. Further progression of atherosclerosis may lead to plaque rupture and subsequent thrombus formation, resulting in acute coronary syndromes such as unstable angina, MI or sudden ischemic death (Davies (2000) Heart 83:361-366; Libby (1996) Curr Opin Lipidol 7(5): 330-5).

Lp-PLA2 plays a key role in the process of atherogenesis by hydrolyzing the sn-2 fatty acid of oxidatively modified LDL, resulting in the formation of lysophosphatidylcholine and oxidized free fatty acids (Macphee (1999) Biochem J 338 (Pt 2): 479-87). Both of these oxidized phospholipid products of Lp-PLA2 action are thought to contribute to the development and progression of atherosclerosis, by their ability to attract monocytes and contribute to foam cell formation, among other pro-inflammatory actions (Macphee (2001) Curr Opin Pharmacol 1(2): 121-5; Macphee (2002) Expert Opin Ther Targets 6(3): 309-14).

**Clinical Studies**

Lp-PLA2 has been previously reported as a potential risk factor for CHD. The predictive value of plasma levels of Lp-PLA2 for CHD has been reported in a large, prospective case-control clinical trial involving 6,595 men with hypercholesterolemia, known as the West of Scotland Coronary Prevention Study (WOSCOPS) (Packard 2000). Lp-PLA2 was measured in 580 CHD cases (defined by non-fatal MI, death from CHD, or a revascularization procedure) and 1,160 matched controls. The results indicated that plasma levels of Lp-PLA2 were significantly associated with development of CHD events by univariate and multivariate analyses, with almost a doubling of the relative risk for CHD events for the highest quintile of Lp-PLA2 compared to the lowest quintile. The association of Lp-PLA2 with CHD was independent of traditional risk factors such as LDL-cholesterol and other variables. This study provided an encouraging preliminary indication of the clinical utility of Lp-PLA2 as a risk factor for CHD.

Furthermore, in a study of angiographically proven CHD, Lp-PLA2 was shown to be significantly associated with the extent of coronary stenosis (Caslake 2000).

In another study, in which only females were examined (n=246, 123 cases and 123 controls), baseline levels of Lp-PLA2 were higher among cases than controls (p=0.016), but was not significantly associated with CHD when adjusted for other cardiovascular risk factors. In this study, cases included 40% of women with stroke, 51 % non-fatal myocardial infarction and 9% fatal CHD (Blake (2001) J Am Coll Cardiol 38(5): 1302-6).

Recently, several large studies have added to the clinical evidence. For example, the Atherosclerosis Risk in Communities Study (ARIC) was designed to study, over a ten year period, the etiology, risk factors, clinical sequelae, and treatment alternatives for atherosclerosis. It was sponsored by the National Institutes of Health (NIH) and involved 15,792 apparently healthy men and women, aged 45 to 64, in four communities in the United States. In a retrospective study using banked samples, individuals with LDL <130 mg/dL but elevated levels of Lp-PLA2 (highest tertile) had a 2.08-fold higher risk of a coronary event compared to those individuals with low levels of Lp-PLA2 (Ballantyne (2004) Circulation. 109(7):837-42).

Recently, Monitoring Trends and Determinants in Cardiovascular Diseases Study (MONICA) was a World Health Organization project collecting data from 282,279 apparently healthy men from urban and rural areas in twenty-one countries. In a subsequent study using serum samples from a sub-population of the MONICA subjects, the association between Lp-PLA2 and coronary events was investigated. In this sub-study, 934 men, aged 45 to 64, were followed for 14 years. Mean baseline levels of Lp-PLA2 were significantly higher in the cases versus the non-cases ($p = 0.01$). A one standard deviation increase in Lp-PLA2 concentration as measured by an ELISA was associated in a univariate analysis with a relative risk of 1.37 ($p = 0.0002$), and the risk association remained statistically significant even after adjusting for other factors such as age, diabetes, smoking, blood pressure, lipid levels, BMI and CRP level (relative risk: 1.21; $p < 0.04$). In this study, individuals with the highest levels of both Lp-PLA2 and CRP had a 1.9-fold greater risk than individuals with low levels of both markers.

**Stroke and Peripheral Vascular Disease**

Stroke is a leading cause of death and disability in the industrialized world. There are approximately 700,000 strokes in the United States per year, of which 500,000 are strokes occurring in patients for the first time. These attacks are the cause of one in every fifteen deaths in the United States and leave a large number of survivors with disabilities (1.1 million in the U.S. in 1999). The total annual cost of stroke was estimated to be $53.6 billion in 2004 in the United States. Data presented from the Rotterdam Study - Oei et al (European Society of Cardiology in August 2004) and from the ARIC Study - Ballantyne et al. (Scientific Sessions of the American Heart Association (AHA) in November 2004) indicate Lp-PLA2 is an independent risk factor for stroke. In addition, the ARIC stroke study indicated that the measurement of both hsCRP and Lp-PLA2 was particularly useful for stroke risk assessment.

Peripheral vascular disease (PVD) is a nearly pandemic condition that has the potential to cause loss of limb, or even loss of life. PVD manifests as insufficient tissue perfusion caused by existing atherosclerosis that may be acutely compounded by either emboli or thrombi. Because of the connection between Lp-PLA2, atherosclerosis and vascular inflammation, measurement of Lp-PLA2 levels may be useful for detecting, diagnosing or monitoring PVD. Recently, Santos et al. reported studies of Lp-PLA2 and ankle-brachial index (ABI) a measure of peripheral vascular disease. They found Lp-PLA2 was a borderline-significant predictor of lower ABI ($p = 0.05$) whereas the other markers studied

CRP and white blood count (WBC) were not significant (Santos (2004) Vasc Med. 9(3):171-6).

**Additional Diseases**

Lp-PLA2 has been implicated in several other diseases including respiratory distress syndrome (Grissom (2003) Crit Care Med. 31 (3):770-5), immunoglobulin A nephropathy (Yoon (2002) Clin Genet. 62(2):128-34 ), graft patency of femoropopliteal bypass (LTnno (2002) Surgery 132(1):66-71), oral-inflammation (McManus and Pinckard (2000) Crit Rev Oral Biol Med. II (2):240-5 8), airway inflammation and hyperreactivity (Henderson (2000) J. Immunol. 15; 1 64(6): 3360-7), HIV and AIDS (Khovidhunkit (1999) Metabolism 48(12):1524-31), asthma (Satoh (1999) Am J Respir Crit Care Med. 159(3):974-9), juvenile rheumatoid arthritis (Tselepis (1999) Arthritis Rheum. 42(2):373-83), human middle ear effusions (Tsuji (1998) ORL J Otorhinolaryngol Relat Spec.60(1):25-9), schizophrenia (Bell (1997) Biochem Biophys Res Commun. 29;241(3):630-5 9), necrotizing enterocolitis development (Muguruma,(1997) Adv Exp Med Biol. 407:3 79-82), and ischemic bowel necrosis (Furukawa (1993) Pediatr Res. 34(2):237-41).

**Lp-PLA2 Inhibitors**

Furthermore, several papers have been published citing the potential of Lp-PLA2 as a therapeutic target for the treatment of coronary artery disease and atherosclerosis (Caslake 2000; Macphee 2001; Carpenter (2001) FEBS Lett. 505(3):357-63.; Leach (2001) Farmaco 56(1-2): 45-50). Evidence that Lp-PLA2 is a therapeutic target for the treatment of CHD has been published in many articles describing several genuses of inhibitors of Lp-PLA2 and their use. These genus include but are not limited to: azetidinone inhibitors, SB-222657, SB-223777 (MacPhee 1999); reversible 2-(alkylthio)-pyrimidin-4-ones (Boyd et al. (2000) Bioorg Med Chem Lett. 10(4):395-8); natural product derived inhibitors, SB-253514 and analogues (Pinto (2000); Bioorg Med Chem Lett. 10(17):2015-7); inhibitors produced by Pseudomonas fluorescens DSM 11579, SB-253514 and analogues (Thirkettle (2000) et al. J Antibiot (Tokyo). 53(7): 664-9; Busby (2000) J Antibiot (Tokyo). 53(7):670-6.; Thirkettle (2000) J Antibiot (Tokyo). 53(7):733-5); 2-(alkylthio)-pyrimidones, orally active 1-((amidolinked)-alkyl)-pyrimidones (Boyd et al. (2000) Bioorg Med Chem Lett. 10(22):2557-61); modified pyrimidone 5-substituent in 1-((amidolinked)-alkyl)-pyrimidones is highly water soluble (Boyd, et al. (2001) Bioorg Med Chem Lett. 2001 11(5):701-4); phenylpiperazineacetamide derivative of lipophilic 1-substituent in 1-((amidolinked)-alkyl)-pyrimidones (Bloomer (2001) Bioorg Med Chem Lett. 11(14):1925-9.); 5-(Pyrazolylmethyl) derivative and 5-(methoxypyrimidinylmethyl) derivative of 1-(biphenylmethylamidoalkyl)-pyrimidones (Boyd et al. (2002) Bioorg Med Chem Lett. 12(1):51-5); cyclopentyl fused derivative, SB-480848, of the pyrimidone 5-substituent in clinical candidate SB-435495 (Blackie (2003) Bioorg Med Chem Lett. 2003 Mar 24;13(6):1067-70). To date, GlaxoSmithKline (GSK) has announced positive clinical data for a novel compound, shown below, that dramatically lowers Lp-PLA2 activity. This Lp-PLA2 inhibitor may represent a new generation of drugs that reduce cardiovascular disease and death.

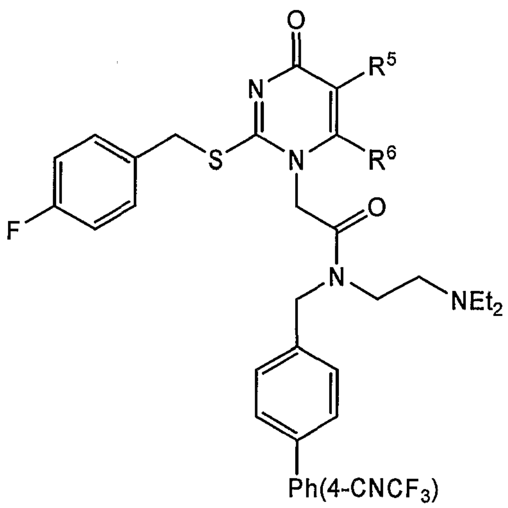

$R^5$ = e.g. alkyl, $R^5$ = H
or
$R^6$, $R^6$ = cycloalkyl

**Lp-PLA2 and Statins**

Winkler recently reported a multicenter, double-blind, randomized study evaluating the effects of fluvastatin XL versus placebo on the level of Lp-PLA2 in 89 patients with type 2 diabetes (42 fluvastatin and 47 placebo) (Winkler

(2004) J Clin Endocrinol Metab. 89(3) 1153-1159). Among these subjects, higher Lp-PLA2 activity was significantly associated with a history of CAD. The highest quartile in terms of Lp-PLA2 activity was at significantly greater risk than the lowest quartile (risk ratio: 2.09; 95% CI: 1.02 - 4.29; p = 0.043). Fluvastatin treatment decreased Lp-PLA2 activity by 22.8%. Blankenberg also reported that taking statins lowered the measurable Lp-PLA2 activity (Blankenberg (2003) J of Lipid Research 44: 1381-1386).

## SUMMARY OF THE INVENTION

One object of the present invention is to provide a method for determining lipoprotein-associated phospholipase A2 (Lp-PLA2) enzyme activity in a sample comprising the steps of contacting an immobilized binder, which specifically binds Lp-PLA2, with a sample; washing the immobilized binder to remove an enzymatically active unbound material or an interfering substance(s); contacting the bound Lp-PLA2 with a substrate converted to a detectable product in the presence of Lp-PLA2; and measuring detectable product indicative of enzymatically active Lp-PLA2 in the sample.

Another object of the present invention is to provide a kit for determining Lp-PLA2 enzyme activity in a sample comprising a binder immobilized to a solid support which specifically binds Lp-PLA2, a washing solution and a substrate converted to a detectable product in the presence of Lp-PLA2.

A further object of the present invention is to provide a method for determining Lp-PLA2 enzyme activity in a sample comprising the steps of contacting a binder, which specifically binds Lp-PLA2, with a sample to form a binder-Lp-PLA2 complex; immobilizing the binder-Lp-PLA2 complex; washing the immobilized binder-Lp-PLA2 complex to remove an enzymatically active unbound material or an interfering substance(s); contacting the immobilized bound Lp-PLA2 with a substrate converted to a detectable product in the presence of Lp-PLA2; and measuring detectable product indicative of enzymatically active Lp-PLA2 in the sample.

Another object of the present invention is to provide a kit for determining Lp-PLA2 enzyme activity in a sample comprising a binder which specifically binds Lp-PLA2, an immobilizing agent immobilized to a solid support, a washing solution and a substrate converted to a detectable product in the presence of Lp-PLA2.

An additional object of the present invention is to provide a method for determining lipoprotein-associated phospholipase A2 (Lp-PLA2) enzyme activity in a sample comprising the steps of incubating the sample with a compound which reduces active thiol(s) in the sample; contacting the incubated sample with a substrate converted to a free thiol product in the presence of enzymatically active Lp-PLA2; and measuring free thiol product indicative of enzymatically active Lp-PLA2 in the sample.

Another object of the present invention is to provide a kit for determining Lp-PLA2 enzyme activity in a sample comprising a compound which reduces active thiol(s) in a sample, and a substrate converted to a free thiol product in the presence of enzymatically active Lp-PLA2.

## BRIEF DESCRIPTION OF THE FIGURES

Figure 1A and Figure 1B display schematics of the Hybrid ImmunoCapture Assay.
Figure 2 displays plasma Lp-PLA2 activity in HIC-ThioPAF Assay with 2c10 as the capturing mAb.
Figure 3 displays plasma Lp-PLA2 activity in HIC-ThioPAF Assay with B200.1 as the capturing mAb.
Figure 4 displays plasma Lp-PLA2 activity in HIC-ThioPAF Assay with B501.1 as the capturing mAb.
Figure 5 displays plasma Lp-PLA2 activity in HIC-MNP Assay with 2c10 as the capturing mAb.
Figure 6 displays plasma Lp-PLA2 activity in a commercial ThioPAF Assay.
Figure 7 displays plasma sample background in an improved ThioPAF Assay, with DTNB but without substrate added.
Figure 8 displays plasma Lp-PLA2 activity post incubation step in the improved ThioPAF Assay.

## DETAILED DESCRIPTION OF THE INVENTION

This invention is directed to a method for measuring enzymatically active Lipoprotein-associated Phospholipase A2 (Lp-PLA2) in a sample comprising contacting an immobilized binder, which specifically binds Lp-PLA2, with the sample; washing the immobilized binder to remove an enzymatically active unbound material or an interfering substance (s); contacting the bound Lp-PLA2 with a substrate converted to a detectable product in the presence of Lp-PLA2; and measuring detectable product indicative of enzymatically active Lp-PLA2 in the sample. In one aspect of the invention the sample is a serum sample, a plasma sample or an EDTA treated plasma sample. In another aspect of the invention the immobilized binder is an antibody. In preferred aspect of the invention the antibody is a monoclonal antibody, a phage display antibody, or a polyclonal antibody. In a highly preferred aspect of the invention the monoclonal antibody is 2C10, 4B4, B200, B501, 90D1E, 90E3A, 90E6C, 90G11D, or 90F2D. In another highly preferred embodiment the

monoclonal antibody is produced by hybridoma cell line 90G11D (ATCC HB 11724), 90F2D (ATCC HB 11725), or 143A (ATCC HB 11900), see U.S. Patent 5,847,088, the contents of which are hereby incorporated by reference. Antibodies which bind Lp-PLA2 (or PAF-AH) are commercially available from sources such as Abcam, Inc. (Cambridge, MA) and AXXORA, LLC (San Diego, CA) and comprise another embodiment of this invention.

In another aspect of the invention the enzymatically active unbound material is a phospholipase. In a further aspect of the invention the interfering substance(s) is a free-thiol compound. In yet another aspect of the invention the substrate is selected from the group consisting of

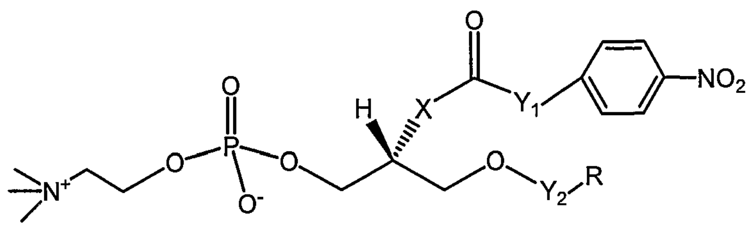

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

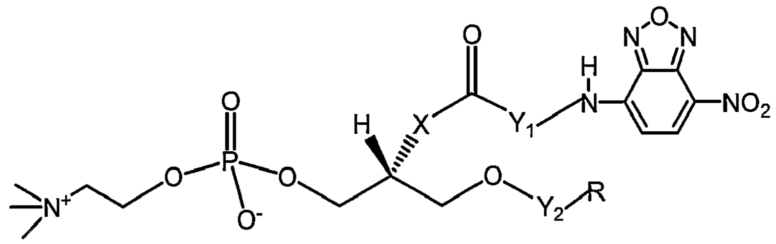

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

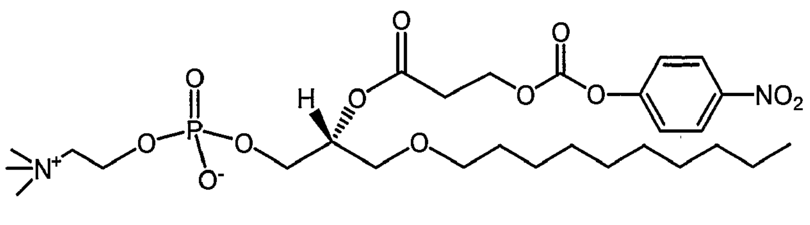

(c)

1-myristoyl-2-(4-nitrophenylsuccinyl) phosphatidylcholine (MNP);

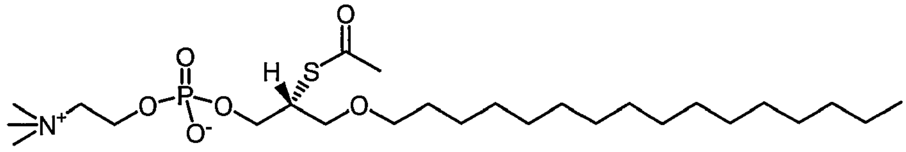

(d)

.

2-thio PAF; and

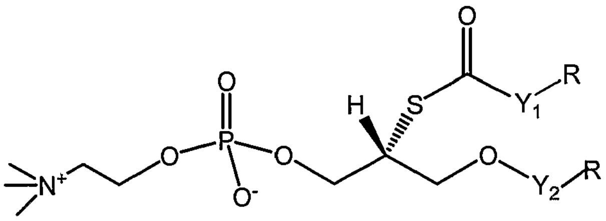

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

In a further aspect of the invention the substrate is an oxidized derivative of (a), (b), (c), (d) or (e) above.

In another aspect of the present invention the detectable product has a radioactive, colorimetric, paramagnetic or fluorescent label. Further, the detectable product is measured fluorimetrically, colorimetrically, paramagnetically or via radiation.

A further aspect of the invention comprises comparing the measured detectable product to detectable product in a control comprising an enzymatically active Lp-PLA2 standard. In a further aspect of the invention the enzymatically active Lp-PLA2 standard is a recombinant Lp-PLA2 protein or a native Lp-PLA2 protein. In yet a further aspect of the invention the recombinant Lp-PLA2 protein is expressed in a baculovirus expression system or a mammalian expression system. In another aspect of the present invention the immobilized binder is bound to a multi-well plate, a magnetic bead, or a latex bead.

This invention is also directed to a method for measuring enzymatically active Lp-PLA2 in a sample comprising: contacting a binder, which specifically binds Lp-PLA2, with the sample to form a binder-Lp-PLA2 complex; immobilizing the binder-Lp-PLA2 complex; washing the immobilized binder-Lp-PLA2 complex to remove any enzymatically active unbound material or any interfering substance(s); contacting the immobilized bound Lp-PLA2 with a substrate converted to a detectable product in the presence of Lp-PLA2; and measuring detectable product indicative of enzymatically active Lp-PLA2 in the sample. In one aspect of the invention the sample is a serum sample, a plasma sample or an EDTA treated plasma sample. In another aspect of the invention the binder is an antibody. In preferred aspect of the invention the antibody is a monoclonal antibody, a phage display antibody, or a polyclonal antibody. In a highly preferred aspect of the invention the monoclonal antibody is 2C10, 4B4, B200, B501, 90D1E, 90E3A, 90E6C, 90G11D, or 90F2D. In another highly preferred embodiment the monoclonal antibody is produced by hybridoma cell line 90G11D (ATCC HB 11724), 90F2D (ATCC HB 11725), or 143A (ATCC HB 11900), see US Patent 5,847,088 , the contents of which are hereby incorporated by reference. Antibodies which bind Lp-PLA2 (or PAF-AH) are commercially available from sources such as Abcam, Inc. (Cambridge, MA) and AXXORA, LLC (San Diego, CA) and comprise another embodiment of this invention.

In another aspect of the invention the binder-Lp-PLA2 complex is immobilized by binding to an immobilized compound. In a further aspect the immobilized compound is an antibody. In preferred aspect of the invention the antibody is a monoclonal antibody, a phage display antibody, or a polyclonal antibody. In a highly preferred aspect of the invention the monoclonal antibody, the phage display antibody, or the polyclonal antibody is a rat, mouse or goat anti-Ig antibody.

In another aspect of the invention the immobilized compound is bound to a multi-well plate, a magnetic bead,

or a latex bead.

In another aspect of the invention the binder is conjugated to an immobilizing agent. In a further aspect of the invention the binder conjugated to an immobilizing agent is an antibody. In preferred aspect of the invention the antibody is a monoclonal antibody, a phage display antibody, or a polyclonal antibody. In a highly preferred aspect of the invention the monoclonal antibody is 2C10, 4B4, B200, B501, 90D1E, 90E3A, 90E6C, 90G11D, or 90F2D. In another highly preferred embodiment the monoclonal antibody is produced by hybridoma cell line 90G11D (ATCC HB 11724), 90F2D (ATCC HB 11725), or 143A (ATCC HB 11900), see U.S. Patent 5,847,088 the contents of which are hereby incorporated by reference. Antibodies which bind Lp-PLA2 (or PAF-AH) are commercially available from sources such as Abcam, Inc. (Cambridge, MA) and AXXORA, LLC (San Diego, CA) and comprise another embodiment of this invention.

In another aspect of the invention the immobilizing agent is an antibody, protein or compound capable of binding an immobilized compound. In a preferred aspect of the invention the antibody is a monoclonal antibody, a phage display antibody, or a polyclonal antibody. In a highly preferred aspect of the invention the monoclonal antibody, the phage display antibody, or the polyclonal antibody is a rat, mouse or goat anti-Ig antibody. In another highly preferred aspect the immobilizing agent is biotin.

In a further aspect of the invention the immobilizing agent, conjugated to the binder-Lp-PLA2 complex, binds to an immobilized compound. In a preferred aspect the immobilized compound is bound to a multi-well plate, a magnetic bead, or a latex bead. In another aspect of the invention the bound compound is an antibody, protein or compound capable of binding the conjugated immobilizing agent. In preferred aspect of the invention the antibody is a monoclonal antibody, a phage display antibody, or a polyclonal antibody. In a highly preferred aspect of the invention the monoclonal antibody, the phage display antibody, or the polyclonal antibody is a rat, mouse or goat anti-Ig antibody. In another highly preferred aspect the immobilizing agent is streptavidin.

In another aspect of the invention the enzymatically active unbound material is a phospholipase. In another aspect of the invention the interfering substance(s) is a free-thiol compound. In yet another aspect of the invention the substrate is selected from the group consisting of

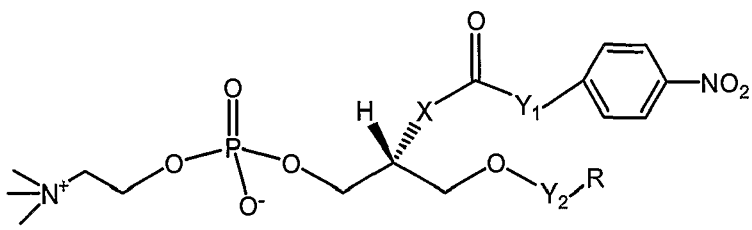

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

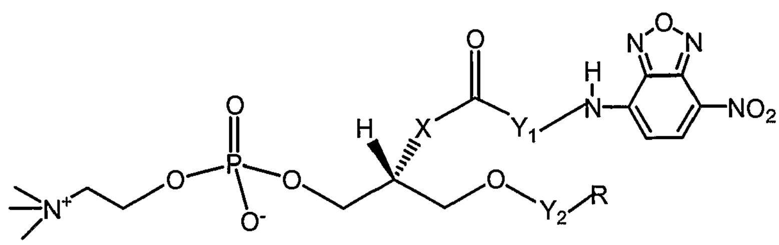

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH{=}CH(CH_2)_2CH_3$;

$Y_1$ is selected from the group consisting of $(CO)_{1-2}$ and $(CH_2)_{2-7}$; and
$Y_2$ is selected from the group consisting of CO and $CH_2$;

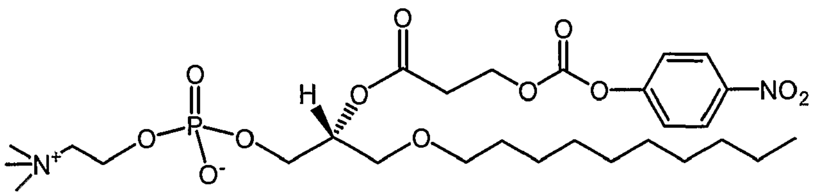

(c)

1-myristoyl-2-(4-nitrophenylsuccinyl) phosphatidylcholine (MNP);

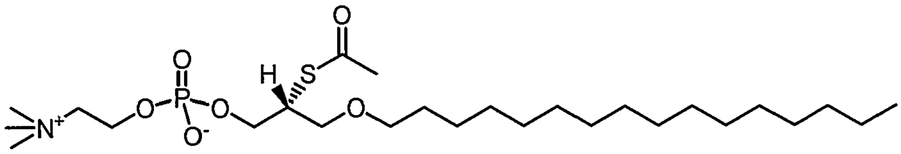

(d)

2-thio PAF; and

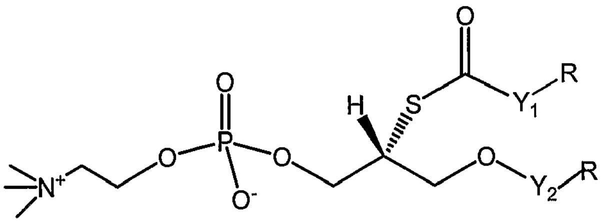

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

In a further aspect of the invention the substrate is an oxidized derivative of (a), (b), (c), (d) or (e) above.

In another aspect of the present invention the detectable product has a radioactive, colorimetric, paramagnetic or fluorescent label. Further, the detectable product is measured fluorimetrically, colorimetrically, paramagnetically or via radiation.

A further aspect of the invention comprises comparing the measured detectable product to detectable product in a control comprising an enzymatically active Lp-PLA2 standard. In a further aspect of the invention the enzymatically active Lp-PLA2 standard is a recombinant Lp-PLA2 protein or a native Lp-PLA2 protein. In yet a further aspect of the invention the recombinant Lp-PLA2 protein is expressed in a baculovirus expression system or a mammalian expression system. In another aspect of the present invention the immobilized binder is bound to a multi-well plate, a magnetic bead, or a latex bead.

The invention is also directed to a kit for measuring enzymatically active Lp-PLA2 in a sample comprising a binder which specifically binds Lp-PLA2 and a substrate converted to a detectable product in the presence of Lp-PLA2. In another aspect of the invention the substrate is selected from the group consisting of

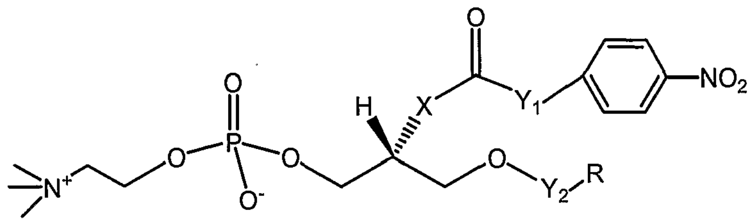

(a)

wherein,

- X is selected from the group consisting of O, S, and -O(CO)-;
- R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
- $Y_1$ is selected from the group consisting of $(CO)_{1-2}$ and $(CH_2)_{2-7}$; and
- $Y_2$ is selected from the group consisting of CO and $CH_2$;

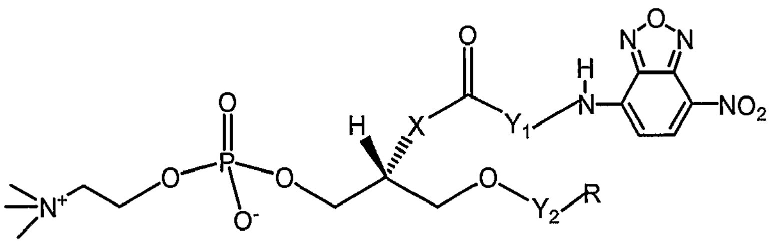

(b)

wherein,

- X is selected from the group consisting of O, S, and -O(CO)-;
- R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
- $Y_1$ is selected from the group consisting of $(CO)_{1-2}$ and $(CH_2)_{2-7}$; and
- $Y_2$ is selected from the group consisting of CO and $CH_2$;

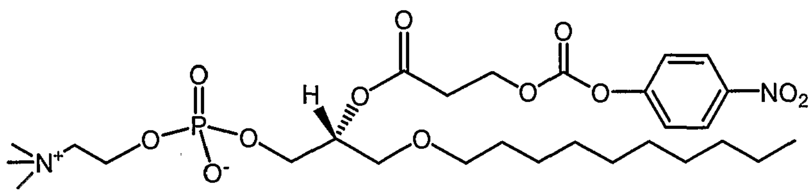

(c)

1-myristoyl-2-(4-nitrophenylsuccinyl) phosphatidylcholine (MNP);

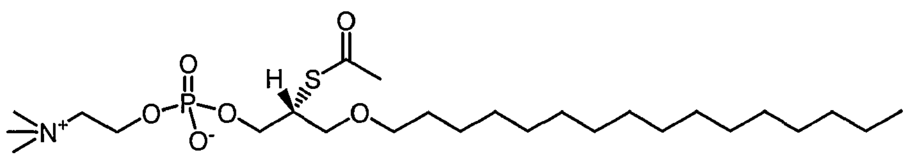

(d)

2-thio PAF; and

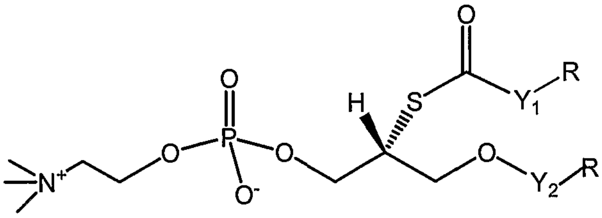

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

In a further aspect of the invention the substrate is an oxidized derivative of (a), (b), (c), (d) or (e) above.

Another aspect of the invention is a kit comprising an enzymatically active Lp-PLA2 standard. In a further aspect of the invention the enzymatically active Lp-PLA2 standard is a recombinant Lp-PLA2 protein or a native Lp-PLA2 protein. In yet a further aspect of the invention the recombinant Lp-PLA2 protein is expressed in a baculovirus expression system or a mammalian expression system.

The invention is also directed to a method for measuring enzymatically active Lp-PLA2 in a sample comprising: incubating the sample with a compound which reduces active thiol(s) in the sample; contacting the incubated sample with a substrate converted to a free thiol product in the presence of enzymatically active Lp-PLA2; and measuring free thiol product indicative of enzymatically active Lp-PLA2 in the sample.

In another aspect of the invention the sample is a serum sample, a plasma sample or an EDTA treated plasma sample. In another aspect of the invention compound which reduces active thiol(s) in the sample is DTNB.

In another aspect of the invention the sample is incubated at room temperature. In yet another aspect of the invention the sample is incubated at 37°C. In a further aspect the sample is incubated from about 2 to about 120 minutes. In another aspect the sample is incubated from about 5 to about 30 minutes.

In yet another aspect the substrate is selected from the group consisting of

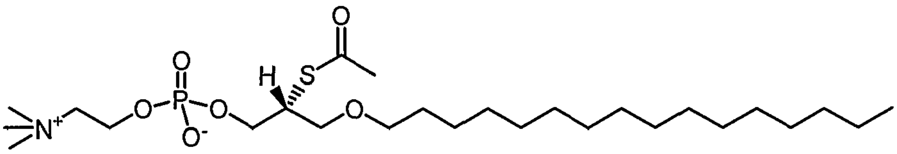

(a)

2-thio PAF; and

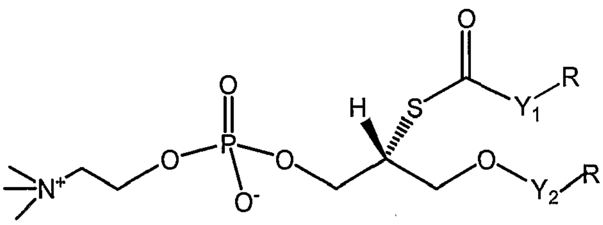

(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;

$Y_1$ is selected from the group consisting of $(CO)_{1-2}$ and $(CH_2)_{2-7}$; and
$Y_2$ is selected from the group consisting of CO and $CH_2$.

In another aspect of the invention the substrate is an oxidized derivative of (a) or (b).

The invention further comprises comparing measured free thiol product of step (c) to free thiol product in a control comprising an enzymatically active Lp-PLA2 standard. In a further aspect of the invention the enzymatically active Lp-PLA2 standard is a recombinant Lp-PLA2 protein or a native Lp-PLA2 protein. In yet a further aspect of the invention the recombinant Lp-PLA2 protein is expressed in a baculovirus expression system or a mammalian expression system. In a preferred aspect of the invention the method above is conducted in a multi-well plate.

The invention is also directed to a kit for measuring enzymatically active Lp-PLA2 in a sample comprising a compound which reduces active thiol(s) and a substrate converted to a detectable product in the presence of Lp-PLA2. In yet another aspect the substrate is selected from the group consisting of

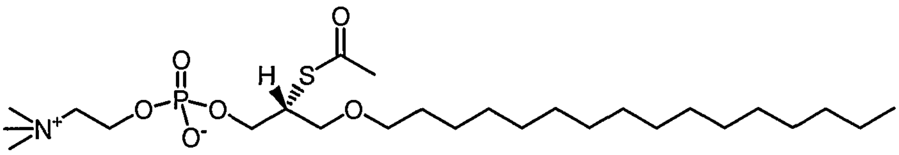

(a)

2-thio PAF; and

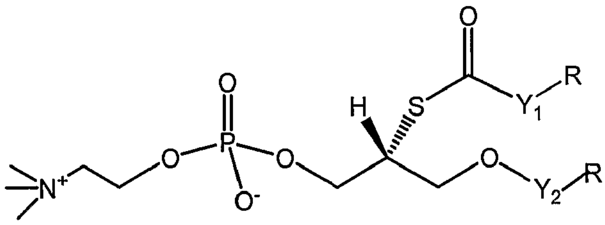

(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$.

In another aspect of the invention the substrate is an oxidized derivative of (a) or (b). In yet another aspect of the invention the kit contains an enzymatically active Lp-PLA2 standard. In a further aspect of the invention the enzymatically active Lp-PLA2 standard is a recombinant Lp-PLA2 protein or a native Lp-PLA2 protein. In yet a further aspect of the invention the recombinant Lp-PLA2 protein is expressed in a baculovirus expression system or a mammalian expression system.

Another aspect of the invention comprises the difference in detectable product in a sample compared to standard is due to a difference in Lp-PLA2 activity in the sample compared to the standard.

A further aspect of the invention comprises a method for detecting vascular disease in an individual comprising utilizing the methods described above to determine the individual's Lp-PLA2 activity in a sample wherein increased activity of Lp-PLA2 in the sample is indicative of vascular disease. In a preferred embodiment the vascular disease is selected from the group consisting of coronary vascular disease (CVD), coronary heart disease (CHD), peripheral vascular disease, peripheral arterial disease, high blood pressure, stroke, congenital cardiovascular defects and congestive heart failure.

Another aspect of the invention comprises a method for selecting an individual for therapy to treat vascular disease comprising utilizing the methods described above to determine the individual's Lp-PLA2 activity in a sample wherein increased activity of Lp-PLA2 in the sample is indicative of an individual who will benefit from therapy to treat vascular disease. In a preferred embodiment the vascular disease is selected from the group consisting of coronary vascular disease (CVD), coronary heart disease (CHD), peripheral vascular disease, peripheral arterial disease, high blood pressure, stroke, congenital cardiovascular defects and congestive heart failure. In another preferred embodiment the therapy is selected from the group consisting of statins and Lp-PLA2 inhibitors.

A further aspect of the invention comprises a method for monitoring an individual's response to therapy to treat vascular disease comprising utilizing the methods described above to determine the individual's Lp-PLA2 activity in a sample wherein decreased activity of Lp-PLA2 in the sample is indicative of an individual who is responding favorably to therapy to treat vascular disease. In a preferred embodiment the vascular disease is selected from the group consisting of coronary vascular disease (CVD), coronary heart disease (CHD), peripheral vascular disease, peripheral arterial disease, high blood pressure, stroke, congenital cardiovascular defects and congestive heart failure. In another preferred embodiment the therapy is selected from the group consisting of statins and Lp-PLA2 inhibitors.

One of ordinary skill in the art will readily recognize additional sources of antibodies exist for the practice of the invention herein. In a highly preferred embodiment a monoclonal antibody is produced by hybridoma cell line 90G11D (ATCC HB 11724), 90F2D (ATCC HB 11725), or 143A (ATCC HB 11900), see U.S. Patent 5,847,088 the contents of which are hereby incorporated by reference. Antibodies which bind Lp-PLA2 (or PAF-AH) are also commercially available from sources such as Abcam, Inc. (Cambridge, MA) and AXXORA, LLC (San Diego, CA)

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. *See, e.g.,* Sambrook et al., Molecular Cloning: A Laboratory Manual, 2d ed., Cold Spring Harbor Laboratory Press (1989) and Sambrook et al., Molecular Cloning: A Laboratory Manual, 3d ed., Cold Spring Harbor Press (2001); Ausubel et al., Current Protocols in Molecular Biology, Greene Publishing Associates (1992, and Supplements to 2000); Ausubel et al., Short Protocols in Molecular Biology: A Compendium of Methods from Current Protocols in Molecular Biology - 4th Ed., Wiley & Sons (1999); Harlow and Lane, Antibodies: A Laboratory Manual, Cold Spring Harbor Laboratory Press (1990); and Harlow and Lane, Using Antibodies: A Laboratory Manual, Cold Spring Harbor Laboratory Press (1999).

Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

An "antibody" as used herein refers to an intact immunoglobulin, or to an antigen-binding portion thereof that competes with the intact antibody for specific binding to a molecular species, *e.g.*, a polypeptide of the instant invention. Antigen-binding portions may be produced by recombinant DNA techniques or by enzymatic or chemical cleavage of intact antibodies. Antigen-binding portions include, *inter alia,* Fab, Fab', $F(ab')_2$, Fv, dAb, and complementarity determining region (CDR) fragments, single-chain antibodies (scFv), chimeric antibodies, diabodies and polypeptides that contain at least a portion of an immunoglobulin that is sufficient to confer specific antigen binding to the polypeptide. A Fab fragment is a monovalent fragment consisting of the VL, VH, CL and CH1 domains; a $F(ab')_2$ fragment is a bivalent fragment comprising two Fab fragments linked by a disulfide bridge at the hinge region; a Fd fragment consists of the VH and CH1 domains; a Fv fragment consists of the VL and VH domains of a single arm of an antibody; and a dAb fragment consists of a VH domain. *See, e.g.,* Ward et al., Nature 341: 544-546 (1989).

By "bind specifically" and "specific binding" as used herein it is meant the ability of the antibody to bind to a first molecular species in preference to binding to other molecular species with which the antibody and first molecular species are admixed. An antibody is said specifically to "recognize" a first molecular species when it can bind specifically to that first molecular species.

A single-chain antibody (scFv) is an antibody in which VL and VH regions are paired to form a monovalent molecule via a synthetic linker that enables them to be made as a single protein chain. *See, e.g.,* Bird et al., Science 242: 423-426 (1988); Huston et al., Proc. Natl. Acad. Sci. USA 85: 5879-5883 (1988). Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites. *See e.g.,* Holliger et al., Proc. Natl. Acad. Sci. USA 90: 6444-6448 (1993); Poljak et al., Structure 2: 1121-1123 (1994). One or more CDRs may be incorporated into a molecule either covalently or noncovalently to make it an immunoadhesin. An immunoadhesin may incorporate the CDR (s) as part of a larger polypeptide chain, may covalently link the CDR(s) to another polypeptide chain, or may incorporate the CDR(s) noncovalently. The CDRs permit the immunoadhesin to specifically bind to a particular antigen of interest. A chimeric antibody is an antibody that contains one or more regions from one antibody and one or more regions from one or more other antibodies.

An antibody may have one or more binding sites. If there is more than one binding site, the binding sites may

be identical to one another or may be different. For instance, a naturally occurring immunoglobulin has two identical binding sites, a single-chain antibody or Fab fragment has one binding site, while a "bispecific" or "bifunctional" antibody has two different binding sites.

An "isolated antibody" is an antibody that (1) is not associated with naturally-associated components, including other naturally-associated antibodies, that accompany it in its native state, (2) is free of other proteins from the same species, (3) is expressed by a cell from a different species, or (4) does not occur in nature. It is known that purified proteins, including purified antibodies, may be stabilized with non-naturally-associated components. The non-naturally-associated component may be a protein, such as albumin (*e.g.*, BSA) or a chemical such as polyethylene glycol (PEG).

A "neutralizing antibody" or "an inhibitory antibody" is an antibody that inhibits the activity of a polypeptide or blocks the binding of a polypeptide to a ligand that normally binds to it. An "activating antibody" is an antibody that increases the activity of a polypeptide.

The term "epitope" includes any protein determinant capable of specific binding to an immunoglobulin or T-cell receptor. Epitopic determinants usually consist of chemically active surface groupings of molecules such as amino acids or sugar side chains and usually have specific three-dimensional structural characteristics, as well as specific charge characteristics. An antibody is said to specifically bind an antigen when the dissociation constant is less than1 $\mu$M, preferably less than100 nM and most preferably less than 10 nM.

As is well known in the art, the degree to which an antibody can discriminate as among molecular species in a mixture will depend, in part, upon the conformational relatedness of the species in the mixture; typically, the antibodies of the present invention will discriminate over adventitious binding to Lp-PLA2 polypeptides by at least two-fold, more typically by at least 5-fold, typically by more than 10-fold, 25-fold, 50-fold, 75-fold, and often by more than 100-fold, and on occasion by more than 500-fold or 1000-fold.

Typically, the affinity or avidity of an antibody (or antibody multimer, as in the case of an IgM pentamer) of the present invention for a protein or protein fragment of the present invention will be at least about 1 x $10^{-6}$ molar (M), typically at least about 5 x $10^{-7}$ M, 1 x $10^{-7}$ M, with affinities and avidities of at least 1 x $10^{-8}$ M, 5 x $10^{-9}$ M, 1 x $10^{-10}$ M and up to 1 X $10^{-13}$ M proving especially useful.

The antibodies of the present invention can be naturally occurring forms, such as IgG, IgM, IgD, IgE, IgY, and IgA, from any avian, reptilian, or mammalian species.

IgG, IgM, IgD, IgE, IgY, and IgA antibodies of the present invention are also usefully obtained from other species, including mammals such as rodents (typically mouse, but also rat, guinea pig, and hamster), lagomorphs (typically rabbits), and also larger mammals, such as sheep, goats, cows, and horses; or egg laying birds or reptiles such as chickens or alligators. In such cases, as with the transgenic human-antibody-producing non-human mammals, fortuitous immunization is not required, and the non-human mammal is typically affirmatively immunized, according to standard immunization protocols, with the polypeptide of the present invention. One form of avian antibodies may be generated using techniques described in WO 00/29444, published 25 May 2000.

As discussed above, virtually all fragments of 8 or more contiguous amino acids of a polypeptide of the present invention can be used effectively as immunogens when conjugated to a carrier, typically a protein such as bovine thyroglobulin, keyhole limpet hemocyanin, or bovine serum albumin, conveniently using a bifunctional linker such as those described elsewhere above, which discussion is incorporated by reference here.

Immunogenicity can also be conferred by fusion of the polypeptide of the present invention to other moieties. For example, polypeptides of the present invention can be produced by solid phase synthesis on a branched polylysine core matrix; these multiple antigenic peptides (MAPs) provide high purity, increased avidity, accurate chemical definition and improved safety in vaccine development. Tam et al., Proc. Natl. Acad. Sci. USA 85: 5409-5413 (1988); Posnett et al., J. Biol. Chem. 263: 1719-1725 (1988).

Protocols for immunizing non-human mammals or avian species are well-established in the art. *See* Harlow et al. (eds.), Using Antibodies: A Laboratory Manual, Cold Spring Harbor Laboratory (1998); Coligan et al. (eds.), Current Protocols in Immunology, John Wiley & Sons, Inc. (2001); Zola, Monoclonal Antibodies: Preparation and Use of Monoclonal Antibodies and Engineered Antibody Derivatives (Basics: From Background to Bench), Springer Verlag (2000); Gross M, Speck J.Dtsch. Tierarztl. Wochenschr. 103: 417-422 (1996). Immunization protocols often include multiple immunizations, either with or without adjuvants such as Freund's complete adjuvant and Freund's incomplete adjuvant, and may include naked DNA immunization (Moss, Semin. Immunol. 2: 317-327 (1990).

Antibodies from non-human mammals and avian species can be polyclonal or monoclonal, with polyclonal antibodies having certain advantages in immunohistochemical detection of the polypeptides of the present invention and monoclonal antibodies having advantages in identifying and distinguishing particular epitopes of the polypeptides of the present invention. Antibodies from avian species may have particular advantage in detection of the polypeptides of the present invention, in human serum or tissues (Vikinge et al., Biosens. Bioelectron. 13: 1257-1262 (1998). Following immunization, the antibodies of the present invention can be obtained using any art-accepted technique. Such techniques are well known in the art and are described in detail in references such as Coligan, *supra*; Zola, *supra*; Howard et al. (eds.), Basic Methods in Antibody Production and Characterization, CRC Press (2000); Harlow, *supra*; Davis (ed.),

Monoclonal Antibody Protocols, Vol. 45, Humana Press (1995); Delves (ed.), Antibody Production: Essential Techniques, John Wiley & Son Ltd (1997); and Kenney, Antibody Solution: An Antibody Methods Manual, Chapman & Hall (1997).

Briefly, such techniques include, *inter alia,* production of monoclonal antibodies by hybridomas and expression of antibodies or fragments or derivatives thereof from host cells engineered to express immunoglobulin genes or fragments thereof. These two methods of production are not mutually exclusive: genes encoding antibodies specific for the polypeptides of the present invention can be cloned from hybridomas and thereafter expressed in other host cells. Nor need the two necessarily be performed together: *e.g.*, genes encoding antibodies specific for the polypeptides of the present invention can be cloned directly from B cells known to be specific for the desired protein, as further described in U.S. Patent No. 5,627,052, the disclosure of which is incorporated herein by reference in its entirety, or from antibody-displaying phage.

Recombinant expression in host cells is particularly useful when fragments or derivatives of the antibodies of the present invention are desired.

Host cells for recombinant antibody production of whole antibodies, antibody fragments, or antibody derivatives can be prokaryotic or eukaryotic.

Prokaryotic hosts are particularly useful for producing phage displayed antibodies of the present invention.

The technology of phage-displayed antibodies, in which antibody variable region fragments are fused, for example, to the gene III protein (pIII) or gene VIII protein (pVIII) for display on the surface of filamentous phage, such as M13, is by now well-established. *See, e.g.,* Sidhu, Curr. Opin. Biotechnol. 11(6): 610-6 (2000); Griffiths et al., Curr. Opin. Biotechnol. 9(1): 102-8 (1998); Hoogenboom et al., Immunotechnology, 4(1): 1-20 (1998); Rader et al., Current Opinion in Biotechnology 8: 503-508 (1997); Aujame et al., Human Antibodies 8: 155-168 (1997); Hoogenboom, Trends in Biotechnol. 15: 62-70 (1997); de Kruif *et al.,* 17: 453-455 (1996); Barbas et al., Trends in Biotechnol. 14: 230-234 (1996); Winter et al., Ann. Rev. Immunol. 433-455 (1994). Techniques and protocols required to generate, propagate, screen (pan), and use the antibody fragments from such libraries have recently been compiled. *See, e.g.,* Barbas (2001), *supra*; Kay, *supra*; and Abelson, *supra.*

Typically, phage-displayed antibody fragments are scFv fragments or Fab fragments; when desired, full length antibodies can be produced by cloning the variable regions from the displaying phage into a complete antibody and expressing the full length antibody in a further prokaryotic or a eukaryotic host cell. Eukaryotic cells are also useful for expression of the antibodies, antibody fragments, and antibody derivatives of the present invention. For example, antibody fragments of the present invention can be produced in *Pichia pastoris* and in *Saccharomyces cerevisiae. See, e.g.,* Takahashi et al., Biosci. Biotechnol. Biochem. 64(10): 2138-44 (2000); Freyre et al., J. Biotechnol. 76(2-3):1 57-63 (2000); Fischer et al., Biotechnol. Appl. Biochem. 30 (Pt 2): 117-20 (1999); Pennell et al., Res. Immunol. 149(6): 599-603 (1998); Eldin et al., J. Immunol. Methods. 201(1): 67-75 (1997); Frenken et al., Res. Immunol. 149(6): 589-99 (1998); and Shusta et al., Nature Biotechinol. 16(8): 773-7 (1998).

Antibodies, including antibody fragments and derivatives, of the present invention can also be produced in insect cells. *See, e.g.,* Li et al., Protein Expr. Purif. 21(1): 121-8 (2001); Ailor et al., Biotechnol. Bioeng. 58(2-3): 196-203 (1998); Hsu et al., Biotechnol. Prog. 13(1): 96-104 (1997); Edelman et al., Immunology 91(1): 13-9 (1997); and Nesbit et al., J. Immunol. Methods 151(1-2): 201-8 (1992).

Antibodies and fragments and derivatives thereof of the present invention can also be produced in plant cells, particularly maize or tobacco, Giddings et al., Nature Biotechnol. 18(11): 1151-5 (2000); Gavilondo et al., Biotechniques 29(1): 128-38 (2000); Fischer et al., J. Biol. Regul. Homeost. Agents 14(2): 83-92 (2000); Fischer et al., Biotechnol. Appl. Biochem. 30 (Pt 2): 113-6 (1999); Fischer et al., Biol. Chem. 380(7-8): 825-39 (1999); Russell, Curr. Top. Microbiol. Immunol. 240: 119-38 (1999); and Ma et al., Plant Physiol. 109(2): 341-6 (1995).

Antibodies, including antibody fragments and derivatives, of the present invention can also be produced in transgenic, non-human, mammalian milk. *See, e.g.* Pollock et al., J. Immunol Methods. 231: 147-57 (1999); Young et al., Res. Immunol. 149: 609-10 (1998); and Limonta et al., Immunotechnology 1: 107-13 (1995).

Mammalian cells useful for recombinant expression of antibodies, antibody fragments, and antibody derivatives of the present invention include CHO cells, COS cells, 293 cells, and myeloma cells. Verma et al., J. Immunol. Methods 216(1-2):165-81 (1998) review and compare bacterial, yeast, insect and mammalian expression systems for expression of antibodies. Antibodies of the present invention can also be prepared by cell free translation, as further described in Merk et al., J. Biochem. (Tokyo) 125(2): 328-33 (1999) and Ryabova et al., Nature Biotechnol. 15(1): 79-84 (1997), and in the milk of transgenic animals, as further described in Pollock et al., J. Immunol. Methods 231(1-2): 147-57 (1999).

The invention further provides antibody fragments that bind specifically to one or more of the polypeptides of the present invention, to one or more of the polypeptides encoded by the isolated nucleic acid molecules of the present invention, or the binding of which can be competitively inhibited by one or more of the polypeptides of the present invention or one or more of the polypeptides encoded by the isolated nucleic acid molecules of the present invention. Among such useful fragments are Fab, Fab', Fv, $F(ab)'_2$, and single chain Fv (scFv) fragments. Other useful fragments are described in Hudson, Curr. Opin. Biotechnol. 9(4): 395-402 (1998).

The present invention also relates to antibody derivatives that bind specifically to one or more of the polypeptides

of the present invention, to one or more of the polypeptides encoded by the isolated nucleic acid molecules of the present invention, or the binding of which can be competitively inhibited by one or more of the polypeptides of the present invention or one or more of the polypeptides encoded by the isolated nucleic acid molecules of the present invention.

Among such useful derivatives are chimeric, primatized, and humanized antibodies; such derivatives are less immunogenic in human beings, and thus are more suitable for *in vivo* administration, than are unmodified antibodies from non-human mammalian species. Another useful method is PEGylation to increase the serum half life of the antibodies.

Chimeric antibodies typically include heavy and/or light chain variable regions (including both CDR and framework residues) of immunoglobulins of one species, typically mouse, fused to constant regions of another species, typically human. *See, e.g.,* Morrison et al., Proc. Natl. Acad. Sci USA.81(21): 6851-5 (1984); Sharon et al., Nature 309(5966): 364-7 (1984); Takeda et al., Nature 314(6010): 452-4 (1985); and U.S. Patent No. 5,807,715 the disclosure of which is incorporated herein by reference in its entirety. Primatized and humanized antibodies typically include heavy and/or light chain CDRs from a murine antibody grafted into a non-human primate or human antibody V region framework, usually further comprising a human constant region, Riechmann et al., Nature 332(6162): 323-7 (1988); Co et al., Nature 351 (6326): 501-2 (1991); and U.S. Patent Nos. 6,054,297; 5,821,337; 5,770,196; 5,766,886; 5,821,123; 5,869,619; 6,180,377; 6,013,256; 5,693,761; and 6,180,370, the disclosures of which are incorporated herein by reference in their entireties. Other useful antibody derivatives of the invention include heteromeric antibody complexes and antibody fusions, such as diabodies (bispecific antibodies), single-chain diabodies, and intrabodies.

Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopyranoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Other substrates can be used to produce products for local deposition that are luminescent. For example, in the presence of hydrogen peroxide ($H_2O_2$), horseradish peroxidase (HRP) can catalyze the oxidation of cyclic diacylhydrazides, such as luminol. Immediately following the oxidation, the luminol is in an excited state (intermediate reaction product), which decays to the ground state by emitting light. Strong enhancement of the light emission is produced by enhancers, such as phenolic compounds. Advantages include high sensitivity, high resolution, and rapid detection without radioactivity and requiring only small amounts of antibody. *See, e.g.,* Thorpe et al., Methods Enzymol. 133: 331-53 (1986); Kricka et al., J. Immunoassay 17(1): 67-83 (1996); and Lundqvist et al., J. Biolumin. Chemilumin. 10(6): 353-9 (1995). Kits for such enhanced chemiluminescent detection (ECL) are available commercially. The antibodies can also be labeled using colloidal gold.

As another example, when the antibodies of the present invention are used, *e.g.*, for flow cytometric detection, for scanning laser cytometric detection, or for fluorescent immunoassay, they can usefully be labeled with fluorophores. There are a wide variety of fluorophore labels that can usefully be attached to the antibodies of the present invention. For flow cytometric applications, both for extracellular detection and for intracellular detection, common useful fluorophores can be fluorescein isothiocyanate (FITC), allophycocyanin (APC), R-phycoerythrin (PE), peridinin chlorophyll protein (PerCP), Texas Red, Cy3, Cy5, fluorescence resonance energy tandem fluorophores such as PerCP-Cy5.5, PE-Cy5, PE-Cy5.5, PE-Cy7, PE-Texas Red, and APC-Cy7.

Other fluorophores include, *inter alia,* Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3, Cy3.5, Cy5, Cy5.5, Cy7, all of which are also useful for fluorescently labeling the antibodies of the present invention. For secondary detection using labeled avidin, streptavidin, captavidin or neutravidin, the antibodies of the present invention can usefully be labeled with biotin.

When the antibodies of the present invention are used, *e.g.*, for western blotting applications, they can usefully be labeled with radioisotopes, such as $^{33}P$, $^{32}P$, $^{35}S$, $^{3}H$, and $^{125}I$. As another example, when the antibodies of the present invention are used for radioimmunotherapy, the label can usefully be $^{3}H$, $^{228}Th$, $^{227}Ac$, $^{225}Ac$, $^{223}Ra$, $^{213}Bi$, $^{212}Pb$, $^{212}Bi$, $^{211}At$, $^{203}Pb$, $^{194}Os$, $^{188}Re$, $^{186}Re$, $^{153}Sm$, $^{149}Tb$, $^{131}I$, $^{125}I$, $^{111}In$, $^{105}Rh$, $^{99m}Tc$ $^{97}Ru$, $^{90}Y$, $^{90}Sr$, $^{88}Y$, $^{72}Se$, $^{67}Cu$, or $^{47}Sc$.

The antibodies and compounds described above are useful in diagnostic assays to detect the presence of Lp-PLA2 in a sample.

In a preferred embodiment, a radioimmunoassay (RIA) or an ELISA is used. An antibody specific to Lp-PLA2

is prepared if one is not already available. In a preferred embodiment, the antibody is a monoclonal antibody. The anti-Lp-PLA2 antibody is bound to a solid support and any free protein binding sites on the solid support are blocked with a protein such as bovine serum albumin. A sample of interest is incubated with the antibody on the solid support under conditions in which the Lp-PLA2 will bind to the anti- Lp-PLA2 antibody. The sample is removed, the solid support is washed to remove unbound material, and an anti-Lp-PLA2 antibody that is linked to a detectable reagent (a radioactive substance for RIA and an enzyme for ELISA) is added to the solid support and incubated under conditions in which binding of the Lp-PLA2 to the labeled antibody will occur. After binding, the unbound labeled antibody is removed by washing. For an ELISA, one or more substrates are added to produce a colored reaction product that is based upon the amount of an Lp-PLA2 in the sample. For an RIA, the solid support is counted for radioactive decay signals by any method known in the art. Quantitative results for both RIA and ELISA typically are obtained by reference to a standard curve.

Other methods to measure Lp-PLA2 levels are known in the art. For instance, a competition assay may be employed wherein an anti- Lp-PLA2 antibody is attached to a solid support and an allocated amount of a labeled Lp-PLA2 and a sample of interest are incubated with the solid support. The amount of labeled Lp-PLA2 attached to the solid support can be correlated to the quantity of Lp-PLA2 in the sample. These assays and variations therefore comprise a further embodiment of the present invention.

The methods described herein can further be utilized as prognostic assays to identify subjects having or at risk of developing a disease or disorder associated with increased or decreased expression levels of Lp-PLA2. The presence of higher (or lower) Lp-PLA2 levels as compared to normal human controls is diagnostic for the human patient being at risk for developing CVD. The effectiveness of therapeutic agents to decrease (or increase) expression or activity of Lp-PLA2 of the invention can also be monitored by analyzing levels of expression of the Lp-PLA2 in a human patient in clinical trials or in vitro screening assays such as in human cells. In this way, the gene expression pattern can serve as a marker, indicative of the physiological response of the human patient or cells, as the case may be, to the agent being tested.

The above tests can be carried out on samples derived from a variety of cells, bodily fluids and/or tissue extracts such as homogenates or solubilized tissue obtained from a patient. Tissue extracts are obtained routinely from tissue biopsy and autopsy material. Bodily fluids useful in the present invention include blood, urine, saliva or any other bodily secretion or derivative thereof. As used herein "blood" includes whole blood, plasma, serum, circulating epithelial cells, constituents, or any derivative of blood.

In addition to detection in bodily fluids, the proteins and nucleic acids of the invention are suitable to detection by cell capture technology. Whole cells may be captured by a variety of methods for example magnetic separation, U.S. Patents 5,200,084; 5,186,827; 5,108,933; 4,925,788, the disclosures of which are incorporated herein by reference in their entireties. Epithelial cells may be captured using such products as Dynabeads® or CELLection™ (Dynal Biotech, Oslo, Norway). Alternatively, fractions of blood may be captured, e.g., the buffy coat fraction (50mm cells isolated from 5ml of blood) containing epithelial cells. Cells may also be captured using the techniques described in WO 00/47998, the disclosure of which is incorporated herein by reference in its entirety. Once the cells are captured or concentrated, the proteins or nucleic acids are detected by the means described in the subject application. Alternatively, nucleic acids may be captured directly from blood samples, see U.S. Patents 6,156,504, 5,501,963; or WO 01/42504, the disclosures of which are incorporated herein by reference in their entireties.

## EXAMPLES

### Example 1: Lp-PLA2 Hybrid ImmunoCapture (HIC) Assays

EGTA, NaCl, HEPES, Ellman's reagent; 5,5'-Dithio-bis(2-nitrobenzoic acid) (DTNB), Tris-HCL were obtained from Sigma (St. Louis, MO). Bovine serum albumin was obtained from GIBCO-Invitrogen (Carlsbad, CA). Microtiter plates were obtained from VWR (West Chester, PA). TBS and SuperBlock/TBS Blocking Solution were obtained from Pierce (Rockford, IL). Citric acid monohydrate buffer was obtained from Teknova (Half Moon Bay, CA). 1-myristoyl-2-(4-nitrophenylsuccinyl) phosphatidylcholine (MNP) was obtained from KARLAN (Santa Rosa, CA) and 2-Thio-PAF was obtained from Cayman Chemical (Ann Arbor, MI). Enzymatically active recombinant mammalian Lp-PLA2 (rmLp-PLA2) was generated at diaDexus (South San Francisco, CA). 200 normal blood plasma samples were used for analysis.

Hybrid ImmunoCapture Lp-PLA2 Activity Assays

A schematic of the Hybrid ImmunoCapture Assay (HIC) is shown in FIG. 1A and FIG. 1B. FIG. 1A shows one embodiment with a direct readout of substrate being converted to product. FIG. 1B shows a secondary readout where the product from the first reaction reacts to form a second product. The HIC-ThioPAF assay is an example of the FIG. 1B embodiment.

Lp-PLA2 HIC assay using 2-thio PAF substrate

Prior to the assay, ethanolic solution of 2-thio PAF was evaporated to dryness under a gentle stream of nitrogen, and reconstituted in 1x Assay Buffer (0.1 M Tris-HCl, pH 7.2, 1mM EGTA) to a final concentration of 400uM. 10 mM DTNB solution was prepared with 0.4M Tri-HCl, pH 7.2. R2 Buffer containing 1-myristoyl-2-(4-nitrophenylsuccinyl) phosphatidylcholine was made by mixing 20mM citric acid monohydrate buffer, pH4.5, and 90mM 1-myristoyl-2-(4-nitrophenylsuccinyl) phosphatidylcholine) in the proportion of 79:1 before assay.

First, microtiter plate was coated with 200 uL of anti Lp-$PLA_2$ mAb 2C10 (5 μg/ml) in 1XTBS and incubated at 4°C, overnight. Then the plate was blocked with 250 μL of SuperBlock TBS Blocking Solution, incubated with shaking at 180rpm at room temperature for 20 min. The plate was washed prior to use.

First, 170 μL of 1xTBS pH 7.4 buffer was added to a well followed by 30μL of sample, or standard (mammalian recombinant Lp-$PLA_2$, lot at 0, 25, 50, 100, 200, 400 ng/ml), and incubated with shaking at room temperature for 1 hr. After the incubation, the plate was washed once with 360μL of Wash Buffer (1xTBS, 0.05% Tween20). Then 160μL of 1x Assay Buffer (0.1M Tris-HCl, pH 7.2, 1mM EGTA), 10μL of DTNB solution (10mM in 0.4M Tris-HCl, pH 7.2), and 55μL of 400 uM 2-thio PAF substrate solution were added to each well, and read at 414 nm in a plate reader (Molecular Device, Sunnyvale, CA) running in kinetic mode (one reading per min), with auto mix on at 37°C, for 5 min. The slope of the curve corresponding to $\Delta OD^{414}$/min was calculated for all standards and samples. The level of Lp-PLA2 activity in ng/mL was calculated from a standard curve of the slope of rLp-PLA2 standards. The results are shown in FIG. 2. FIG 3 shows the results of the HIC-ThioPAF assay with the antibody B200.1 as the capture antibody. FIG 4 shows the results of the HIC-ThioPAF assay with the antibody B501.1 as the capture antibody.

Lp-PLA2 HIC-DAZ Assay

A second HIC assay to analyze Lp-PLA2 activity was developed using 1-myristoyl-2-(4-nitrophenylsuccinyl) phosphatidylcholine (MNP) as a substrate. This assay is referred to herein as the Lp-PLA2 HIC-DAZ Assay.

*p-Nitrophenol Standard Curve*

To prepare methanol stock solutions 100, 75, 50, 25, 10 and 5 uL of 1 M p-Nitrophenol (Sigma, Cat. #1048-25g) was mixed with 900, 925, 950, 975 and 990 ul of methanol to prepare 100, 75, 50, 25, 10 and 5 nmol/uL methanol solutions, respectively. Working solutions for each standard were prepared by diluting 40 uL of stock solution into 960 uL of methanol (1:25 dilution). Stock solutions and working solution were stored at 4°C in dark. 25uL of p-Nitrophenol standard working solution was added to 8 replicates wells, and 25 uL of PBS was added to 8 blank control wells before addition of 110 uL of R2 (described above) to each well. The plate was mixed and read at absorbance at 405nm as described above.

A standard curve was generated by plotting average OD values (8 replicates) for the 7 standards vs. amount of p-Nitrophenol (0, 5, 10,25, 50, 75, and 100 nmol). The slope ($\Delta OD^{405}$/nmol) of the p-Nitrophenol standard curves were then calculated to determine Lp-PLA2 activity in nmol/min/mL using the following formula:

$$\text{Lp-PLA2 activity (nmol/min/mL)} = \text{slope of sample } (\Delta OD^{405}/\text{min}) \div \text{slope of standard curve } (\Delta OD^{405}/\text{nmol}) \div 0.025 \text{ mL.}$$

p-Nitrophenol standard curves may were run at regular intervals to as calibration QC of the plate reader.

*Lp-PLA2 HIC-DAZ Assay*

A half-area 96-well microtiter plate (VWR, Cat. #3690) was coated with primary antibody (anti-Lp-PLA2 mAb 2C10) at 25 μL/well (1 ug/25 uL) in 1xTBS (Pierce, Cat. #28372) at 4°C, overnight The coating buffer was aspirated without washing prior to addition of 150 μL/well of SuperBlock TBS Blocking Solution from Pierce (Rockford, IL) (Cat. #37535). The plate was covered and incubated with shaking at 180 rpm at room temperature twice for 10 minutes. Blocking solution was discarded by aspiration and the plate was used within 24 hours.

Fresh R2 buffer was prepared by mixing R1 (200 mM HEPES, 200 mM NaCl, 5 mM EDTA, 10 mM CHAPS, 10 mM sodium 1-nonanesulfonate, pH 7.6) and R2B (90 mM 1-myristoyl-2-(4-nitrophenylsuccinyl) phosphatidylcholine) in the proportion of 20 uL:0.12 uL (per well) before assay. Once R1 and R2B were mixed; the solution was stored at

room temperature in dark, and the mixture R2 was used within 2 hours.

20 μL of sample, or standard, or control was added to each well of a coated plate and incubated with shaking at 180 rpm at room temperature for 30 minutes. (The standards used were 0, 100, 250, 500, 1000 ng/mL of rmLp-PLA2 diluted in 1% gamma-globulin/lxPBS at pH 7.4. Control samples included 6 normal plasma samples with one spiked with rmLp-PLA2 protein.) Plates were washed twice with 75 uL 1xTBS with 0.05% Tween-20. Following washing, 20 uL of reaction buffer R2 (described above) was added per well. Immediately put plate into plate reader and start reading. Addition of R2 to a whole plate must be finished within 40 seconds (multi-channel pipette and robot were used in our assay).

After the addition of R2, the change in absorption was immediately measured using a plate reader in kinetic mode at 405 nm for 5 minutes. The plate reader was set at room temperature, with auto mix set to mix once before read, and data points were obtained at 15 second intervals. The slope of the curve, corresponding to ΔmOD405/min, was calculated for all standards and samples from 15 seconds to 60 seconds. The level of Lp-PLA2 activity in ng/mL was calculated from a standard curve of the slope of rLp-PLA2 standards and the level Lp-PLA2 activity in nmol/min/mL was calculated from the slope:

$$\text{nmol/min/mL} = \text{slope } (\Delta\text{mOD}^{405}/\text{min}) \times 1.136.$$

*Lp-PLA2 HIC-DAZ AssayResults*

Lower Limit of Quantitation (LLOQ) for the assay was determined by calculating average and standard deviation (STDEV) of background signals from 22 replicates of blanks, using the following formula:

$$\text{Lower Limit of Quantitation (LLOQ)} = \text{Background} + (6 \times \text{STDEV Background})$$

| | nmol/min/mL |
|---|---|
| Range of Background signal | 0 - 0.432 |
| Average Background signal | 0.151 |
| STDEV Background signal | 0.107 |
| 6 x STDEV | 0.642 |
| Lower Limit of Quantitation: 0.793 nmol/min/mL | |

Upper Limit of Quantitation was estimated from the highest concentration of rLp-PLA2 Standard that can be reliably measured. Multiple replicates of the standards were evaluated to confirm that the highest standard can reproducibly quantifiable.

| rLp-PLA2 (ng/mL) | Slope ($\Delta$mOD$^{405}$/min) | nmol/min/mL |
|---|---|---|
| 0 | 0.0 | 0.0 |
| 0.5 | 0.0 | 0.0 |
| 1 | 0.0 | 0.0 |
| 5 | 0.7 | 0.8 |
| 10 | 1.3 | 1.6 |
| 25 | 2.7 | 3.3 |
| 50 | 5.3 | 6.5 |
| 100 | 10.7 | 13.2 |
| 250 | 26.0 | 32.0 |
| 500 | 41.9 | 51.6 |

(continued)

| rLp-PLA2 (ng/mL) | Slope ($\Delta mOD^{405}/min$) | nmol/min/mL |
|---|---|---|
| 1000 | 65.1 | 80.1 |

Quantiles for activity levels in commercially available samples are shown in the table below.

| | | nmol/min/mL |
|---|---|---|
| 100.0% | maximum | 49.052 |
| 99.5% | | 48.966 |
| 97.5% | | 26.345 |
| 90.0% | | 18.839 |
| 75.0% | quartile | 15.418 |
| 50.0% | median | 13.136 |
| 25.0% | quartile | 10.184 |
| 10.0% | | 8.143 |
| 2.5% | | 5.616 |
| 0.5% | | 3.231 |
| 0.0% | minimum | 3.223 |

The table below displays the target (or expected) LLOQ and ULOQ compared to the actual LLOQ and ULOQ for the Percentile and corresponding Quantile from the in the samples tested. The target LLOQ and ULOQ were generated using the methods described above and the actual LLOQ and ULOQ were determined by the evaluation of the samples.

| Percentile | Quantile (nmol/min/mL) | Target (nmol/min/mL) | Actual (nmol/min/mL) |
|---|---|---|---|
| 0.0500 | 7.0 | *LLOQ: 0.7 | LLOQ: 0.8 |
| 0.9500 | 22.9 | **ULOQ: 68.7 | ULOQ: 80.1 |
| *LLOQ = 5th percentile ÷ 10<br>**ULOQ = 95th percentile x 3 | | | |

The Actual LLOQ achieved was very close to the target LLOQ (1/10 x $5^{th}$ percentile of Normal Range Samples values) for this Lp-PLA2 HIC assay. The target sensitivity will be reached with minor modification. The ULOQ (3 x $95^{th}$ percentile of Normal Range Sample values) was successfully achieved for the Lp-PLA2 HIC-DAZ assay. In addition, extremely tight CV's were obtained for the upper range of the Lp-PLA2 HIC-DAZ assay utilizing the MNP substrate. These data demonstrate that the Lp-PLA2 HIC-DAZ assay is useful to accurately for measuring Lp-PLA2 activity.

**Example 2: Improved Lp-PLA2 ThioPAF Assay**

FIG. 6 shows the results of the commercially available ThioPAF assay, available from Cayman Chemicals (Ann Arbor, MI) following the manufacturer's protocol. Specifically, in that protocol, the DTNB is added concurrently with 2-thio PAF.

Prior to performing the improved assay, ethanolic solution of 2-thio PAF was evaporated to dryness under a gentle stream of nitrogen, and reconstituted in 1x Assay Buffer (0.1 M Tris-HCl, pH 7.2, 1mM EGTA) to a final concentration of 400μM. DTNB solution was prepared with 0.4M Tri-HCl, pH 7.2 to achieve a final concentration of 10mM (4mg DTNB in 1ml buffer).

<u>Lp-PLA2 activity assay using 2-thio PAF substrate</u>

In the assay, 83 μL of 1x Assay Buffer was mixed with 20 μL of sample, or standard (recombinant mammalian

Lp-PLA2 at 800, 400, 200, 100, 50, 25, and 0 ng/mL), and 10μL of the 10mM DTNB solution (in 0.4M Tris-HCl, pH 7.2), and incubated at room temperature for 15 min. FIG. 7 shows the results. After preincubation with DTNB to eliminate background signal from free thiol(s), 112μl of the 400μM 2-thio PAF solution was added and the plate was read at 414 nm in a plate reader (Molecular Device, Sunnyvale, CA) running in kinetic mode (one reading per min), with auto mix off at room temperature, for 5 min. The slope of the curve corresponding to $\Delta OD^{414}$/mmin was calculated for all standards and samples. The level of Lp-PLA2 activity in ng/mL was calculated from a standard curve of the slope of rLp-PLA2 standards. The results are shown in FIG. 8.

The table below shows the level of Lp-PLA2 activity in ng/mL for a set of 24 diverse plasma samples, determined twice per assay (Commercial Thio-PAF and Improved 2-thio PAF). In addition to the activity values, the Coefficient of Variations (CVs) is reported for each sample per assay and an overall average for each assay. Using the Improved 2-thio PAF assay improved the CVs from an average of 10.7% to 4.5%.

| Sample ID | Comm. Thio-PAF result #1 | Comm. Thio-PAF result #2 | CV Comm. Thio-PAF Assay | Improved Thio-PAF result #1 | Improved Thio-PAF result #2 | CV Improved Thio-PAF |
|---|---|---|---|---|---|---|
| 1 | 387.807 | 544.836 | 16.8% | 212.993 | 229.352 | 3.7% |
| 2 | 326.023 | 471.18 | 18.2% | 180.75 | 192.002 | 3.0% |
| 3 | 269.221 | 283.475 | 2.6% | 174.951 | 189.252 | 3.9% |
| 4 | 251.099 | 250.848 | 0.1% | 133.483 | 136.896 | 1.3% |
| 5 | 341.257 | 236.061 | 18.2% | 186.841 | 215.285 | 7.1% |
| 6 | 374.072 | 346.048 | 3.9% | 174.088 | 182.849 | 2.5% |
| 7 | 468.923 | 429.38 | 4.4% | 209.196 | 231.258 | 5.0% |
| 8 | 447.66 | 324.269 | 16.0% | 169.298 | 193.695 | 6.7% |
| 9 | 209.819 | 287.641 | 15.6% | 144.495 | 165.483 | 6.8% |
| 10 : | 373.44 | 385.316 | 1.6% | 180.249 | 216.328 | 9.1% |
| 11 : | 337.737 | 335.674 | 0.3% | 189.426 | 200.751 | 2.9% |
| 12 | 265.701 | 204.651 | 13.0% | 128.509 | 144.265 | 5.8% |
| 13 | 317.323 | 349.698 | 4.9% | 166.692 | 187.637 | 5.9% |
| 14 | 216.858 | 243.403 | 5.8% | 119.438 | 148.434 | 10.8% |
| 15 | 406.76 | 410.396 | 0.4% | 184.451 | 205.195 | 5.3% |
| 16 | 213.429 | 232.885 | 4.4% | 138.28 | 136.004 | 0.8% |
| 17 | 237.182 | 318.948 | 14.7% | 145.778 | 148.521 | 0.9% |
| 18 | 198.898 | 262.459 | 13.8% | 135.28 | 150.828 | 5.4% |
| 19 : | 254.131 | 383.769 | 20.3% | 153.296 | 176.586 | 7.1% |
| 20 | 213.483 | 329.673 | 21.4% | 109.556 | 119.442 | 4.3% |
| 21 | 238.446 | 342.15 | 17.9% | 150.295 | 172.425 | 6.9% |
| 22 | 225.685 | 275.164 | 9.9% | 158.967 | 164.756 | 1.8% |
| 23 | 246.947 | 357.185 | 18.2% | 137.172 | 136.212 | 0.4% |
| 24 | 228.32 | 300.263 | 13.6% | 153.578 | 155.559 | 0.6% |
| Ave. CV | | | 10.7% | | | 4.5% |

**Example 3: Lp-PLA2 DAZ Assay**

A colorimetric activity assay was developed to determine Lp-PLA2 activity utilizing 1-myristoyl-2-(4-nitrophenylsuccinyl) phosphatidylcholine (MNP) as a substrate which is herein referred to the Lp-PLA2 DAZ assay. This substrate has been used in commercially available assays such as the Auto-PAF-AH assay from Karlan Research Products

Corporation (Santa Rosa, CA). The Lp-PLA2 DAZ assay described below is useful for detecting Lp-PLA2 activity in a sample in addition to changes in Lp-PLA2 activity in a sample treated with an Lp-PLA2 inhibitor. Dilution of samples to perform analysis may increase Lp-PLA2 inhibitor disassociation from Lp-PLA2 in the sample resulting erroneously high Lp-PLA2 activity levels or low inhibition levels. The Lp-PLA2 DAZ assay reduces sample dilution and reports Lp-PLA2 activity or inhibition more accurately, which is useful in monitoring the ability or efficacy of a compound to inhibit Lp-PLA2 activity in a sample or a patient.

p-Nitrophenol Standard Curve

To prepare methanol stock solutions 100, 75, 50, 25, 10 and 5 uL of 1 M p-Nitrophenol (Sigma, Cat. #1048-25g) was mixed with 900, 925, 950, 975 and 990 ul of methanol to prepare 100, 75, 50, 25, 10 and 5 nmol/uL methanol solutions, respectively. Working solutions for each standard were prepared by diluting 40 uL of stock solution into 960 uL of methanol (1:25 dilution). Stock solutions and working solution were stored at 4°C in dark. 25uL of p-Nitrophenol standard working solution was added to 8 replicates wells, and 25 uL of PBS was added to 8 blank control wells before addition of 110 uL of R2 (described above) to each well. The plate was mixed and read at absorbance at 405nm as described above.

A standard curve was generated by plotting average OD values (8 replicates) for the 7 standards vs. amount of p-Nitrophenol (0, 5, 10, 25, 50, 75, and 100 nmol). The slope ($\Delta OD^{405}$/nmol) of the p-Nitrophenol standard curves were then calculated to determine Lp-PLA2 activity in nmol/min/mL using the following formula:

$$\text{Lp-PLA2 activity (nmol/min/mL)} = \text{slope of sample } (\Delta OD^{405}/\text{min}) \div \text{slope of standard curve } (\Delta OD^{405}/\text{nmol}) \div 0.025 \text{ mL.}$$

p-Nitrophenol standard curves may were run at regular intervals to as calibration QC of the plate reader.

DAZ Protocol

Fresh R2 buffer was prepared by mixing R1 (200 mM HEPES, 200 mM NaCl, 5 mM EDTA, 10 mM CHAPS, 10 mM sodium 1-nonanesulfonate, pH 7.6) and R2B (90 mM 1-myristoyl-2-(4-nitrophenylsuccinyl) phosphatidylcholine) in the proportion of 110 uL:0.66 uL (per well) before assay. Once R1 and R2B were mixed; the solution was stored at room temperature in dark, and the mixture R2 was used within 2 hours.

25 uL of sample, or standards and controls, were added into 96-well NBS plate (Coming, Cat. #3641), followed by 110 uL of R2 (mixture of R1 and R2B). Addition of R2 to a whole plate was completed within 40 seconds for optimal performance. Standards samples in the assay included 0, 100, 250, 500, 1000, 2000 ng/mL of mammalian recombinant Lp-PLA2 (rLp-PLA2) diluted in 1% gamma-globulin/lxPBS, pH 7.4. Control samples in the assay included 6 normal plasma samples, one of which was spiked with recombinant mammalian Lp-PLA2 (rmLp-PLA2).

After the addition of R2, the change in absorption was immediately measured using a plate reader in kinetic mode at 405 nm for 5 minutes. The plate reader was set at room temperature, with auto mix set to mix for 20 seconds once before read, and data points were obtained at 15 second intervals. The slope of the curve, corresponding to ΔmOD405/min, was calculated for all standards and samples from 60 seconds to 180 seconds. The level of Lp-PLA2 activity in ng/mL was calculated from a standard curve of the slope of rLp-PLA2 standards and the level Lp-PLA2 activity in nmol/min/mL was calculated from the slope:

$$\text{nmol/min/mL} = \text{slope } (\Delta mOD^{405}/\text{min}) \times 1.429.$$

**Example 4: Monitoring Lp-PLA2 Activity by HIC, Improved ThioPAF and DAZ Assays**

Plasma samples are collected from healthy volunteers at baseline and scheduled time points after dosing with either an Lp-PLA2 inhibitor or a placebo. Plasma samples are evaluated for Lp-PLA2 activity with the assays above.

Significant inhibition of Lp-PLA2 activity in Lp-PLA2 inhibitor treated volunteers is observed shortly after administration of inhibitor for several hours after administration of inhibitor. In contrast, no significant decrease in Lp-PLA2 activity is detected in volunteers who received placebo.

The relative inhibition of Lp-PLA2 activity is compared among the assay formats described above and elsewhere

including WO 2005/001416 which is incorporated by reference. The relative inhibition of Lp-PLA2 activity in samples is compared among assay formats. Each assay is used to determine the percent of total Lp-PLA2 activity at each time point post Lp-PLA2 inhibitor administration compared to baseline. Inter-assay variability is determined by calculating the arithmetic difference of the percent of inhibition of a given sample (time point), measured in a reference assay and an alternate assay format. The Lp-PLA2 DAZ, HIC, HIC-DAZ and Improved ThioPAF assay formats are useful for monitoring change in Lp-PLA2 activity in a volunteer over time. Furthermore, these assays are useful for monitoring a volunteer's response to a therapy which alters Lp-PLA2 activity such as statins and Lp-PLA2 inhibitors. By monitoring response to Lp-PLA2 activity modulating therapies, these assays are further useful for determining the benefit of a therapy to a volunteer.

**Example 5: Correlations and Distributions in Sample Populations**

We defined correlations among Lp-PLA2 mass, activity and combination mass/activity assays including the Auto PAF-AH assay (APAF) commercially available from Karlan (Santa Rosa, CA), the commercially available PLAC™ test for measuring Lp-PLA2 mass (PLAC), the DAZ assay (DAZ)described above, and the HIC-DAZ assay (HIC) described above. R-values were calculated for the 60 PromedDx (Norton, MA) normal sample population. Note: all R-values >0.75 have been highlighted as bold for ease of comparison.

R-values between assays analyzing 60 PromedDx* samples

| | PLAC | DAZ | HIC |
|---|---|---|---|
| APAF | 0.6933 | 0.9274 | 0.4679 |
| PLAC | | 0.6534 | 0.5807 |
| DAZ | | | 0.4122 |
| *Data from 5 samples taken out due to missing values | | | |

Depending on the patient sample population (range of Lp-PLA2 values and type of sample, correlations may vary dramatically among activity-based, mass-based, and hybrid activity/mass-based assay formats:

For the PromedDx normal samples the PLAC™ Test assay correlated well with the APAF, DAZ and HIC assays (R = 0.5-0.6). As expected, since the dynamic range of the Lp-PLA2 values was limited the R-values were attenuated.

Separation between clinical samples and PromedDx normal samples is best achieved with the PLAC™ test, followed by the Lp-PLA2 HIC-DAZ assay. The DAZ and APAF assays do not separate the population with high risk for CHD from normals as well as the PLAC™ test or HIC formats. The direct enzymatic formats, DAZ and APAF, worked equivalently compared to each other (R = 0.92).

Assays which are capable of separating individuals at risk of disease from those not at risk in a population are most suitable for patient risk assessment, in epidemiologic studies as well as in clinical patient diagnosis. The DAZ assay described above, and the HIC-DAZ assay (HIC) described demonstrated the highest correlation with the FDA cleared PLAC™ test assessing risk of atherosclerosis. Therefore, the DAZ and HIC-DAZ assays are suitable for assessing risk of atherosclerosis and other types of coronary vascular disease.

**Example 6: Assay Performance**

To assess Intra-assay, Inter-assay and Inter-operator variability of the Lp-PLA2 DAZ and HIC-DAZ assays described above 8 normal plasma samples were analyzed in quadruplicate, in 3 separate plates, by 3 operators.

Intra-assay Variability

The average %CV was calculated from %CVs of 8 plasma samples run in quadruplicate. The tables below show Average %CV from 3 runs, and the Average Intra-assay %CV from the 3 runs for each operator. Precision for the HIC assay was calculated for both nmol/min/mL and ng/mL values calculated from the standard curve of rLp-PLA2.

DAZ Assay

| Operator | Run 1 Ave %CV | Run 2 Ave %CV | Run 3 Ave %CV | Ave Intra-assay %CV |
|---|---|---|---|---|
| 1 | 2.4 | 3.0 | 2.8 | 2.7 |
| 2 | 3.8 | 1.8 | 4.1 | 3.2 |
| 3 | 1.8 | 1.5 | 1.8 | 1.7 |

HIC-DAZ (nmol/min/mL)

| Operator | Run 1 Ave %CV | Run 2 Ave | Run 3 Ave %CV | Ave Intra-assay %CV |
|---|---|---|---|---|
| 1 | 4.6 | 5.2 | 5.8 | 5.2 |
| 2 | 5.6 | 5.2 | 5.7 | 5.5 |
| 3 | 8.4 | 6.2 | 5.5 | 6.7 |

HIC-DAZ (ng/mL)

| Operator | Run 1 Ave %CV | Run 2 Ave %CV | Run 3 Ave %CV | Ave Intra-assay %CV |
|---|---|---|---|---|
| 1 | 4.8 | 6.0 | 6.8 | 5.9 |
| 2 | 5.8 | 5.8 | 5.8 | 5.8 |
| 3 | 8.4 | 6.3 | 5.6 | 6.8 |

Inter-assay Variability

The tables below show average inter-assay %CV from values for 8 plasma samples (P1-P8) analyzed on 3 independent assays. Average inter-assay %CV for each operator was calculated from the inter-assay %CVs of the 8 samples. Precision for HIC assay was calculated for both nmol/min/mL and ng/mL values calculated from the standard curve of rLp-PLA2.

DAZ Assay

| Operator | P1 | P2 | P3 | P4 | P5 | P6 | P7 | P8 | Average Inter-assay %CV |
|---|---|---|---|---|---|---|---|---|---|
| 1 | 1.9% | 2.1% | 2.0% | 0.8% | 2.6% | 1.3% | 2.2% | 0.4% | 1.7 |
| 2 | 3.8% | 2.2% | 3.9% | 1.0% | 2.8% | 1.6% | 3.8% | 0.3% | 2.4 |
| 3 | 1.0% | 1.3% | 1.3% | 2.5% | 1.8% | 2.8% | 2.0% | 2.0% | 1.8 |

HIC-DAZ (nmol/min/mL)

| Operator | P1 | P2 | P3 | P4 | P5 | P6 | P7 | P8 | Average Inter-assay %CV |
|---|---|---|---|---|---|---|---|---|---|
| 1 | 13.0% | 5.8% | 6.5% | 6.0% | 12.1% | 2.8% | 5.5% | 3.5% | 6.9 |
| 2 | 4.7% | 8.6% | 6.3% | 5.4% | 9.9% | 9.2% | 6.4% | 7.6% | 7.3 |
| 3 | 6.8% | 11.8% | 10.6% | 9.8% | 5.5% | 5.0% | 5.7% | 7.1% | 7.8 |

HIC-DAZ (ng/mL)

| Operator | P1 | P2 | P3 | P4 | P5 | P6 | P7 | P8 | Average Inter-assay %CV |
|---|---|---|---|---|---|---|---|---|---|
| 1 | 13.8% | 6.0% | 6.8% | 6.5% | 13.9% | 3.7% | 5.4% | 5.0% | 7.6 |
| 2 | 4.6% | 8.8% | 6.6% | 7.4% | 10.2% | 9.4% | 5.7% | 8.3% | 7.6 |

(continued)

| Operator | P1 | P2 | P3 | P4 | P5 | P6 | P7 | P8 | Average Inter-assay %CV |
|---|---|---|---|---|---|---|---|---|---|
| 3 | 8.6% | 13.6% | 12.6% | 11.5% | 6.1% | 6.4% | 7.2% | 8.7% | 9.3 |

Inter-operator Variability

Inter-operator %CV for 3 operators was calculated for each assay based on data from 8 normal plasma Promed-Dx samples analyzed in quadruplicate, in 3 separate plates. Precision for HIC-DAZ assay was calculated for both nmol/min/mL and ng/mL values calculated from the standard curve of rLp-PLA2.

DAZ Assay

| Sample | Operator1 Plate1 | Operator1 Plate2 | Operator1 Plate3 | Operator2 Plate1 | Operator2 Plate2 | Operator2 Plate3 | Operator3 Plate1 | Operator3 Plate2 | Operator3 Plate3 | Mean | SD | %CV |
|---|---|---|---|---|---|---|---|---|---|---|---|---|
| P1 | 36.95 | 37.31 | 35.94 | 39.76 | 37.77 | 40.66 | 32.47 | 31.87 | 31.97 | 36.08 | 3.3 | 9% |
| P2 | 53.73 | 56.04 | 54.86 | 57.17 | 55.23 | 57.49 | 46.86 | 47.73 | 46.51 | 52.85 | 4.5 | 9% |
| P3 | 59.44 | 60.06 | 57.81 | 65.08 | 60.46 | 61.33 | 50.68 | 51.99 | 51.28 | 57.57 | 5.1 | 9% |
| P4 | 59.60 | 58.67 | 59.18 | 59.92 | 59.98 | 61.03 | 48.49 | 50.93 | 49.26 | 56.34 | 5.2 | 9% |
| P8 | 70.60 | 70.67 | 67.50 | 73.71 | 69.71 | 72.21 | 58.17 | 60.25 | 58.68 | 66.83 | 6.1 | 9% |
| P9 | 67.43 | 69.23 | 68.54 | 72.73 | 70.46 | 71.09 | 56.28 | 59.52 | 57.61 | 65.88 | 6.3 | 10% |
| P10 | 67.19 | 70.15 | 68.35 | 74.60 | 69.82 | 74.66 | 58.00 | 60.31 | 58.80 | 66.88 | 6.4 | 10% |
| P7 | 67.56 | 67.17 | 67.03 | 72.20 | 71.89 | 72.32 | 56.64 | 58.88 | 57.40 | 65.68 | 6.4 | 10% |
| | | | | | | | | | | | | **9%** |

HIC-DAZ (nmol/min/mL)

| Sample | Operator1 | | | Operator2 | | | Operator3 | | | | | |
|---|---|---|---|---|---|---|---|---|---|---|---|---|
| | Plate1 | Plate2 | Plate3 | Plate1 | Plate2 | Plate2 | Plate1 | Plate2 | Plate3 | Mean | SD | %CV |
| P1 | 14.10 | 15.58 | 18.20 | 14.85 | 13.75 | 13.67 | 10.10 | 10.92 | 11.56 | 13.64 | 2.5 | 18% |
| P2 | 14.97 | 15.66 | 16.78 | 16.29 | 14.76 | 13.74 | 9.76 | 11.18 | 12.39 | 13.95 | 2.4 | 17% |
| P3 | 20.91 | 21.46 | 23.62 | 19.86 | 18.40 | 17.53 | 13.39 | 15.04 | 16.58 | 18.53 | 3.3 | 18% |
| P4 | 23.03 | 25.04 | 25.88 | 23.36 | 23.00 | 21.08 | 16.54 | 16.12 | 19.26 | 21.48 | 3.5 | 16% |
| P8 | 25.61 | 25.94 | 31.56 | 26.73 | 23.95 | 21.94 | 18.91 | 17.33 | 19.21 | 23.46 | 4.6 | 19% |
| P9 | 27.24 | 28.20 | 28.81 | 28.44 | 25.45 | 23.74 | 17.28 | 18.84 | 18.92 | 24.10 | 4.6 | 19% |
| P10 | 28.11 | 27.58 | 30.53 | 27.48 | 24.72 | 24.58 | 19.37 | 21.01 | 21.68 | 25.01 | 3.7 | 15% |
| P7 | 26.70 | 28.51 | 28.29 | 28.41 | 26.26 | 24.40 | 17.49 | 18.78 | 20.16 | 24.33 | 4.4 | 18% |
| | | | | | | | | | | | | 18% |

HIC-DAZ (ng/mL)

| | Operator1 | | | Operator2 | | | Operator3 | | | | | |
|---|---|---|---|---|---|---|---|---|---|---|---|---|
| Sample | Plate1 | Plate2 | Plate3 | Plate1 | Plate2 | Plate2 | Plate1 | Plate2 | Plate3 | Mean | SD | %CV |
| P1 | 96.246 | 100.153 | 123.51 | 110.24 | 102.83 | 101.169 | 94.544 | 104.161 | 112.439 | 105.03 | 9.0 | 9% |
| P2 | 102.225 | 100.745 | 112.241 | 121.43 | 111.39 | 101.786 | 91.799 | 106.169 | 120.61 | 107.60 | 9.7 | 9% |
| P3 | 146.667 | 149.685 | 166.256 | 149.27 | 143.195 | 131.091 | 125.766 | 144.153 | 162.195 | 146.48 | 12.9 | 9% |
| P4 | 162.593 | 180.252 | 184.132 | 176.44 | 183.381 | 158.737 | 156.734 | 154.758 | 188.78 | 171.76 | 13.4 | 8% |
| P8 | 182.037 | 187.92 | 233.08 | 202.65 | 191.655 | 165.434 | 179.96 | 166.653 | 188.293 | 188.63 | 20.4 | 11% |
| P9 | 194.352 | 207.248 | 207.254 | 215.94 | 204.764 | 179.401 | 163.992 | 181.532 | 185.366 | 193.32 | 16.9 | 9% |
| P10 | 200.833 | 201.891 | 220.855 | 208.48 | 198.424 | 185.906 | 184.556 | 202.823 | 212.805 | 201.84 | 11.7 | 6% |
| P7 | 190.278 | 209.874 | 203.174 | 215.75 | 211.855 | 184.566 | 166.048 | 180.927 | 197.683 | 195.57 | 16.5 | 8% |
| | | | | | | | | | | | | 9% |

These precision studies demonstrated acceptable reproducibility for both the Lp-PLA2 DAZ and HIC-DAZ assay formats for intra- and inter-assay %CV. Inter-operator precision for the DAZ assay was acceptable. For the HIC-DAZ format, inter-operator CV was high (18%), but is reducible by means of increased operator proficiency or applying a standard curve.

Linearity of Dilution

To determine linearity of dilution, 10 plasma samples from PromedDx were diluted to 50% concentration with 1% gamma-globulin, then tested in the Lp-PLA2 DAZ and HIC-DAZ assays. In addition, 5 pairs of plasma samples from PromedDx were mixed in 1:1 ratio, and tested in the DAZ and HIC assays.

*50% Dilution*

DAZ %recoverv (actual/expected)

| sample ID | 100% sample | 50% sample |
|---|---|---|
| 1 | 100% | 119% |
| 2 | 100% | 127% |
| 3 | 100% | 127% |
| 4 | 100% | 121% |
| 5 | 100% | 123% |
| 6 | 100% | 135% |
| 7 | 100% | 134% |
| 8 | 100% | 135% |
| 9 | 100% | 120% |
| 10 | 100% | 129% |
| Average | | 127% |

HIC-DAZ %recoverv (actual/expected)

| sample ID | 100% sample | 50% sample |
|---|---|---|
| 1 | 100% | 135% |
| 2 | 100% | 107% |
| 3 | 100% | 121% |
| 4 | 100% | 91% |
| 5 | 100% | 119% |
| 6 | 100% | 100% |
| 7 | 100% | 107% |
| 8 | 100% | 108% |
| 9 | 100% | 118% |
| 10 | 100% | 120% |
| Average | | 113% |
| *1:1 Ratio Mix* | | |

DAZ %recoverv (actual/expected)

| Sample | Expected nmol/min/mL | Actual nmol/min/mL | %recovery to expected |
|---|---|---|---|
| 2&7 | 72.64 | 73.73 | 101% |
| 3&8 | 79.04 | 79.51 | 101% |
| 4&9 | 74.65 | 76.22 | 102% |
| 5&10 | 80.36 | 80.85 | 101% |
| 6&11 | 86.01 | 86.28 | 100% |
| Average | | | 101% |

HIC-DAZ %recovery (actual/expected)

| Sample | Expected nmol/min/mL | Actual nmol/min/mL | %recovery to expected |
|---|---|---|---|
| 2+7 | 26.08 | 25.68 | 98% |
| 3+8 | 26.25 | 25.44 | 97% |
| 4+9 | 32.46 | 28.93 | 89% |
| 5+10 | 27.21 | 32.50 | 119% |
| 6+11 | 27.60 | 30.87 | 112% |
| Average | | | 103% |

In the DAZ assay, the recoverable Lp-PLA2 activity in 50% diluted plasma samples (in 1% gamma-globulin) ranged from 119% to 135%, with average recovery of 127%. When 5 pairs of plasma samples were mixed in 1:1 ratio, the Lp-PLA2 activity recovered were very close to the expected value, range from 100% to 102%. In the HIC-DAZ assay, the recoverable Lp-PLA2 activity in 50% diluted plasma samples (in 1% gamma-globulin) ranged from 91% to 135%, average recovery was 113%. When 5 pairs of plasma samples were mixed in 1:1 ratio, the Lp-PLA2 activity recovered ranged from 89% to 119%, the average was 103%. The observed over-recovery of 50% diluted samples in the DAZ and HIC-DAZ assays is being evaluated.

In summary, these assay formats - the Lp-PLA2 DAZ assay (using the MNP substrate), the Lp-PLA2 Improved ThioPAF assay (using the 2-ThiolPAF substrate), and the Lp-PLA2 hybrid immunocapture (HIC) assay (using the MNP or 2-ThiolPAFsubstrates for detection) - have been developed to detect Lp-PLA2 activity in a sample. All of these assays have been optimized to ensure minimal dissociation of an inhibitor from the Lp-PLA2 enzyme during the course of the assay. This was achieved in part by maximizing sample volumes, minimizing dilutions and minimizing incubations times. The assays are therefore useful for epidemiologic studies, risk assessment and diagnosis of diseases such as CHD.

**Example 7: Automation of Lp-PLA2 DAZ and HIC Assay Formats**

As shown above, we have developed several activity-based assay formats for Lp-PLA2: a modified version of the MNP substrate activity assay (DAZ), a modified version of the 2-thio PAF substrate activity assay (Improved ThioPAF), and a hybrid immunocapture (HIC) format that utilizes the anti Lp-PLA2 2C10 capture antibody from the PLAC test and the MNP substrate for detection (HIC-DAZ). Both the HIC-DAZ and DAZ assay formats have demonstrated correlation with the $^{3}$H-PAF radiometric method in determining Lp-PLA2 levels in plasma of subjects. We have additionally developed high-throughput automated DAZ and HIC-DAZ assays that can be used to reliably generate more than 1000 data points per day, per operator. Furthermore, we have tested Lp-PLA2 activity in matched plasma and serum samples and demonstrated good correlation of the recovered values between these two sample types.

**Descriptions of Automation Platforms**

<u>MultiPROBE</u>

The MultiPROBE II HT is a robotic liquid handling station manufactured by Perkin Elmer/Packard (Torrance,

CA). The instrument consists of eight pipettor probes that can function together, or independently from each other, a dedicated probe rinsing/washing mechanism, and a large deck that can accommodate 28 standard 96-well microtiter plates or pipette tip boxes. The deck can be equipped with an extra twister robotic arm, and a plate stacker or hotel to increase its throughput capacity (not available on the unit used herein). Due to the flexible pipetting options and built-in liquid sensing capability that can detect inaccurate aspirated fluid volumes or clogged tips, it is advantageous for transferring or aliquoting plasma or serum samples. This robotic station can transfer samples from tubes to 96-well plates. However, because the probes dispense samples and reagents 8 channels at a time, it does take longer for the MultiPROBE to dispense reagent to the whole 96-well plate, when compared to robotic stations equipped with a 96-well dispensing head (e.g., MiniTrak). Further description of the MultiPROBE robotic station can be found in the following website:

Internet address: las.perkinelmer.com/catalog/Product.aspx?ProductId=AMP8B00

MiniTrak

The MiniTrak is a robotic liquid handling station manufactured by Perkin Elmer/Packard (Torrance, CA). The instrument consist of a 96-well pipetting head that can dispense reagents and samples to all 96-wells at once, 4 stackers that can accommodate 12 pipette tip boxes or 50 standard 96-well microtiter plates, a conveyor belt that transports plates to and from different stations, and a small deck that has 9 positions for standard 96-well microtiter plates or pipette tip boxes. However, the 96-well pipetting head does not have a liquid sensing capability that can detect inaccurate aspirated fluid volumes or clogged tips, and thus, is less advantageous for transferring plasma or serum samples. Furthermore, the robot can transfer and/or dispense samples and reagents to and from 96-well formats, but cannot transfer samples to and from tubes. However, because the MiniTrak can dispense samples and reagents for the entire 96-well plate all at once, it does have a significant advantage when dispensing the MNP substrate in the DAZ or HIC assays, where the timing is important. Further description of the MiniTrak robotic station can be found in the following website:

Internet address: las.perkinelmer.com/content/TechnicalInfo/P10503-MinitrakSpecificationsSheet.pdf

**Lp-PLA2 DAZ Assay Automation**

For the automation of the DAZ assay described above, we have broken down the assay into 3 main tasks: Sample loading to 96-well assay plate, Substrate addition and Reading. Automation of each task was evaluated on MultiPROBE and MiniTrak robotic platforms.

Automated DAZ on MultiPROBE

For the automated sample transfer on the MultiPROBE platform, tubes were placed into the tube holder, or Stock Sample Plates were placed onto the deck (14 maximum). The sample transfer program on the MultiPROBE system was started which includes the following actions: Flush wash, Pick up tips, Pick up samples, Dispense samples into sample/assay plate (96-well), Discard tips and Repeat. Sample plates were labeled and stored at 4°C until use. For long-term storage, plate were sealed and stored at -80°C until use.

To perform the assay the samples/assay plates (28 maximum) were placed onto the MultiPROBE deck. 1.4 mL of R2 substrate was drawn into each probe without disposable tip attached, then 110 uL of R2 substrate was dispensed into all wells over about 40 seconds. The change in absorption was immediately measured using a plate reader in kinetic mode at 405 nm for 5 minutes after the addition of R2. The plate reader was set at room temperature, with auto mix set to mix for 20 seconds once before read, and data points were obtained at 15 second intervals. The protocol was repeated with subsequent plates once the reading of each previous plate was completed.

*MultiPROBE Lp-PLA2 DAZ Performance*

The reproducibility, accuracy, and potential plate effect of the MultiPROBE DAZ assay was tested using a single simulated plasma sample (calf serum spiked with rLp-PLA2) on two assay plates to generate 192 replicates of the sample (see table below). Following are the results of the analysis showing the mean, median, standard deviation, and %CV of each row and column of each plate.

Plate effect analysis of MultiPROBE DAZ assay (Plate 1)

| | | | | | | | | | | | | | **Mean** | Median | Std Dev | % CV |
|---|---|---|---|---|---|---|---|---|---|---|---|---|---|---|---|---|
| **Plate 1** | 110.86 | 111.21 | 109.95 | 84.71 | 108.10 | 108.35 | 109.48 | 109.42 | 110.33 | 110.27 | 111.22 | 110.29 | 107.85 | 110.11 | 7.35 | 6.82 |
| | 111.56 | 118.61 | 111.28 | 83.26 | 106.17 | 109.90 | 115.20 | 109.76 | 110.76 | 110.48 | 112.83 | 114.29 | 109.51 | 111.02 | 8.84 | 8.07 |
| | 108.51 | 110.00 | 109.86 | 87.40 | 107.86 | 107.58 | 108.40 | 108.50 | 109.04 | 109.02 | 111.02 | 110.74 | 107.33 | 108.77 | 6.37 | 5.93 |
| | 108.26 | 110.04 | 107.78 | 84.39 | 106.16 | 107.20 | 108.33 | 107.51 | 108.08 | 108.94 | 109.08 | 110.31 | 106.34 | 108.17 | 7.01 | 6.59 |
| | 108.49 | 110.24 | 108.71 | 85.18 | 105.93 | 108.25 | 107.01 | 107.74 | 108.74 | 109.83 | 109.98 | 110.76 | 106.74 | 108.60 | 6.93 | 6.49 |
| | 109.58 | 110.55 | 104.07 | 85.31 | 107.87 | 106.81 | 106.46 | 108.78 | 109.36 | 109.51 | 109.26 | 110.13 | 106.47 | 109.02 | 6.91 | 6.49 |
| | 109.15 | 111.03 | 110.63 | 84.18 | 108.38 | 108.21 | 108.38 | 109.27 | 109.20 | 109.54 | 110.42 | 111.01 | 107.45 | 109.23 | 7.40 | 6.88 |
| | 111.69 | 110.73 | 109.21 | 87.12 | 108.04 | 108.19 | 110.98 | 110.15 | 113.26 | 110.24 | 111.24 | 108.92 | 108.31 | 110.20 | 6.84 | 6.32 |
| | 109.76 | 111.55 | 108.94 | 85.19 | 107.31 | 108.06 | 109.28 | 108.89 | 109.85 | 109.73 | 110.63 | 110.81 | 107.50 | 109.50 | 7.12 | 6.62 |
| **Mean** | 109.76 | 111.55 | 108.94 | 85.19 | 107.31 | 108.06 | 109.28 | 108.89 | 109.85 | 109.73 | 110.63 | 110.81 | | | | |
| **Median** | 109.67 | 110.88 | 109.07 | 85.19 | 107.58 | 108.12 | 108.84 | 108.89 | 109.60 | 109.73 | 110.63 | 110.75 | | | | |
| **SD** | 1.32 | 2.70 | 2.10 | 1.33 | 0.97 | 0.87 | 2.59 | 0.88 | 1.51 | 0.54 | 1.14 | 1.45 | | | | |
| **% CV** | 1.21 | 2.42 | 1.93 | 1.56 | 0.90 | 0.81 | 2.37 | 0.81 | 1.38 | 0.49 | 1.03 | 1.30 | | | | |

Plate effect analysis of MultiPROBE DAZ assay (Plate 2)

| | | | | | | | | | | | | | Mean | Median | Std Dev | % CV |
|---|---|---|---|---|---|---|---|---|---|---|---|---|---|---|---|---|
| **Plate 2** | 101.04 | 102.43 | 103.02 | 102.85 | 83.74 | 102.71 | 99.97 | 93.14 | 101.68 | 100.47 | 105.41 | 104.19 | 100.05 | 102.06 | 5.98 | 5.97 |
| | 105.86 | 102.48 | 104.21 | 108.86 | 83.75 | 105.77 | 99.45 | 98.76 | 99.11 | 99.71 | 89.93 | 106.58 | 100.37 | 101.09 | 7.26 | 7.23 |
| | 95.82 | 101.05 | 102.21 | 104.75 | 83.43 | 103.16 | 102.01 | 95.47 | 98.56 | 101.24 | 101.16 | 102.37 | 99.27 | 101.20 | 5.72 | 5.76 |
| | 95.22 | 99.51 | 105.32 | 102.85 | 84.81 | 97.85 | 97.57 | 95.83 | 97.67 | 101.74 | 100.93 | 102.78 | 98.51 | 98.68 | 5.30 | 5.38 |
| | 97.18 | 99.65 | 103.45 | 105.74 | 83.34 | 100.67 | 104.48 | 97.80 | 99.29 | 103.16 | 102.95 | 103.74 | 100.12 | 101.81 | 5.95 | 5.94 |
| | 99.32 | 100.96 | 104.67 | 103.41 | 85.86 | 105.40 | 104.33 | 102.72 | 99.66 | 104.20 | 104.07 | 98.95 | 101.13 | 103.07 | 5.31 | 5.25 |
| | 104.07 | 105.21 | 105.10 | 107.45 | 82.79 | 105.10 | 104.31 | 103.63 | 105.68 | 103.31 | 103.13 | 104.2 | 102.84 | 104.28 | 6.42 | 6.25 |
| | 97.00 | 100.92 | 105.48 | 105.77 | 85.33 | 102.77 | 104.01 | 106.65 | 103.12 | 102.09 | 104.51 | 102.66 | 101.69 | 102.95 | 5.75 | 5.65 |
| | 99.44 | 101.53 | 104.18 | 105.21 | 84.13 | 102.93 | 102.02 | 99.25 | 100.60 | 101.99 | 101.51 | 103.19 | 100.50 | 101.76 | 5.45 | 5.42 |
| **Mean** | 99.44 | 101.53 | 104.18 | 105.21 | 84.13 | 102.93 | 102.02 | 99.25 | 100.60 | 101.99 | 101.51 | 103.19 | | | | |
| **Median** | 99.38 | 101.29 | 104.19 | 105.21 | 83.94 | 102.93 | 102.02 | 99.01 | 100.13 | 101.99 | 102.23 | 103.19 | | | | |
| **SD** | 3.66 | 1.72 | 1.11 | 2.05 | 1.01 | 2.50 | 2.53 | 4.35 | 2.52 | 1.42 | 4.61 | 2.03 | | | | |
| **% CV** | 3.68 | 1.70 | 1.07 | 1.94 | 1.20 | 2.43 | 2.48 | 4.39 | 2.51 | 1.39 | 4.54 | 1.97 | | | | |

The results of the analysis show that no significant plate effect was observed for the MultiPROBE Lp-PLA2 DAZ assay. Furthermore, the assay showed excellent intra- and inter-assay CVs demonstrating that the Lp-PLA2 DAZ assay can be automated on the MultiPROBE platform.

Automated DAZ on MiniTrak

For the automated sample transfer on the MiniTrak platform, stock sample plates were loaded onto the sample stacker (12 maximum), and an equal number of empty assay plates were loaded onto the assay plate stacker. The sample transfer program on the MiniTrak system was started which includes the following actions: Pick up tips, Pick up 25 uL of samples, Dispense samples into assay plate, Discard tips and Repeat. Sample plates were labeled and stored at 4°C until use. For long-term storage, plate were sealed and stored at -80°C until use.

To perform the assay the sample-loaded assay plates (12 maximum) were placed onto the assay plate stacker. The MiniTrak platform picked up tips and drew up 115 uL of R2 substrate into each tip. 110 uL of R2 substrate was dispensed into all wells and the tips were discarded. The change in absorption was immediately measured using a plate reader in kinetic mode at 405 nm for 5 minutes after the addition of R2. The plate reader was set at room temperature, with auto mix set to mix for 20 seconds once before read, and data points were obtained at 15 second intervals. The protocol was repeated with subsequent plates once the reading of each previous plate was completed.

*MiniTrak Lp-PLA2 DAZ Performance*

Performance of the MiniTrak Lp-PLA2 DAZ assay was evaluated for reproducibility and accuracy during a batch run, evidence of plate effect, correlation to the manual DAZ assay in normal plasma, and precision. A typical batch run consisted of testing 10 plates which can be completed by one operator in 2 hours.

*MiniTrak Lp-PLA2 DAZ Batch run reproducibility and plate effect*

To test the reproducibility and accuracy in a batch run, and potential plate effect of the MiniTrak DAZ assay, we tested a single simulated plasma sample (calf serum spiked with rLp-PLA2) on ten assay plates to generate 960 replicates of the sample. Following are the results of the analysis showing the mean, median, standard deviation, and %CV of each row and column of each plate.

Plate effect analysis of MiniTrak DAZ assay (Plate 1)

| Plate1 | | | | | | | | | | | | | Mean | Median | %CV | SD |
|---|---|---|---|---|---|---|---|---|---|---|---|---|---|---|---|---|
| | 89.593 | 87.747 | 86.927 | 89.827 | 87.64 | 91.287 | 91.213 | 90.133 | 89.7 | 83.1 | 89.9 | 88.507 | **88.80** | **89.65** | **2.54** | **2.26** |
| | 89.747 | 86.6 | 89.36 | 91.087 | 84.993 | 89.047 | 90.173 | 85.673 | 90.607 | 84.087 | 88.953 | 84.307 | **87.89** | **89.00** | **2.93** | **2.58** |
| | 87.04 | 87.193 | 89.22 | 90.153 | 84.94 | 89.92 | 90.847 | 85.233 | 90.18 | 90.493 | 86.207 | 89.84 | **88.44** | **89.53** | **2.45** | **2.17** |
| | 88.353 | 86.707 | 84.573 | 89 | 86.427 | 88.62 | 90.827 | 89.08 | 87.96 | 91.073 | 89.56 | 90.58 | **88.56** | **88.81** | **1.94** | **1.94** |
| | 88.867 | 89.893 | 85.693 | 91.473 | 90.24 | 88.42 | 89.92 | 95.26 | 87.28 | 90.44 | 85.993 | 85.607 | **89.09** | **89.38** | **3.13** | **2.78** |
| | 87.313 | 91.707 | 87.973 | 91.733 | 89.76 | 85.393 | 90.773 | 88.54 | 87.567 | 89.527 | 84.967 | 87.927 | **88.60** | **88.26** | **2.49** | **2.20** |
| | 87.333 | 89.473 | 86.58 | 90.88 | 89.827 | 87.967 | 90.693 | 90.273 | 89.04 | 89.527 | 90.113 | 87.553 | **89.10** | **89.50** | **1.59** | **1.42** |
| | 87.973 | 88.713 | 83.593 | 89.46 | 90.66 | 84.013 | 88.153 | 90.213 | 86.4 | 88.747 | 89.933 | 86.18 | **87.84** | **88.43** | **2.65** | **2.33** |
| **Mean** | **88.28** | **88.50** | **86.74** | **90.45** | **88.06** | **88.08** | **90.32** | **89.30** | **88.59** | **88.37** | **88.20** | **87.56** | | | | |
| **Median** | **88.16** | **88.23** | **86.75** | **90.52** | **88.70** | **88.52** | **90.73** | **89.61** | **88.50** | **89.53** | **89.26** | **87.74** | | | | |
| **%CV** | **1.19** | **2.02** | **2.40** | **1.09** | **2.70** | **2.67** | **1.07** | **3.50** | **1.71** | **3.45** | **2.40** | **2.43** | | | | |
| **SD** | **1.05** | **1.79** | **2.08** | **0.99** | **2.38** | **2.35** | **0.97** | **3.12** | **1.51** | **3.05** | **2.11** | **2.13** | | | | |

Plate-to-plate reproducibility analysis of MiniTrak Lp-PLA2 DAZ assay

| | Mean | Median | %CV | SD |
|---|---|---|---|---|
| Plate 1 | 90.12 | 90.18 | 4.04 | 3.64 |
| Plate 2 | 90.04 | 90.16 | 4.08 | 3.67 |
| Plate 3 | 89.47 | 89.67 | 5.49 | 4.91 |
| Plate 4 | 90.18 | 89.34 | 4.39 | 3.96 |
| Plate 5 | 81.73 | 81.25 | 5.65 | 4.62 |
| Plate 6 | 88.54 | 89.02 | 2.49 | 2.21 |
| Plate 7 | 87.64 | 87.81 | 1.58 | 1.39 |
| Plate 8 | 94.57 | 94.25 | 3.07 | 2.90 |
| Plate 9 | 88.17 | 88.07 | 4.71 | 4.15 |
| Plate 10 | 84.24 | 84.19 | 6.03 | 5.08 |
| | | | | |
| Average | 88.47 | 88.39 | 4.15 | 3.65 |

The results of the analysis show that no significant plate effect was observed for the MiniTrak DAZ assay. Furthermore, the assay showed excellent intra- and inter- assay CVs. The values were reproducible throughout the 10-plate batch run, indicating that accurate values without drift are obtained during a batch run.

*Correlation between Automated MiniTrak DAZ and manual DAZ Assays*

To test the correlations between values derived from the manual DAZ and automated MiniTrak DAZ assays, 80 normal plasma samples were tested with both assays, and the values were correlated in a linear regression plot. A best fit line for the linear regression plot had a slope of y= 0.9557x - 0.3013. Additionally the $R^2$ value for the data set was 0.9487.

These results demonstrate an excellent correlation between the manual DAZ and MiniTrak DAZ assays. The automated DAZ assay on MiniTrak is equivalent to the manual DAZ assay for detecting change of Lp-PLA2 activity due to administration of an Lp-PLA2 inhibitor.

*MiniTrak Lp-PLA2 DAZ Precision*

Intra-assay variability for the MiniTrak Lp-PLA2 DAZ assay was evaluated by the average %CV calculated from %CVs of 24 plasma samples run in triplicate. The table below shows average %CVs from the 24 samples in the 3 runs, and the average intra-assay %CV. Precision for DAZ assay was calculated for nmol/min/mL.

MiniTrak Lp-PLA2 DAZ (nmol/min/mL)

| Plasma Sample | Run1 (%CV) | Run2 (%CV) | Run3 (%CV) | Average Intra-Assay % CV |
|---|---|---|---|---|
| 1 | 5.9 | 0.5 | 2.4 | 2.9 |
| 2 | 1.1 | 0.4 | 1.9 | 1.1 |
| 3 | 0.9 | 0.4 | 5.1 | 2.1 |
| 4 | 0.1 | 1.4 | 0.4 | 0.6 |
| 5 | 0.9 | 0.5 | 1.2 | 0.9 |
| 6 | 1.0 | 0.2 | 1.1 | 0.8 |
| 7 | 1.3 | 2.6 | 0.6 | 1.5 |
| 8 | 1.4 | 1.7 | 1.4 | 1.5 |
| 9 | 1.3 | 2.5 | 2.5 | 2.1 |

(continued)

| Plasma Sample | Run1 (%CV) | Run2 (%CV) | Run3 (%CV) | Average Intra-Assay % CV |
|---|---|---|---|---|
| 10 | 2.6 | 1.6 | 2.3 | 2.2 |
| 11 | 1.9 | 3.0 | 0.7 | 1.9 |
| 12 | 0.6 | 0.9 | 2.2 | 1.3 |
| 13 | 1.1 | 0.8 | 2.8 | 1.6 |
| 14 | 5.4 | 1.9 | 1.1 | 2.8 |
| 15 | 1.3 | 2.7 | 0.5 | 1.5 |
| 16 | 1.2 | 1.6 | 1.4 | 1.4 |
| 17 | 0.4 | 3.0 | 2.3 | 1.9 |
| 18 | 2.0 | 3.8 | 0.5 | 2.1 |
| 19 | 1.9 | 0.6 | 1.0 | 1.2 |
| 20 | 1.2 | 0.7 | 1.7 | 1.2 |
| 21 | 0.8 | 1.4 | 0.8 | 1.0 |
| 22 | 1.7 | 1.8 | 0.4 | 1.3 |
| 23 | 2.6 | 4.9 | 1.2 | 2.9 |
| 24 | 2.0 | 2.3 | 7.6 | 3.9 |
| Average %CV | 1.7 | 1.7 | 1.8 | 1.7 |

Inter-assay variability for the MiniTrak Lp-PLA2 DAZ assay was evaluated by %CV from values for 24 plasma samples analyzed in 3 independent assays. Average inter-assay %CV for each was calculated from the slopes of the 24 samples. Precision for MiniTrak Lp-PLA2 DAZ assay was calculated for nmol/min/mL.

MiniTrak Lp-PLA2 DAZ (nmol/min/mL)

| Plasma | Run1 | Run2 . | Run3 | Inter-Assay CV |
|---|---|---|---|---|
| 1 | 104.61 | 112.90 | 107.76 | 3.8% |
| 2 | 159.66 | 174.56 | 163.28 | 4.6% |
| 3 | 82.79 | 89.25 | 82.59 | 4.4% |
| 4 | 114.32 | 125.53 | 118.53 | 4.6% |
| 5 | 114.33 | 126.18 | 118.38 | 4.9% |
| 6 | 131.09 | 141.90 | 134.49 | 4.0% |
| 7 | 148.08 | 162.57 | 155.41 | 4.6% |
| 8 | 178.05 | 194.40 | 184.75 | 4.3% |
| 9 | 271.28 | 311.94 | 294.44 | 6.9% |
| 10 | 154.96 | 168.24 | 159.61 | 4.1% |
| 11 | 137.72 | 152.27 | 148.34 | 5.0% |
| 12 | 154.04 | 173.76 | 165.58 | 5.9% |
| 13 | 86.47 | 98.32 | 92.47 | 6.3% |
| 14 | 116.64 | 127.12 | 122.57 | 4.2% |
| 15 | 114.79 | 128.17 | 122.58 | 5.4% |
| 16 | 113.25 | 125.25 | 121.47 | 5.0% |

(continued)

| Plasma | Run1 | Run2 . | Run3 | Inter-Assay CV |
|---|---|---|---|---|
| 17 | 153.93 | 168.41 | 161.36 | 4.4% |
| 18 | 138.74 | 155.41 | 149.92 | 5.6% |
| 19 | 74.32 | 81.62 | 77.87 | 4.6% |
| 20 | 53.02 | 57.38 | 53.38 | 4.4% |
| 21 | 55.99 | 60.46 | 55.89 | 4.5% |
| 22 | 82.80 | 90.45 | 84.46 | 4.6% |
| 23 | 100.27 | 109.14 | 103.85 | 4.2% |
| 24 | 99.92 | 108.42 | 97.05 | 5.8% |
| Average %CV | 122.54 | 135.15 | 128.17 | 4.8% |

We have successfully demonstrated the adaptation of an Lp-PLA2 activity based assay, such as the DAZ assay, to an automated platform, such as the MultiPROBE and MiniTrak automated platforms. Additionally, we have demonstrated that the automated DAZ assay on the MiniTrak platform shows excellent reproducibility and precision, and has good correlation to the manual DAZ method. Furthermore, the automated MiniTrak DAZ assay is a high-throughput assay that can analyze 10 plates per batch (960 determinations), per operator in 2 hours. Based on this throughput, we estimate that one operator can reliably perform two batch runs per day, resulting in the analysis of 20 plates, or 1920 determinations per day.

We have successfully applied both robotic systems to sample loading to 96-well assay plate and substrate addition, but have relied on an operator to move the plate from the robotic station to a plate reader. However, it is anticipated that with the appropriate equipment and modifications, all of the steps can be automated. Adapting a Zymark twister arm to the MiniTrak, would allow from automation of the whole process. Additionally, it is preferred that loading the samples onto the assay plate, especially when transferring samples from tubes to plate, be performed manually, or with a robotic system equipped with a sensitive liquid detection capability (e.g., MultiPROBE). It is advantageous that the system can detect clogged tips, due to the obstructive particulates that are commonly found in plasma and serum samples. Furthermore, it is also advantageous that the addition of substrate into all 96-wells of the assay plate be done simultaneously, using a robotic system equipped with a 96-head dispenser (e.g., MiniTrak), to maintain good precision. Thus, both robotic systems, the MultiPROBE (or manual pipetting) for sample loading to 96-well assay plate and MiniTrak for substrate addition, are useful in the automation of the Lp-PLA2 DAZ assay. It is anticipated with the proper equipment, all processes/tasks for the Lp-PLA2 DAZ assay can be fully automated in one integrated system.

**Lp-PLA2 HIC-DAZ Assay Automation**

For the automation of the HIC-DAZ assay described above, we have broken down the assay into 5 main tasks: Sample loading to 96-well assay plate, Incubation, Wash, Substrate addition and Reading. Automation of each task was evaluated on the MiniTrak robotic platform due to the success observed with the DAZ assay automation.

<u>Automated HIC on MiniTrak</u>

20 uL of plasma samples, standards, and controls were transferred into anti Lp-PLA2 2C10 antibody-coated halfwell assay plates using either the MultiPROBE platform (method described above), the MiniTrak (method described above.), or manually. This was performed for 5 plates (one batch run is 5 plates). The time was marked for Plate 1 and subsequent steps were started for plate 1. The same procedure was followed for each plate, in sequence, at 6 minute intervals.

After addition of the plasma samples, standards, and controls the plate was sealed and incubated with shaking 180 rpm at room temperature for 30 minutes. Following incubation, plates were washed twice with 75 uL 1xTBS with 0.05% Tween-20 and placed on the MiniTrak. The Pick-up tips function on the MiniTrak was initiated and 23 uL of R2 substrate was drawn into each tip. After 20 uL R2 substrate was dispensed into each well the tips were discarded. The change in absorption was immediately measured using a plate reader in kinetic mode at 405 nm for 5 minutes after the addition of R2. The plate reader was set at room temperature, with auto mix set to mix for 10 seconds once before read, and data points were obtained at 15 second intervals. The protocol was repeated with subsequent plates once the reading of each previous plate was completed.

*MiniTrak Lp-PLA2 HIC-DAZ Performance*

Performance of the MiniTrak Lp-PLA2 HIC assay was evaluated for reproducibility and accuracy during a batch run, evidence of plate effect, correlation to the manual HIC-DAZ assay in normal plasma samples, and precision. A typical batch run consisted of testing 5 plates, which can be completed by one operator in 1.5 hours.

*MiniTrak Batch run reproducibility and plate effect*

To test the reproducibility and accuracy in a batch run, and potential plate effect of the MiniTrak HIC-DAZ assay, we tested a single simulated plasma sample (calf serum spiked with rLp-PLA2) on 5 assay plates to generate 480 replicates of the sample. Following are the results of the analysis showing the mean, median, standard deviation, and %CV of each row and column of each plate.

Plate effect analysis of MiniTrak Lp-PLA2 HIC-DAZ assay

| | | | | | | | | | | | | | Mean | Median | STDEV | CV |
|---|---|---|---|---|---|---|---|---|---|---|---|---|---|---|---|---|
| Plate1 | 35.52 | 27.44 | 28.96 | 33.6 | 27.64 | 30.8 | 33.32 | 28.2 | 28.76 | 33.08 | 26.2 | 29.04 | 30.2133 | 29.00 | 2.975 | 9.8% |
| | 31.04 | 26.48 | 27.44 | 31 | 30.68 | 27.2 | 31.88 | 27.32 | 27.4 | 33.56 | 28.68 | 28.04 | 29.2267 | 28.36 | 2.294 | 7.8% |
| | 37.08 | 28.36 | 27 | 32.16 | 23.32 | 26.6 | 30.8 | 26.36 | 28 | 29.2 | 24.24 | 25.96 | 28.2567 | 27.50 | 3.744 | 13.3% |
| | 30.92 | 25.36 | 26.32 | 30.4 | 26.64 | 24.72 | 30.76 | 24.28 | 27.4 | 28.44 | 25.68 | 26.04 | 27.2467 | 26.48 | 2.357 | 8.7% |
| | 30.36 | 31.76 | 25.68 | 31.4 | 32.2 | 24.84 | 31.52 | 34.32 | 25.88 | 28.84 | 32.76 | 25.44 | 29.5833 | 30.88 | 3.316 | 11.2% |
| | 30.16 | 30.28 | 25.04 | 31.24 | 32.2 | 22.96 | 29.44 | 37.08 | 25.16 | 27.44 | 30.28 | 23.8 | 28.7567 | 29.80 | 4.057 | 14.1% |
| | 28.4 | 30.4 | 23.44 | 28.84 | 33 | 23.92 | 26.6 | 31.8 | 23.96 | 26.36 | 30 | 22.84 | 27.4633 | 27.50 | 3.459 | 12.6% |
| | 29 | 30.28 | 28.48 | 27.88 | 30.04 | 23.72 | 26.16 | 32.36 | 25.44 | 31.04 | 30.76 | 19.52 | 27.89 | 28.74 | 3.649 | 13.1% |
| Mean | 31.56 | 28.795 | 26.545 | 30.815 | 29.465 | 25.595 | 30.06 | 30.215 | 26.5 | 29.745 | 28.575 | 25.085 | | | | |
| Median | 30.64 | 29.32 | 26.66 | 31.12 | 30.36 | 24.78 | 30.78 | 30 | 26.64 | 29.02 | 29.34 | 25.7 | | | | |
| STDEV | 3.088 | 2.235 | 1.824 | 1.805 | 3.345 | 2.543 | 2.526 | 4.369 | 1.636 | 2.590 | 2.929 | 3.020 | | | | |
| CV | 9.8% | 7.8% | 6.9% | 5.9% | 11.4% | 9.9% | 8.4% | 14.5% | 6.2% | 8.7% | 10.3% | 12.0% | | | | |

Plate-to-plate reproducibility analysis of MiniTrak Lp-PLA2 HIC-DAZ assay

| | Mean | Median | SD | CV |
|---|---|---|---|---|
| Plate1 | 28.58 | 28.46 | 3.31 | 11.6% |
| Plate2 | 30.16 | 29.70 | 4.13 | 13.7% |
| Plate3 | 29.28 | 28.96 | 3.89 | 13.3% |
| Plate4 | 28.93 | 28.86 | 3.92 | 13.5% |
| Plate5 | 26.70 | 26.14 | 3.56 | 13.3% |
| | | | | |
| Average | 28.73 | 28.42 | 3.76 | 13.1% |

The results of the analysis show that, although the MiniTrak HIC-DAZ assay had higher well-to-well variation than the MiniTrak DAZ assay, in general, no significant plate effect was observed in the MiniTrak HIC-DAZ assay. Furthermore, the assay showed acceptable intra- and inter-assay CVs, and the values were reproducible throughout the 5-plate batch run, indicating that accurate values without drift can be obtained during a batch run.

*Correlation between MiniTrak HIC-DAZ and manual HIC-DAZ*

To test the correlation between values derived from the manual HIC-DAZ and automated MiniTrak HIC-DAZ assays, 80 normal plasma samples were tested with both assays and the values were correlated in a linear regression plot. Four samples failed to meet the CV range, and thus, were excluded from the analysis. A best fit line for the linear regression plot had a slope of y= 0.8971x + 2.5573. Additionally the $R^2$ value for the data set was 0.8493.

The results show that there is a good correlation between the manual HIC and MiniTrak HIC assays. Furthermore, the automated HIC-DAZ assay on the MiniTrak is comparable to the manual HIC-DAZ assay for detecting change of Lp-PLA2 activity due to administration of an Lp-PLA2 inhibitor.

*MiniTrak Lp-PLA2 HIC-DAZ Precision*

Intra-assay variability for the MiniTrak Lp-PLA2 HIC-DAZ assay was evaluated by the average %CV calculated from %CVs of 24 plasma samples run in triplicate. The table below shows average %CVs from 3 runs, and the average intra-assay %CV from each of these 3 runs. Precision for HIC-DAZ assay was calculated for nmol/min/mL.

MiniTrak Lp-PLA2 HIC-DAZ (nmol/min/mL)

| Plasma | Run1 %CV | Run2 %CV | Run3 %CV | Average Intra-Assay %CV |
|---|---|---|---|---|
| 1 | 10.08 | 14.43 | 8.52 | 11.01 |
| 2 | 14.84 | 15.24 | 10.71 | 13.60 |
| 3 | 12.02 | 14.38 | 13.79 | 13.40 |
| 4 | 4.18 | 11.98 | 6.75 | 7.64 |
| 5 | 8.66 | 14.35 | 11.37 | 11.46 |
| 6 | 12.48 | 14.97 | 10.91 | 12.79 |
| 7 | 7.57 | 11.56 | 11.79 | 10.31 |
| 8 | 7.99 | 15.07 | 12.49 | 11.85 |
| 9 | 14.55 | 14.60 | 12.64 | 13.93 |
| 10 | 21.25 | 17.10 | 15.64 | 18.00 |
| 11 | 12.61 | 15.62 | 13.03 | 13.75 |
| 12 | 23.14 | 18.19 | 15.09 | 18.81 |
| 13 | 14.45 | 16.96 | 10.64 | 14.01 |

(continued)

| Plasma | Run1 %CV | Run2 %CV | Run3 %CV | Average Intra-Assay %CV |
|---|---|---|---|---|
| 14 | 12.40 | 17.39 | 10.69 | 13.49 |
| 15 | 8.61 | 14.84 | 7.07 | 10.18 |
| 16 | 22.65 | 17.53 | 7.45 | 15.88 |
| 17 | 13.52 | 14.53 | 12.47 | 13.50 |
| 18 | 11.85 | 10.28 | 8.68 | 10.27 |
| 19 | 16.43 | 16.33 | 14.84 | 15.87 |
| 20 | 6.62 | 14.60 | 20.08 | 13.76 |
| 21 | 12.53 | 17.44 | 9.82 | 13.26 |
| 22 | 17.33 | 12.45 | 10.04 | 13.27 |
| 23 | 14.36 | 16.42 | 19.60 | 16.79 |
| 24 | 8.93 | 12.97 | 7.05 | 9.65 |
| Average %CV | 12.88 | 14.97 | 11.71 | 13.19 |

Intra-assay variability for the MiniTrak Lp-PLA2 HIC-DAZ assay was evaluated by the average inter-assay %CV from values for 24 plasma samples analyzed in 3 independent assays. Average inter-assay %CV for each was calculated from the slope of the 24 samples. Precision for HIC assay was calculated for nmol/min/mL.

MiniTrak Lp-PLA2 HIC-DAZ (nmol/min/mL)

| Plasma | Average Run1 nmol/min/ml | Average Run2 nmol/min/ml | Average Run3 nmol/min/ml | Inter-Assay CV |
|---|---|---|---|---|
| 1 | 33.52 | 33.34 | 35.64 | 3.7% |
| 2 | 40.25 | 36.40 | 36.61 | 5.7% |
| 3 | 30.10 | 30.37 | 28.34 | 3.7% |
| 4 | 34.99 | 32.69 | 31.85 | 4.9% |
| 5 | 29.23 | 29.25 | 29.13 | 0.2% |
| 6 | 32.25 | 31.16 | 32.04 | 1.8% |
| 7 | 38.22 | 39.08 | 38.56 | 1.1% |
| 8 | 30.41 | 29.93 | 28.41 | 3.5% |
| 9 | 50.67 | 50.97 | 53.06 | 2.5% |
| 10 | 25.51 | 22.93 | 23.23 | 5.9% |
| 11 | 34.13 | 33.28 | 31.32 | 4.4% |
| 12 | 34.67 | 29.32 | 29.32 | 9.9% |
| 13 | 31.93 | 32.32 | 32.76 | 1.3% |
| 14 | 29.90 | 27.26 | 28.81 | 4.6% |
| 15 | 37.38 | 38.68 | 37.90 | 1.7% |
| 16 | 25.66 | 28.40 | 29.41 | 7.0% |
| 17 | 37.64 | 36.67 | 37.34 | 1.3% |
| 18 | 38.68 | 37.73 | 38.96 | 1.7% |
| 19 | 23.30 | 22.86 | 22.99 | 1.0% |
| 20 | 14.56 | 12.03 | 13.00 | 9.7% |

(continued)

| Plasma | Average Run1 nmol/min/ml | Average Run2 nmol/min/ml | Average Run3 nmol/min/ml | Inter-Assay CV |
|---|---|---|---|---|
| 21 | 16.86 | 15.83 | 16.63 | 3.3% |
| 22 | 21.37 | 21.18 | 21.75 | 1.4% |
| 23 | 28.32 | 29.41 | 28.99 | 1.9% |
| 24 | 31.20 | 30.14 | 32.40 | 3.6% |
| Average | 31.28 | 30.47 | 30.77 | 3.6% |

We have successfully demonstrated the adaptation of an Lp-PLA2 HIC-DAZ assay to an automated platform such as the MiniTrak automated platform. We have demonstrated that the automated HIC assay on the MiniTrak shows acceptable reproducibility and precision, and has good correlation to the manual HIC-DAZ method. Furthermore, the automated MiniTrak HIC-DAZ assay is a high-throughput assay that can analyze 5 plates per batch (480 determinations), per operator in 1.5 hours. Based on this throughput, one operator can reliably perform three batch runs per day, resulting in the analysis of 15 plates, or 1440 determinations, per day.

As with the MiniTrak DAZ assay, we have successfully applied robotic systems described previously to sample loading to 96-well assay plate and substrate addition to the HIC-DAZ assay, but have relied on an operator to wash the plate and to move the plate from the robotic station to a plate reader. However, it is anticipated that, with the appropriate equipment and modifications, all of the steps can be automated in an integrated system. Adapting a Zymark twister arm to the MiniTrak, would automate the entire process. It is advantageous that loading of the samples onto the assay plate, especially when transferring samples from tubes to plate, be performed manually, or with a robotic system equipped with a sensitive liquid detection capability (e.g., MultiPROBE) that can detect clogged tips, due to particulates that are commonly found in plasma and serum samples.

Plasma/Serum Correlations and Ranges

The suitability of the activity-based Lp-PLA2 detection methods described above for testing serum samples in future epidemiological studies was evaluated. A comparison was made between matched serum and plasma samples for the DAZ and HIC-DAZ assay formats. Correlations and normal range comparisons between matched serum and plasma samples are reported.

Matched plasma and serum samples from 200 PromedDx, and 38 diaDexus donors were tested for correlation of measured Lp-PLA2 activity levels in each sample. All samples were tested with the automated DAZ and HIC-DAZ assays on the MiniTrak. The values were analyzed on linear regression plot.

The linear regression plot between the matched plasma and serum samples from the 38 diaDexus donors using the automated Lp-PLA2 DAZ assay generated a best fit line with a slope of y=1.0386x + 0.7997. Additionally, the $R^2$ value for the data set was 0.9835.

The linear regression plot between the matched plasma and serum samples from the 200 PromeDx (Norton, MA) samples using the automated Lp-PLA2 DAZ assay generated a best fit line with a slope of y=0.9771x + 3.7692. Additionally, the $R^2$ value for the data set was 0.9356.

The linear regression plot between the matched plasma and serum samples from the 38 diaDexus donors using the automated Lp-PLA2 HIC-DAZ assay generated a best fit line with a slope of y=0.9106x + 1.3824. Additionally, the $R^2$ value for the data set was 0.786.

The linear regression plot between the matched plasma and serum samples from the 200 PromeDx samples using the automated Lp-PLA2 HIC-DAZ assay generated a best fit line with a slope of y=0.7728x + 4.5458. Additionally, the $R^2$ value for the data set was 0.6846.

The results show that correlations between Lp-PLA2 activity levels from plasma and serum are excellent, especially for the DAZ assay. Very high correlations between plasma and serum samples in the DAZ assay indicate that there is virtually no difference between plasma and serum samples for that format. The HIC-DAZ assay demonstrated lower correlation between plasma and serum samples than the DAZ assay, which might be due to higher analytical variation in the HIC-DAZ assay. Nevertheless, the data show a good correlation between HIC-DAZ values derived from plasma and serum, indicating that, in general, there seem to be no significant differences between Lp-PLA2 activity levels detected from plasma and serum.

Plasma/Serum Normal Ranges

To compare the DAZ and HIC-DAZ normal range values derived from plasma and serum samples, we plotted distribution curves of DAZ and HIC-DAZ values from the 200 matching plasma and serum samples described above. These normal range plots are represented in the tables below.

Normal Ranges for Plasma and Serum samples - DAZ

| DAZ Quantiles | | Plasma (nmol/min/mL) | Serum (nmol/min/mL) |
|---|---|---|---|
| 100.00% | maximum | 250.93 | 268.00 |
| 99.50% | | 249.82 | 264.59 |
| 97.50% | | 227.48 | 236.29 |
| 90.00% | | 187.85 | 193.49 |
| 75.00% | quartile | 165.94 | 167.26 |
| 50.00% | median | 145.47 | 149.48 |
| 25.00% | quartile | 124.02 | 125.67 |
| 10.00% | | 100.63 | 103.76 |
| 2.50% | | 85.61 | 88.96 |
| 0.50% | | 64.05 | 69.91 |
| 0.00% | minimum | 63.29 | 69.62 |

Normal Ranges for Plasma and Serum samples - HIC-DAZ

| HIC Quantiles | | Plasma (nmol/min/mL) | Serum (nmol/min/mL) |
|---|---|---|---|
| 100.00% | maximum | 36.95 | 38.48 |
| 99.50% | | 36.92 | 37.80 |
| 97.50% | | 34.40 | 33.44 |
| 90.00% | | 30.95 | 29.90 |
| 75.00% | quartile | 27.13 | 26.69 |
| 50.00% | median | 23.64 | 23.05 |
| 25.00% | quartile | 19.80 | 20.28 |
| 10.00% | | 15.59 | 15.84 |
| 2.50% | | 11.36 | 11.14 |
| 0.50% | | 8.56 | 8.60 |
| 0.00% | minimum | 8.16 | 8.11 |

The data above indicate that the normal range distribution is almost identical between plasma and serum samples for both DAZ and HIC assays. These results again support our previous observation, which showed very high correlation between the DAZ and HIC values derived from the 200 matching plasma and serum samples.

**Example 8: Conversion Factors Among Assay Formats**

The 200 PromeDx normal plasma samples were utilized to determine a conversion factor of nmol/min/mL to ng/mL based on distribution using both the DAZ and HIC-DAZ MiniTrak automated assay formats. The SoftMax Pro 4.7.1 software from Molecular Devices (Sunnyvale, CA) was utilized to collect optical density of each sample generating a slope value. The slope was converted to nmol/min/mL by multiplying 1.11 and 1.43 for HIC-DAZ and DAZ, respectively. These conversions were determined based on p-Nitrophenol (PNP) standards described above.

Values for nmol/min/mL and ng/mL were evaluated using the JMP4 statistical discovery software from the SAS Institute (Cary, NC). Data was processed using the Distribution tool under the Analyze option group. Only those values within the linear range of the standard curve of each assay were analyzed. The linear range of HIC-DAZ was determined to be 0-270 ng/mL and DAZ was 0-1000 ng/mL. Values from equivalent percentiles within these ranges were compared by performing a linear regression analysis. The equations generated in this program were designated as conversion factors. The ng/mL to nmol/min/ml conversion factor for the HIC-DAZ assay is y = 8.4464x - 10.771, with an $R^2$ value of 0.9874. The ng/mL to nmol/min/ml conversion factor for the DAZ assay is y = 4.3404x - 98.625, with an $R^2$ value of 0.9956. Both assay formats were able to highly correlate activity and mass measurements which is useful for comparing results from various assays. The tables below demonstrate the conversion between units at various quantiles in the sample set and the ration of between the units.

DAZ Assay Quantiles

| Quantiles | nmol/min/mL | ng/mL | Ratio |
|---|---|---|---|
| 100% | 250.93 | 1013.4 | 4.04 |
| 99.50% | 250.9 | 1013.2 | 4.04 |
| 97.50% | 225.96 | 873.2 | 3.86 |
| 90% | 185.66 | 701 | 3.78 |
| 75% | 164.13 | 588.80 | 3.59 |
| 50% | 146.60 | 506.10 | 3.45 |
| 25% | 125.64 | 428.20 | 3.41 |
| 10% | 101.74 | 335.10 | 3.29 |
| 2.50% | 84.16 | 277.90 | 3.30 |
| 0.50% | 63.31 | 196.70 | 3.11 |
| 0% | 63.29 | 196.60 | 3.11 |

HIC-DAZ Assay Quantiles

| Quantiles | nmol/min/mL | ng/mL | Ratio |
|---|---|---|---|
| 75% | 27.40 | 232.14 | 8.47 |
| 50% | 24.40 | 194.91 | 7.99 |
| 25% | 21.34 | 159.89 | 7.49 |
| 10% | 18.12 | 136.50 | 7.53 |
| 2.50% | 14.21 | 104.44 | 7.35 |
| 0.50% | 7.98 | 61.15 | 7.66 |
| 0% | 7.97 | 61.11 | 7.67 |

Conversion of nmol/min/mL to ng/mL allows for the correlation of results various assay formats. Elevated Lp-PLA2 levels (ng/mL) in serum have been shown to be associated several forms of CVD. Conversion of mass and activity levels of Lp-PLA2 is useful for correlating differing assay formats for use in assessing risk, diagnosing an Lp-PLA2 disorder, selecting persons for CVD therapy and monitoring response to CVD therapy such as statins and Lp-PLA2 inhibitors.

In summary, we have successfully developed automated high-throughput Lp-PLA2 assays that allow for completion of 1920 and 1440 determinations on DAZ and HIC-DAZ assays, respectively, for an operator during an 8-hour shift. These results demonstrate that full automation of Lp-PLA2 DAZ and HIC-DAZ assays is possible. The automated assays are reproducible and accurate, and generate data that are virtually identical to the manual methods in normal samples. Evaluation of matching plasma and serum samples show that both Lp-PLA2 DAZ and HIC-DAZ assays generate comparable values in plasma and serum, indicating the potential of utilizing serum samples in future epidemiological studies.

The following numbered statements further describe the invention:

1. A method for measuring enzymatically active Lipoprotein-associated Phospholipase A2 (Lp-PLA2) in a sample comprising:

(a) contacting an immobilized binder, which specifically binds Lp-PLA2, with the sample;
(b) washing the immobilized binder to remove an enzymatically active unbound material or an interfering substance(s);
(c) contacting the bound Lp-PLA2 with a substrate converted to a detectable product in the presence of Lp-PLA2; and
(d) measuring detectable product indicative of enzymatically active Lp-PLA2 in the sample.

2. The method of statement 1, wherein the sample is a serum sample, a plasma sample, an EDTA treated plasma sample or an EDTA treated serum sample.
3. The method of statement 1, wherein the immobilized binder is an antibody.
4. The method of statement 3, wherein the antibody is a monoclonal antibody, a phage display antibody, or a polyclonal antibody.
5. The method of statement 4, wherein the antibody is a monoclonal antibody.
6. The method of statement 1 wherein the enzymatically active unbound material is a phospholipase.
7. The method of statement 1 wherein the interfering substance(s) is a free-thiol compound.
8. The method of statement 1, wherein the substrate is selected from the group consisting of

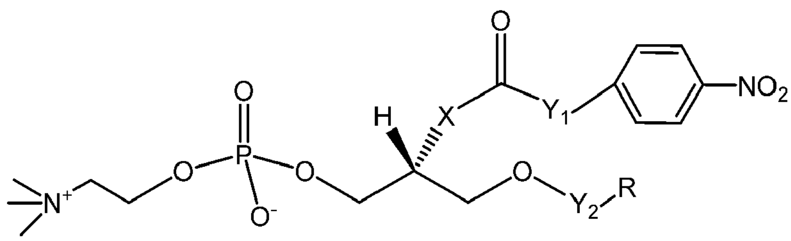

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH{=}CH(CH_2)_2CH3$;
$Y_1$ is selected from the group consisting of $(CO)_{1\text{-}2}$ and $(CH_2)_{2\text{-}7}$; and
$Y_2$ is selected from the group consisting of CO and $CH_2$;

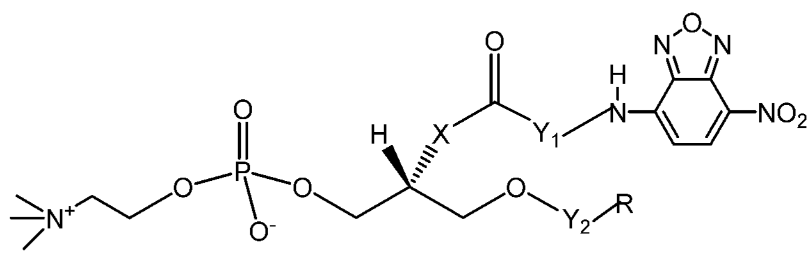

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH{=}CH(CH_2)_2CH_3$;
$Y_1$ is selected from the group consisting of $(CO)_{1\text{-}2}$ and $(CH_2)_{2\text{-}7}$; and
$Y_2$ is selected from the group consisting of CO and $CH_2$;

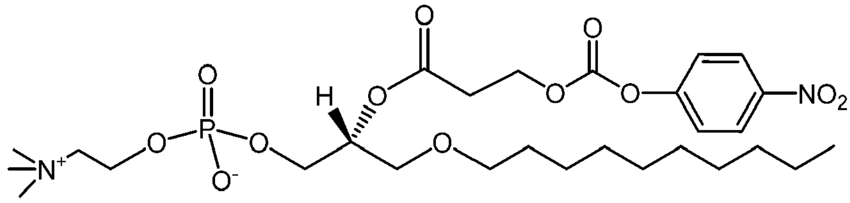

(c)

1-myristoyl-2-(4-nitrophenylsuccinyl) phosphatidylcholine (MNP);

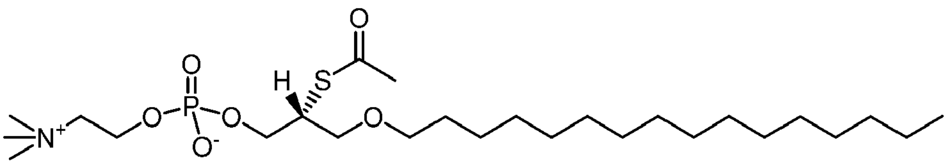

(d)

2-thio PAF; and

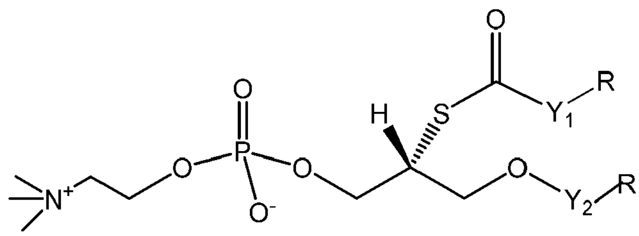

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

9. The method of statement 8 where in the substrate is an oxidized derivative of (a), (b), (c), (d) or (e).
10. The method of statement 1, wherein the detectable product has a radioactive, colorimetric, paramagnetic or fluorescent label.
11. The method of statement 1 wherein the detectable product is measured fluorimetrically, colorimetrically, paramagnetically or via radiation.
12. The method of statement 1 which further comprising comparing the measured detectable product of step (d) to detectable product in a control comprising an enzymatically active Lp-PLA2 standard.
13. The method of statement 12 wherein the enzymatically active Lp-PLA2 standard is a recombinant Lp-PLA2 protein or a native Lp-PLA2 protein.
14. The method of statement 13 wherein the recombinant Lp-PLA2 protein is expressed in a baculovirus expression system or a mammalian expression system.
15. The method of statement 1 wherein the immobilized binder is bound to a multi-well plate, a magnetic bead, or a latex bead.
16. The method of statement 12 wherein a difference in detectable product in the sample compared to the standard is due to a difference in Lp-PLA2 activity in the sample compared to the standard.
17. A method for detecting vascular disease in an individual comprising utilizing the method of statement 16 to determine the individual's Lp-PLA2 activity in a sample wherein increased activity of Lp-PLA2 in the sample is indicative of vascular disease.

18. The method of statement 17 wherein the vascular disease is selected from the group consisting of coronary vascular disease (CVD), coronary heart disease (CHD), peripheral vascular disease, peripheral arterial disease, stroke, congenital cardiovascular defects and congestive heart failure.

19. A method for selecting an individual for therapy to treat vascular disease comprising utilizing the method of statement 16 to determine the individual's Lp-PLA2 activity in a sample wherein increased activity of Lp-PLA2 in the sample is indicative of an individual who will benefit from therapy to treat vascular disease.

20. The method of statement 19 wherein the vascular disease is selected from the group consisting of coronary vascular disease (CVD), coronary heart disease (CHD), peripheral vascular disease, peripheral arterial disease, stroke, congenital cardiovascular defects and congestive heart failure.

21. The method of statement 19 wherein the therapy is selected from the group consisting of statins and Lp-PLA2 inhibitors.

22. A method for monitoring an individuals response to therapy to treat vascular disease comprising utilizing the method of statement 16 to determine the individual's Lp-PLA2 activity in a sample wherein decreased activity of Lp-PLA2 in the sample is indicative of an individual who is responding favorably to therapy to treat vascular disease.

23. The method of statement 22 wherein the vascular disease is selected from the group consisting of coronary vascular disease (CVD), coronary heart disease (CHD), peripheral vascular disease, peripheral arterial disease, stroke, congenital cardiovascular defects and congestive heart failure.

24. The method of statement 22 wherein the therapy is selected from the group consisting of statins and Lp-PLA2 inhibitors.

25. A method for measuring enzymatically active Lipoprotein-associated Phospholipase A2 (Lp-PLA2) in a sample comprising:

(a) contacting an binder, which specifically binds Lp-PLA2, with the sample to form a binder-Lp-PLA2 complex;
(b) immobilizing the binder-Lp-PLA2 complex;
(c) washing the immobilized binder-Lp-PLA2 complex to remove an enzymatically active unbound material or an interfering substance(s);
(d) contacting the immobilized bound Lp-PLA2 with a substrate converted to a detectable product in the presence of Lp-PLA2; and
(e) measuring detectable product indicative of enzymatically active Lp-PLA2 in the sample.

26. The method of statement 25, wherein the sample is a serum sample, a plasma sample or an EDTA treated plasma sample.

27. The method of statement 25, wherein the binder is an antibody.

28. The method of statement 27, wherein the antibody is a monoclonal antibody, a phage display antibody, or a polyclonal antibody.

29. The method of statement 28, wherein the antibody is a monoclonal antibody.

30. The method of statement 25 wherein the binder-Lp-PLA2 complex is immobilized by binding to an immobilized compound.

31. The method of statement 30 wherein the immobilized compound is an antibody, protein or compound capable of binding the binder-Lp-PLA2 complex.

32. The method of statement 31, wherein the antibody is a monoclonal antibody, a phage display antibody, or a polyclonal antibody.

33. The method of statement 32, wherein the monoclonal antibody, the phage display antibody, or the polyclonal antibody is a rat, mouse or goat anti-Ig antibody.

34. The method of statement 30 wherein the immobilized compound is bound to a multi-well plate, a magnetic bead, or a latex bead.

35. The method of statement 25 wherein the binder is conjugated to an immobilizing agent.

36. The method of statement 35, wherein the binder conjugated to an immobilizing agent is an antibody.

37. The method of statement 36, wherein the antibody is a monoclonal antibody, a phage display antibody, or a polyclonal antibody.

38. The method of statement 37, wherein the antibody is a monoclonal antibody.

39. The method of statement 35 wherein the immobilizing agent is an antibody, protein or compound capable of binding an immobilized compound.

40. The method of statement 39, wherein the antibody is a monoclonal antibody, a phage display antibody, or a polyclonal antibody.

41. The method of statement 40, wherein the monoclonal antibody, the phage display antibody, or the polyclonal antibody is a rat, mouse or goat anti-Ig antibody.

42. The method of statement 35 wherein the immobilizing agent is biotin.

43. The method of statement 35 wherein the immobilizing agent, conjugated to the binder-Lp-PLA2 complex, binds to an immobilized compound.
44. The method of statement 43 wherein the immobilized compound is bound to a multi-well plate, a magnetic bead, or a latex bead.
45. The method of statement 44 wherein the bound compound is an antibody, protein or compound capable of binding the conjugated immobilizing agent.
46. The method of statement 45, wherein the antibody is a monoclonal antibody, a phage display antibody, or a polyclonal antibody.
47. The method of statement 46, wherein the monoclonal antibody, the phage display antibody, or the polyclonal antibody is a rat, mouse or goat anti-Ig antibody.
48. The method of statement 45 wherein the bound substance is streptavidin.
49. The method of statement 25 wherein the enzymatically active unbound material is a phospholipase.
50. The method of statement 25 wherein the interfering substance(s) is a free-thiol compound.
51. The method of statement 25, wherein the substrate is selected from the group consisting of

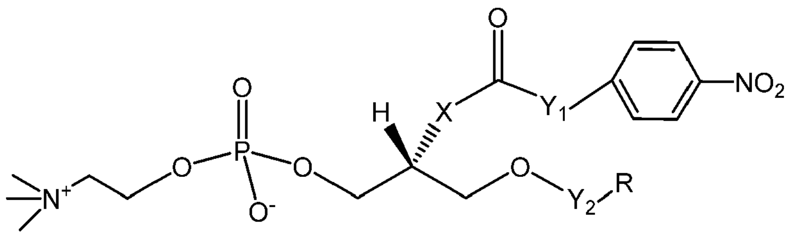

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

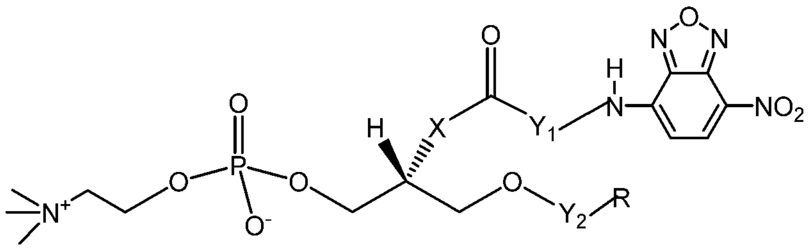

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

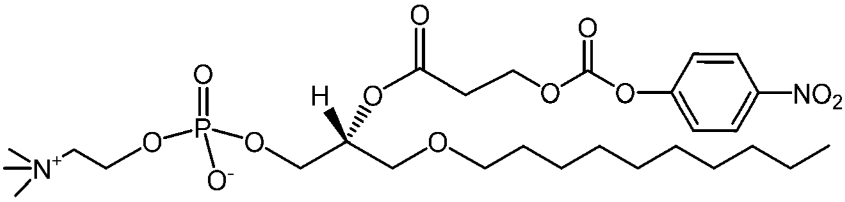

(c)

1-myristoyl-2-(4-nitrophenylsuccinyl) phosphatidylcholine (MNP);

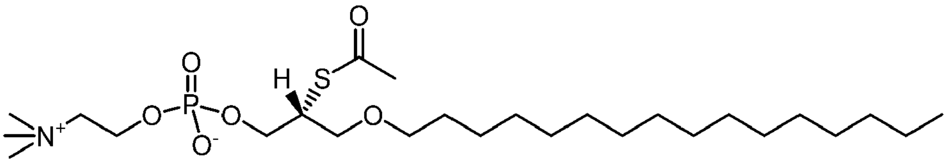

(d)

2-thio PAF; and

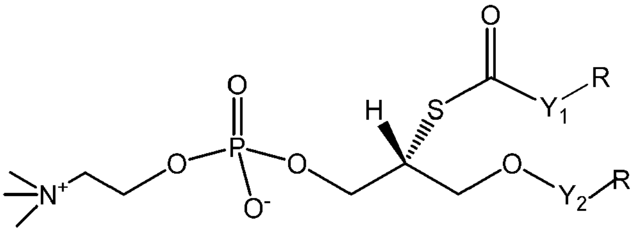

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

52. The method of statement 51 where in the substrate is an oxidized derivative of (a), (b), (c), (d) or (e).
53. The method of statement 25, wherein the detectable product has a radioactive, colorimetric, paramagnetic or fluorescent label.
54. The method of statement 25 wherein the detectable product is measured fluorimetrically, colorimetrically, paramagnetically or via radiation.
55. The method of statement 25 further comprising comparing the measured detectable product of step (e) to detectable product in a control comprising an enzymatically active Lp-PLA2 standard.
56. The method of statement 55 wherein the enzymatically active Lp-PLA2 standard is a recombinant Lp-PLA2 protein or a native Lp-PLA2 protein.
57. The method of statement 56 wherein the recombinant Lp-PLA2 protein is expressed in a baculovirus expression system or a mammalian expression system.
58. The method of statement 55 wherein a difference in detectable product in the sample compared to the standard is due to a difference in Lp-PLA2 activity in the sample compared to the standard.
59. A method for detecting vascular disease in an individual comprising utilizing the method of statement 58 to determine the individual's Lp-PLA2 activity in a sample wherein increased activity of Lp-PLA2 in the sample is indicative of vascular disease.
60. The method of statement 59 wherein the vascular disease is selected from the group consisting of coronary vascular disease (CVD), coronary heart disease (CHD), peripheral vascular disease, peripheral arterial disease,

stroke, congenital cardiovascular defects and congestive heart failure.

61. A method for selecting an individual for therapy to treat vascular disease comprising utilizing the method of statement 58 to determine the individual's Lp-PLA2 activity in a sample wherein increased activity of Lp-PLA2 in the sample is indicative of an individual who will benefit from therapy to treat vascular disease.

62. The method of statement 61 wherein the vascular disease is selected from the group consisting of coronary vascular disease (CVD), coronary heart disease (CHD), peripheral vascular disease, peripheral arterial disease, stroke, congenital cardiovascular defects and congestive heart failure.

63. The method of statement 61 wherein the therapy is selected from the group consisting of statins and Lp-PLA2 inhibitors.

64. A method for monitoring an individuals response to therapy to treat vascular disease comprising utilizing the method of statement 58 to determine the individual's Lp-PLA2 activity in a sample wherein decreased activity of Lp-PLA2 in the sample is indicative of an individual who is responding favorably to therapy to treat vascular disease.

65. The method of statement 64 wherein the vascular disease is selected from the group consisting of coronary vascular disease (CVD), coronary heart disease (CHD), peripheral vascular disease, peripheral arterial disease, stroke, congenital cardiovascular defects and congestive heart failure.

66. The method of statement 64 wherein the therapy is selected from the group consisting of statins and Lp-PLA2 inhibitors.

67. A kit for measuring enzymatically active Lipoprotein-associated Phospholipase A2 (Lp-PLA2) in a sample comprising a binder which specifically binds Lp-PLA2 and a substrate converted to a detectable product in the presence of Lp-PLA2.

68. The kit of statement 67 wherein the substrate is selected from the group consisting of

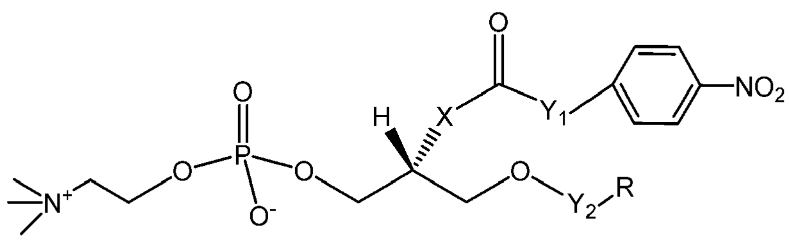

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

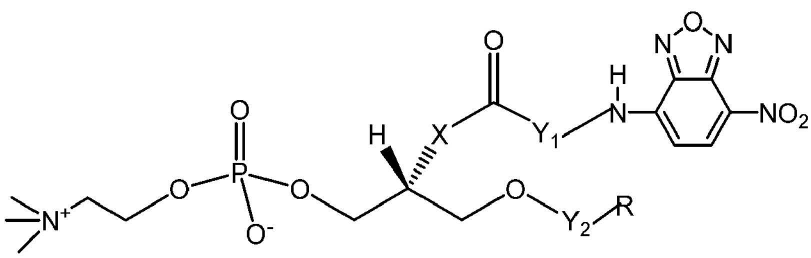

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$;

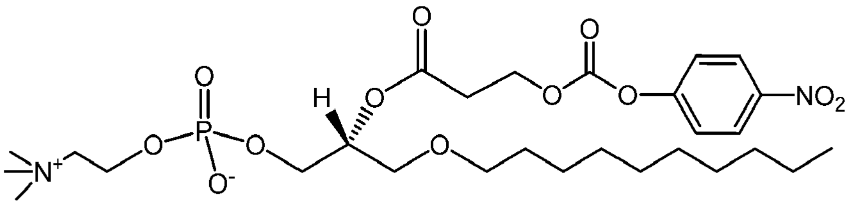

(c)

1-myristoyl-2-(4-nitrophenylsuccinyl) phosphatidylcholine (MNP);

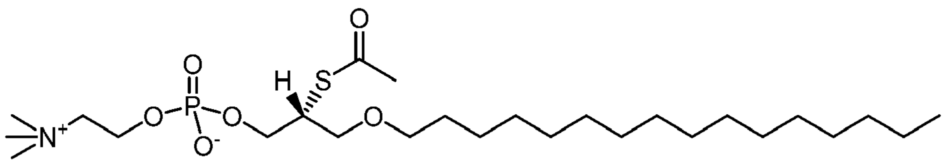

(d)

2-thio PAF; and

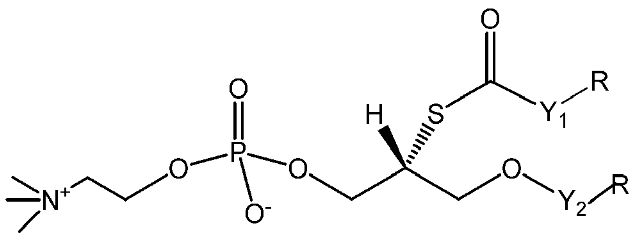

(e)

wherein

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$.

69. The kit of statement 68 wherein the substrate is an oxidized derivative of (a), (b), (c), (d) or (e).
70. The kit of statement 67 further comprising an enzymatically active Lp-PLA2 standard.
71. The kit of statement 70 wherein the enzymatically active Lp-PLA2 standard is a recombinant Lp-PLA2 protein or a native Lp-PLA2 protein.
72. The kit of statement 71 wherein the recombinant Lp-PLA2 protein is expressed in a baculovirus expression system or a mammalian expression system.
73. A method for measuring enzymatically active Lipoprotein-associated Phospholipase A2 (Lp-PLA2) in a sample comprising:

(a) incubating the sample with a compound which reduces active thiol(s) in the sample;
(b) contacting the incubated sample with a substrate converted to a free thiol product in the presence of enzymatically active Lp-PLA2; and
(c) measuring free thiol product indicative of enzymatically active Lp-PLA2 in the sample.

74. The method of statement 73, wherein the sample is a serum sample, a plasma sample or an EDTA treated plasma sample.
75. The method of statement 73 wherein the compound which reduces active thiol(s) in the sample is DTNB.
76. The method of statement 73 wherein the sample is incubated at room temperature.

77. The method of statement 73 wherein the sample is incubated at 37°C.
78. The method of statement 73 wherein the sample is incubated from about 2 to about 120 minutes.
79. The method of statement 73 wherein the sample is incubated from about 5 to about 30 minutes.
80. The method of statement 73 wherein the substrate is selected from the group consisting of

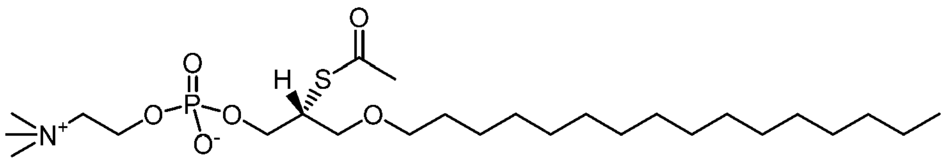
(a)

2-thio PAF; and

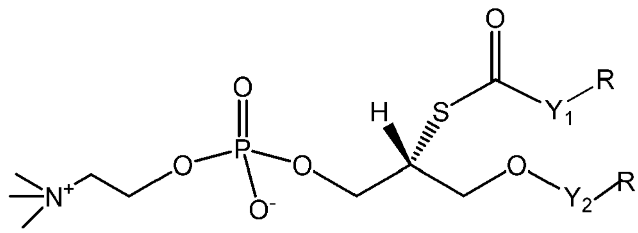
(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH{=}CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$.

81. The method of statement 80 where in the substrate is an oxidized derivative of (a) or (b).
82. The method of statement 73 further comprising comparing measured free thiol product of step (c) to free thiol product in a control comprising an enzymatically active Lp-PLA2 standard.
83. The method of statement 82 wherein the enzymatically active Lp-PLA2 standard is a recombinant Lp-PLA2 protein or a native Lp-PLA2 protein.
84. The method of statement 83 wherein the recombinant Lp-PLA2 protein is expressed in a baculovirus expression system or a mammalian expression system.
85. The method of statement 73 wherein the steps (a), (b), and (c) are conducted in a multi-well plate.
86. A kit for measuring enzymatically active Lipoprotein-associated Phospholipase A2 (Lp-PLA2) in a sample comprising a compound which reduces active thiol(s) and a substrate converted to a detectable product in the presence of Lp-PLA2.
87. The kit of statement 86 wherein the substrate is selected from the group consisting of

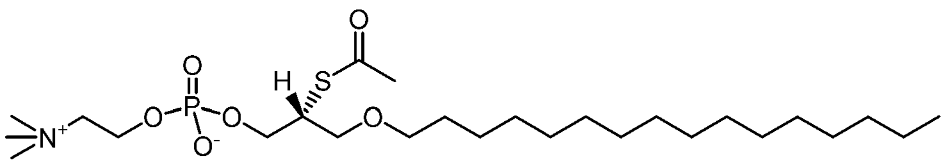
(a)

2-thio PAF; and

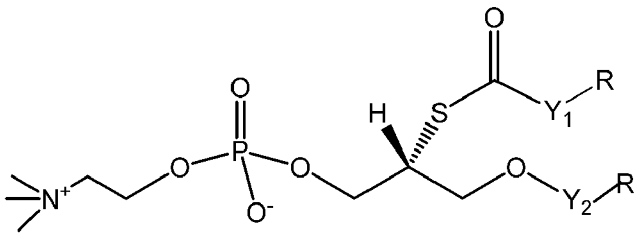

(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$.

88. The kit of statement 87 where in the substrate is an oxidized derivative of (a) or (b).
89. The kit of statement 86 further comprising an enzymatically active Lp-PLA2 standard.
90. The kit of statement 89 wherein the enzymatically active Lp-PLA2 standard is a recombinant Lp-PLA2 protein or a native Lp-PLA2 protein.
91. The kit of statement 90 wherein the recombinant Lp-PLA2 protein is expressed in a baculovirus expression system or a mammalian expression system.

## Claims

**1.** A method for measuring enzymatically active Lipoprotein-associated Phospholipase A2 (Lp-PLA2) in a sample, the method comprising:

(a) contacting an immobilized binder, which specifically binds Lp-PLA2, with the sample;
(b) washing the immobilized binder to remove an enzymatically active unbound material or an interfering substance(s);
(c) contacting the bound Lp-PLA2 with a substrate converted to a detectable product in the presence of Lp-PLA2; and
(d) measuring detectable product indicative of enzymatically active Lp-PLA2 in the sample.

**2.** A method for measuring enzymatically active Lipoprotein-associated Phospholipase A2 (Lp-PLA2) in a sample, the method comprising:

(a) contacting a binder, which specifically binds Lp-PLA2, with the sample to form a binder-Lp-PLA2 complex;
(b) immobilizing the binder-Lp-PLA2 complex;
(c) washing the immobilized binder-Lp-PLA2 complex to remove an enzymatically active unbound material or an interfering substance(s);
(d) contacting the immobilized bound Lp-PLA2 with a substrate converted to a detectable product in the presence of Lp-PLA2; and
(e) measuring detectable product indicative of enzymatically active Lp-PLA2 in the sample.

**3.** The method of claim 1 or 2, wherein the sample is a serum sample, a plasma sample or an EDTA treated plasma sample.

**4.** The method of claim 1 or 2, wherein the immobilized binder or binder is an antibody.

**5.** The method of claim 4, wherein the antibody is a monoclonal antibody, a phage display antibody, or a polyclonal antibody.

**6.** The method of claim 1 or 2, wherein the substrate is selected from the group consisting of

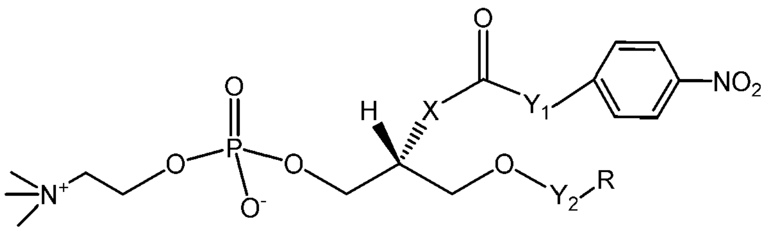

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1}$-$_{2}$ and $(CH_2)_{2-7}$; and
$Y_2$ is selected from the group consisting of CO and $CH_2$;

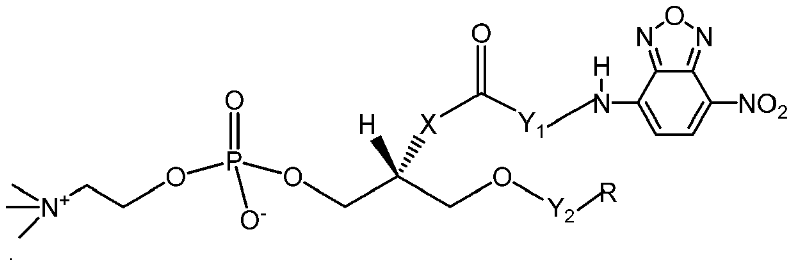

(b)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_2$-$_7$; and
$Y_2$ is selected from the group consisting of CO and $CH_2$;

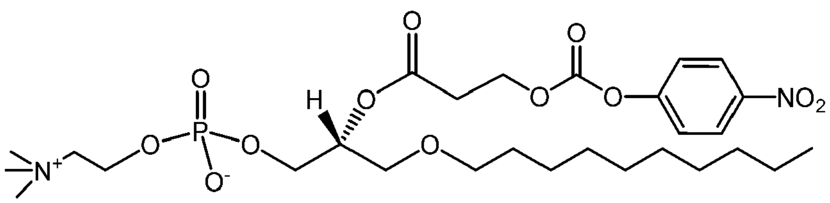

(c)

1-myristoyl-2-(4-nitrophenylsuccinyl) phosphatidylcholine (MNP);

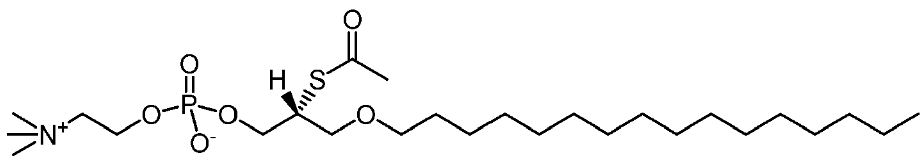

(d)

2-thio PAF; and

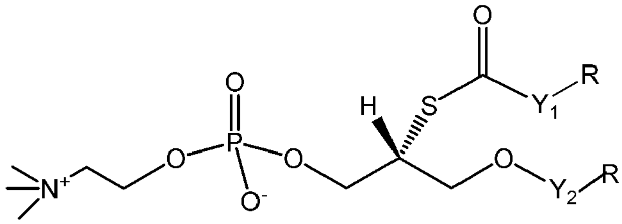

(e)

wherein

X is selected from the group consisting of O, S, and- O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$,
or an oxidized derivative of (a), (b), (c), (d) or (e).

**7.** The method of claim 1 or 2 which further comprises comparing the measured detectable product of step (d) or (e) respectively to detectable product in a control comprising an enzymatically active Lp-PLA2 standard.

**8.** The method of claim 2 wherein the binder-Lp-PLA2 complex is immobilized by binding to an immobilized compound, said immobilized compound comprising an antibody, protein or compound capable of binding the binder-Lp-PLA2 complex.

**9.** A method for detecting vascular disease in an individual comprising utilizing the method of any of claims 1 to 8 to determine the individual's Lp-PLA2 activity in a sample wherein increased activity of Lp-PLA2 in the sample is indicative of vascular disease.

**10.** A method for selecting an individual for therapy to treat vascular disease comprising utilizing the method of any of claims 1 to 8 to determine the individual's Lp-PLA2 activity in a sample wherein increased activity of Lp-PLA2 in the sample is indicative of an individual who will benefit from therapy to treat vascular disease.

**11.** A method for monitoring an individual's response to therapy to treat vascular disease comprising utilizing the method of any of claims 1 to 8 to determine the individual's Lp-PLA2 activity in a sample wherein decreased activity of Lp-PLA2 in the sample is indicative of an individual who is responding favorably to therapy to treat vascular disease.

**12.** A kit for measuring enzymatically active Lipoprotein-associated Phospholipase A2 (Lp-PLA2) in a sample comprising a binder which specifically binds Lp-PLA2 and a substrate converted to a detectable product in the presence of Lp-PLA2.

**13.** The kit of claim 12 wherein the substrate is selected from the group consisting of

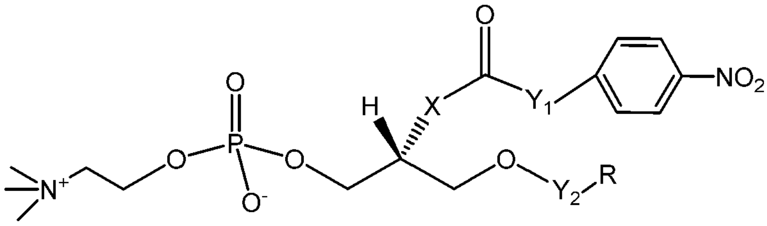

(a)

wherein,

X is selected from the group consisting of O, S, and -O(CO)-;
R is selected from the group consisting of $(CH_2)_4CH_3$, $(CH_2)_6CH_3$, $(CH_2)_8CH_3$, $(CH_2)_{10}CH_3$, $(CH_2)_{12}CH_3$,

$(CH_2)_{14}CH_3$, and $(CH_2)_7CH=CH(CH_2)_2CH_3$;

$Y_1$ is selected from the group consisting of $(CO)_{1-2}$ and $(CH_2)_{2-7}$; and

$Y_2$ is selected from the group consisting of CO and $CH_2$;

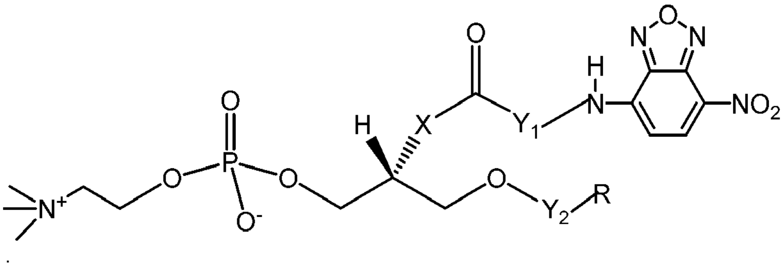

(b)

wherein,

X is selected from the group consisting of O, S, and -O(CO)-;

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;

$Y_1$ is selected from the group consisting of $(CO)_{1-2}$ and $(CH_2)_{2-7}$; and

$Y_2$ is selected from the group consisting of CO and $CH_2$;

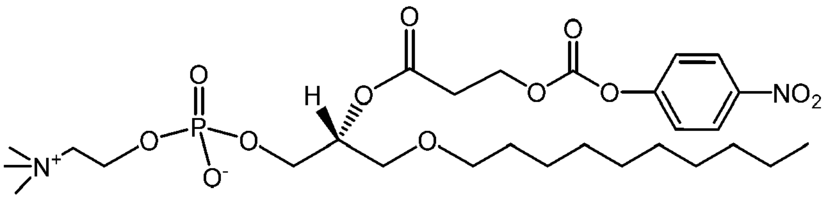

(c)

1-myristoyl-2-(4-nitrophenylsuccinyl) phosphatidylcholine (MNP);

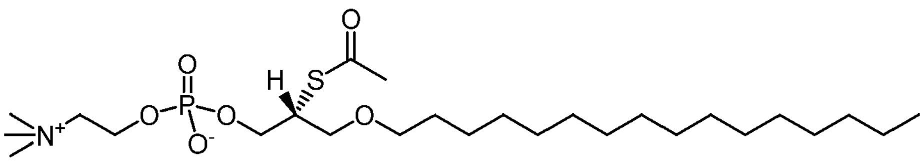

(d)

2-thio PAF; and

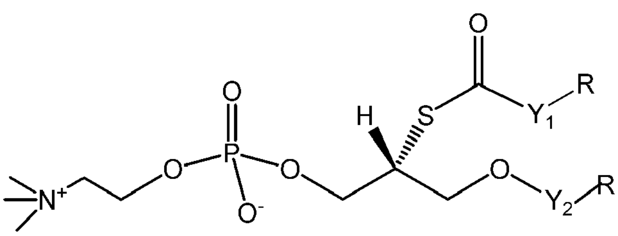

(e)

wherein

X is selected from the group consisting of O, S, and- O(CO)-;

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$, and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO or $CH_2$,
or an oxidized derivative of (a), (b), (c), (d) or (e).

**14.** A method for measuring enzymatically active Lipoprotein-associated Phospholipase A2 (Lp-PLA2) in a sample, the method comprising:

(a) incubating the sample with a compound which reduces active thiol(s) in the sample;
(b) contacting the incubated sample with a substrate converted to a free thiol product in the presence of enzymatically active Lp-PLA2; and
(c) measuring free thiol product indicative of enzymatically active Lp-PLA2 in the sample.

**15.** The method of claim 14, wherein the sample is a serum sample, a plasma sample or an EDTA treated plasma sample.

**16.** The method of claim 14 wherein the sample is incubated at room temperature or at 37°C.

**17.** The method of claim 14 wherein the sample is incubated from about 2 to about 120 minutes.

**18.** The method of claim 14 wherein the substrate is selected from the group consisting of

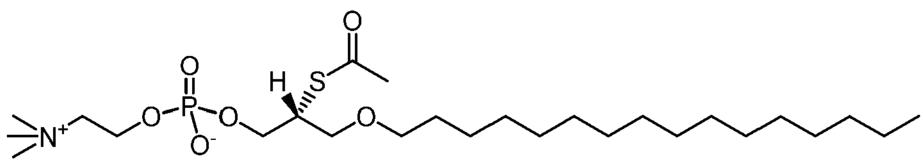
(a)

2-thio PAF; and

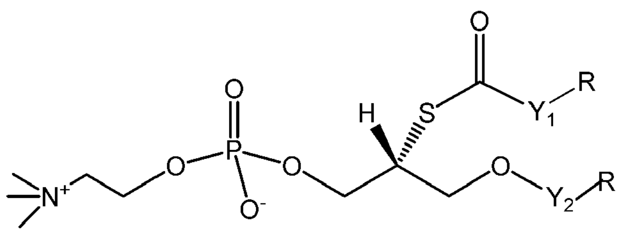
(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$,
or an oxidized derivative of (a) or (b).

**19.** The method of claim 14 further comprising comparing measured free thiol product of step (c) to free thiol product in a control comprising an enzymatically active Lp-PLA2 standard.

**20.** A kit for measuring enzymatically active Lipoprotein-associated Phospholipase A2 (Lp-PLA2) in a sample comprising a compound which reduces active thiol(s) and a substrate converted to a detectable product in the presence of Lp-PLA2.

**21.** The kit of claim 20 wherein the substrate is selected from the group consisting of

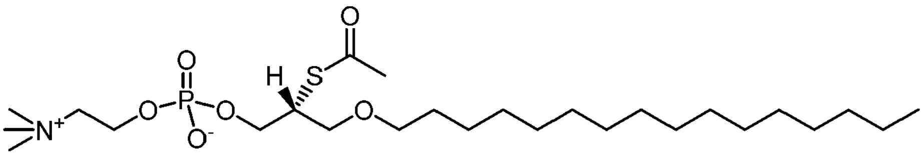

(a)

2-thio PAF; and

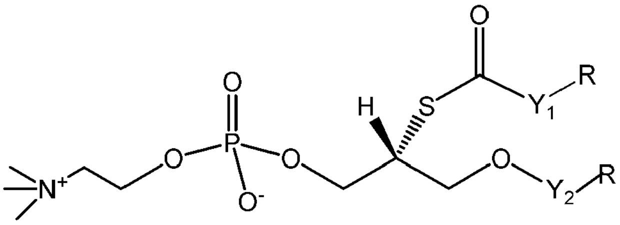

(b)

wherein,

R is selected from the group consisting of $(CH_2)_4CH_3$, $(CH_2)_6CH_3$, $(CH_2)_8CH_3$, $(CH_2)_{10}CH_3$, $(CH_2)_{12}CH_3$, $(CH_2)_{14}CH_3$ and $(CH_2)_7CH=CH(CH_2)_2CH_3$;
$Y_1$ is selected from the group consisting of $(CO)_{1-2}$ and $(CH_2)_{2-7}$; and
$Y_2$ is selected from the group consisting of CO and $CH_2$, or an oxidized derivative of (a) or (b).

# FIG. 1A and FIG. 1B
## Hybrid ImmunoCapture (HIC) Assay

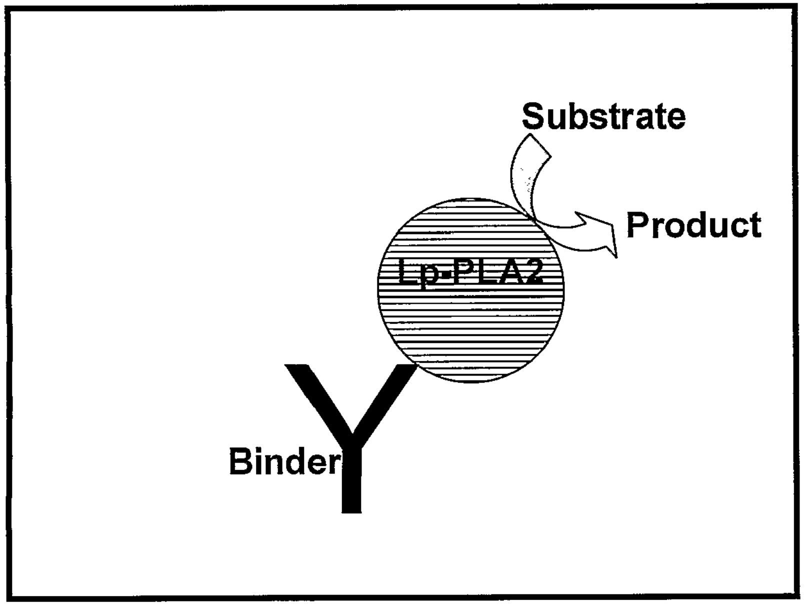


Fig. 1A

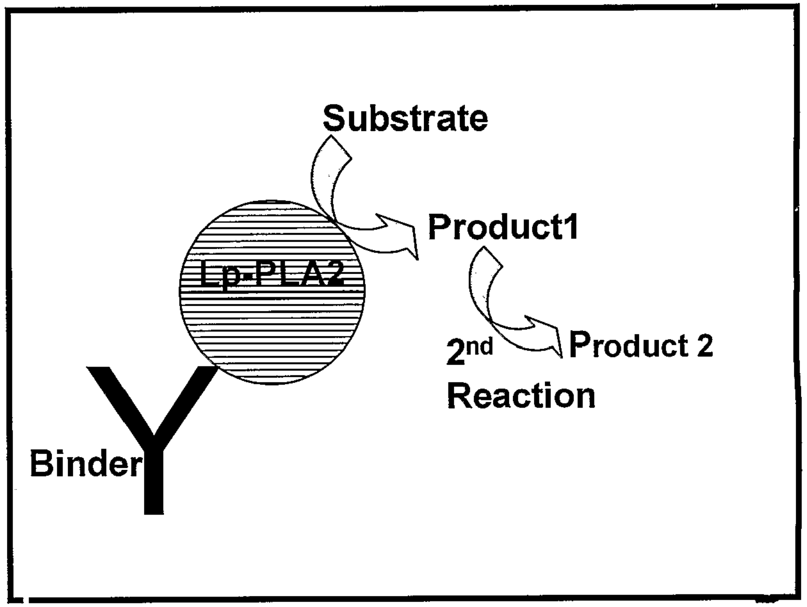


Fig. 1B

FIG. 2: Plasma Lp-PLA2 Activity in HIC-ThioPAF Assay (2c10 as capturing mAb)

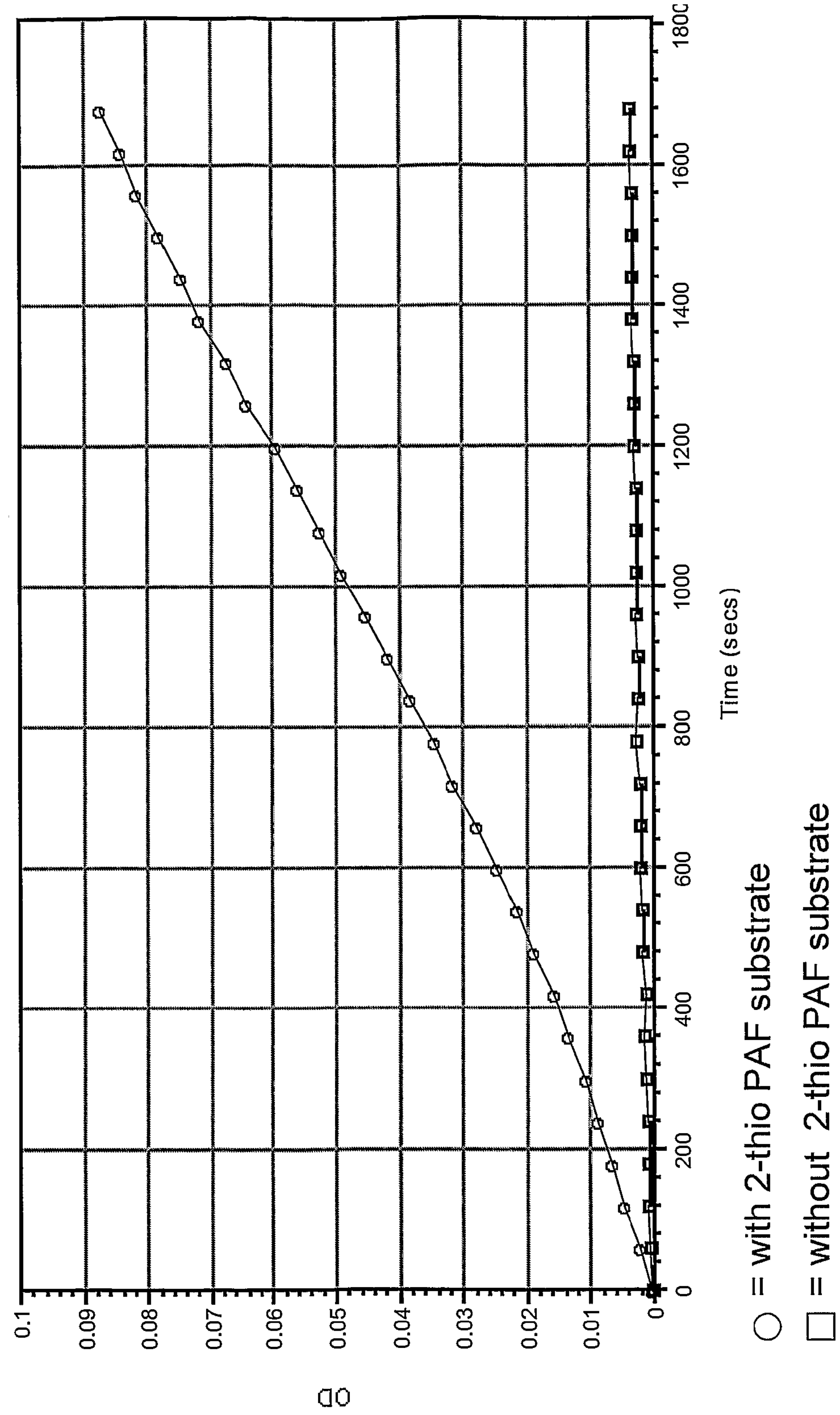

FIG. 3: Plasma Lp-PLA2 Activity in HIC-ThioPAF Assay (B200.1 as capturing mAb)

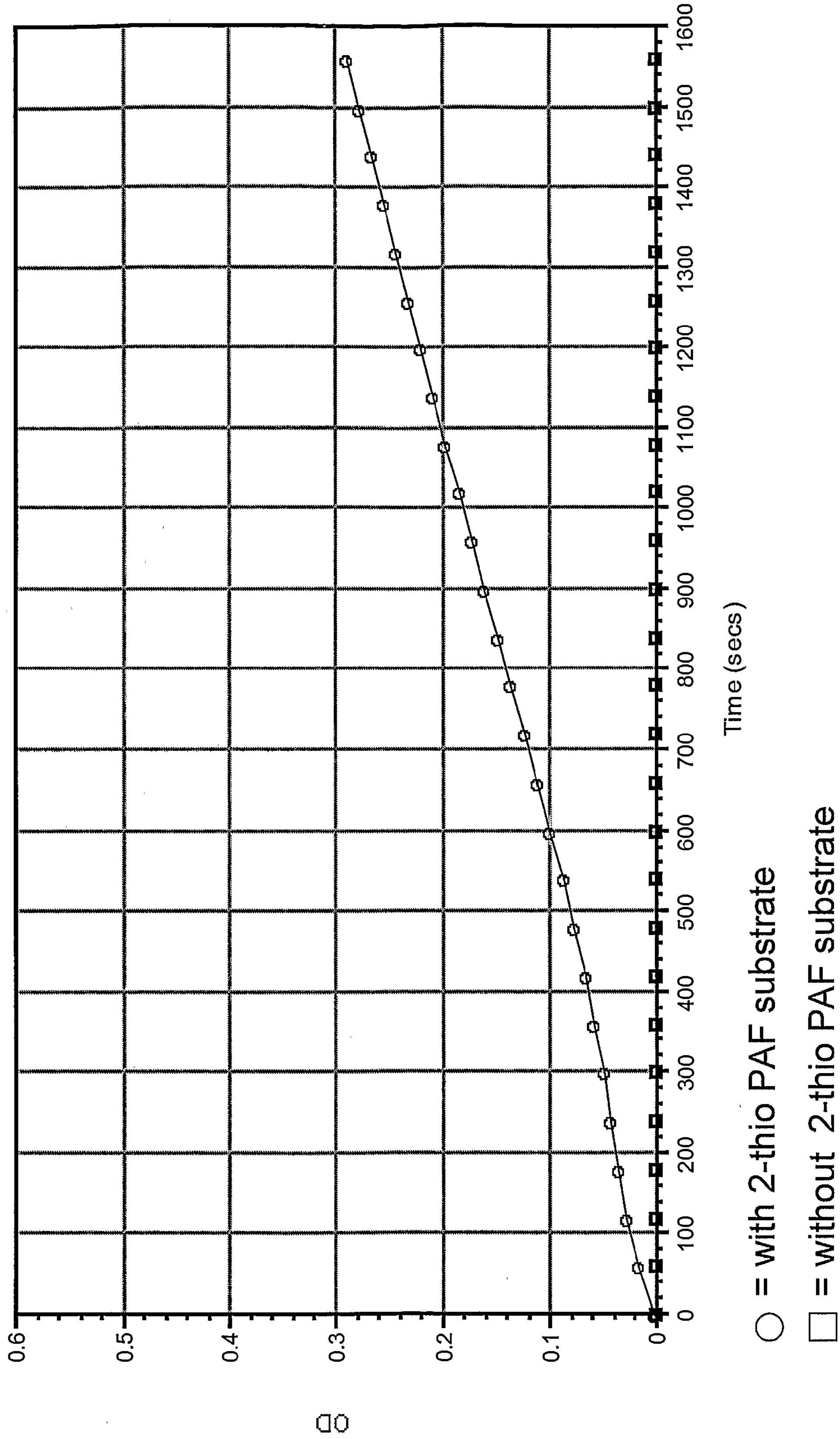

FIG. 4: Plasma Lp-PLA2 Activity in HIC-ThioPAF Assay (B501.1 as capturing mAb)

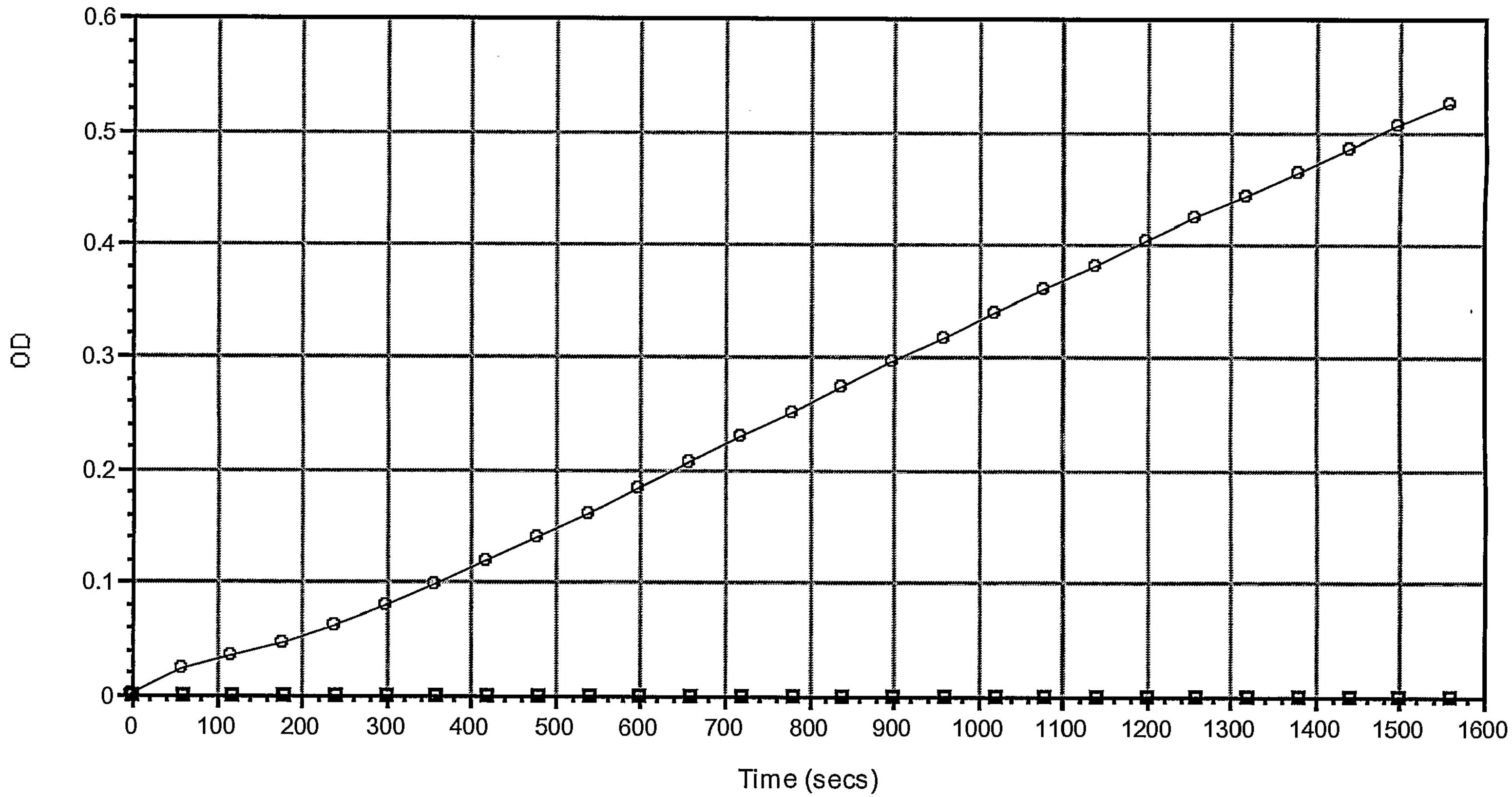


○ = with 2-thio PAF substrate

□ = without 2-thio PAF substrate

# FIG. 5: Plasma Lp-PLA2 Activity in HIC-MNP Assay (2c10 as capturing mAb)

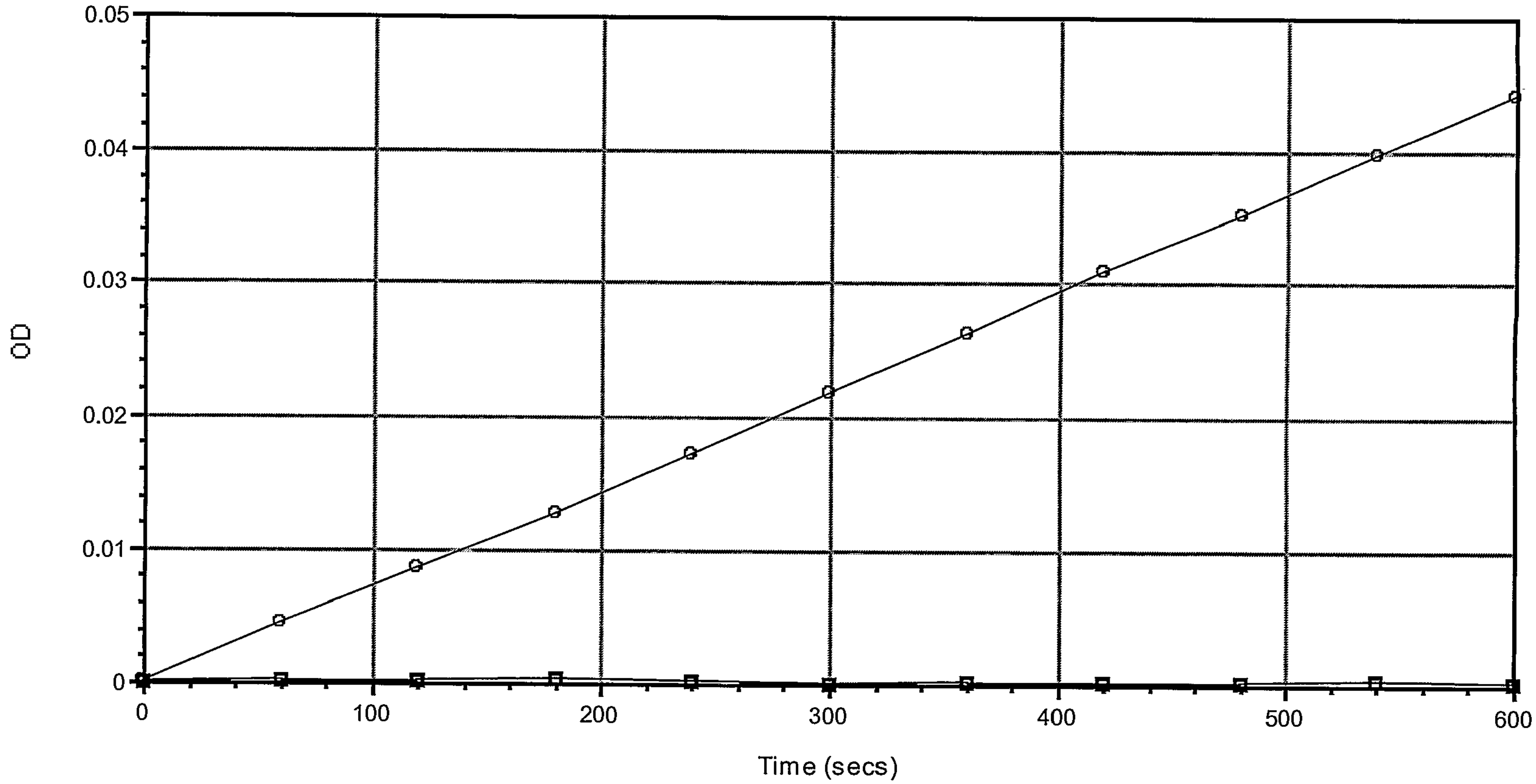


○ = with 1-myristoyl-2-(4-nitrophenylsuccinyl) phosphatidylcholine substrate (MNP)

□ = without 1-myristoyl-2-(4-nitrophenylsuccinyl) phosphatidylcholine substrate

FIG. 6: Plasma Lp-PLA2 Activity In Commercial ThioPAF Assay

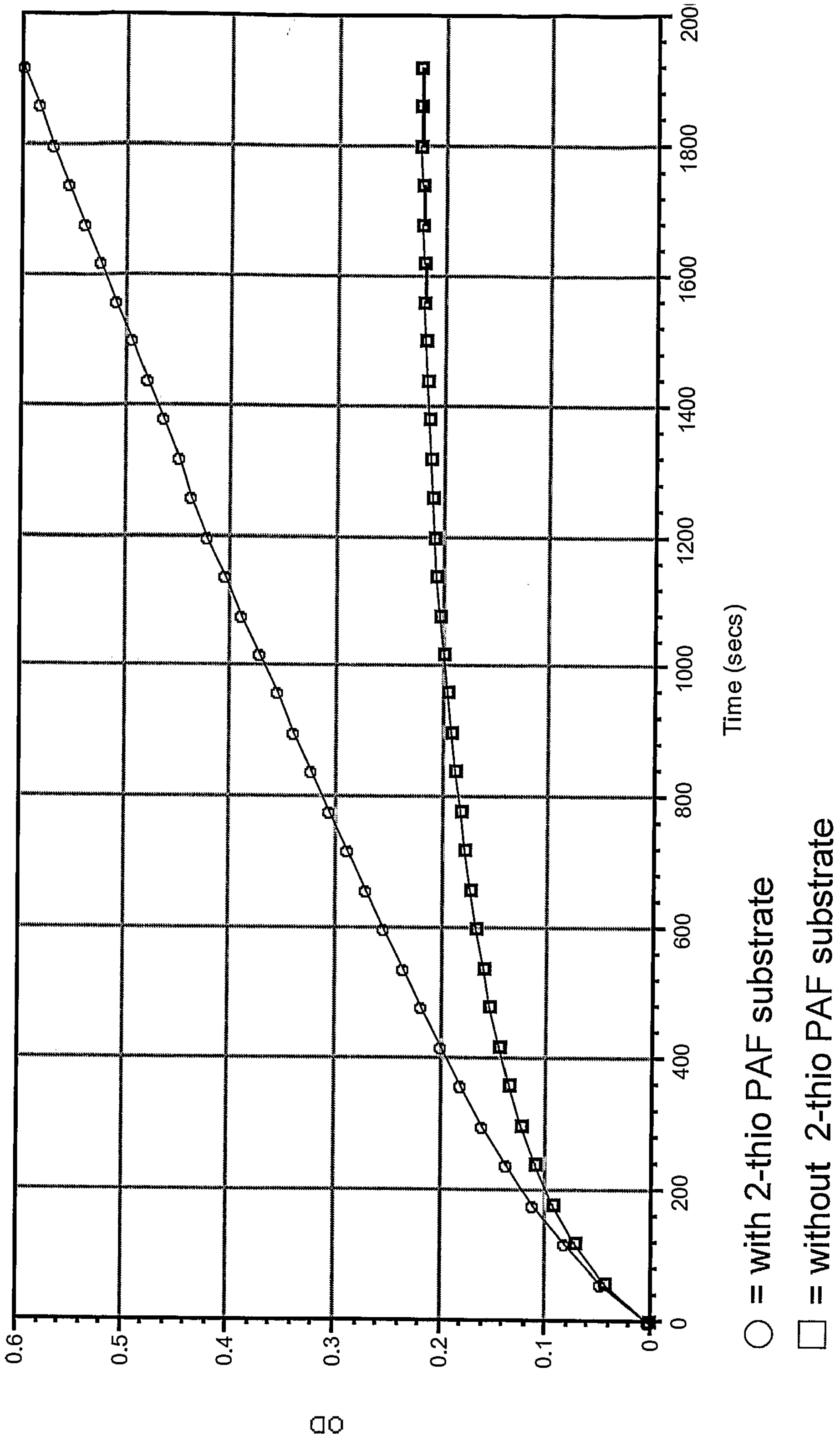

FIG. 7: Plasma sample background in Improved ThioPAF Assay, with DTNB but w/o substrate added

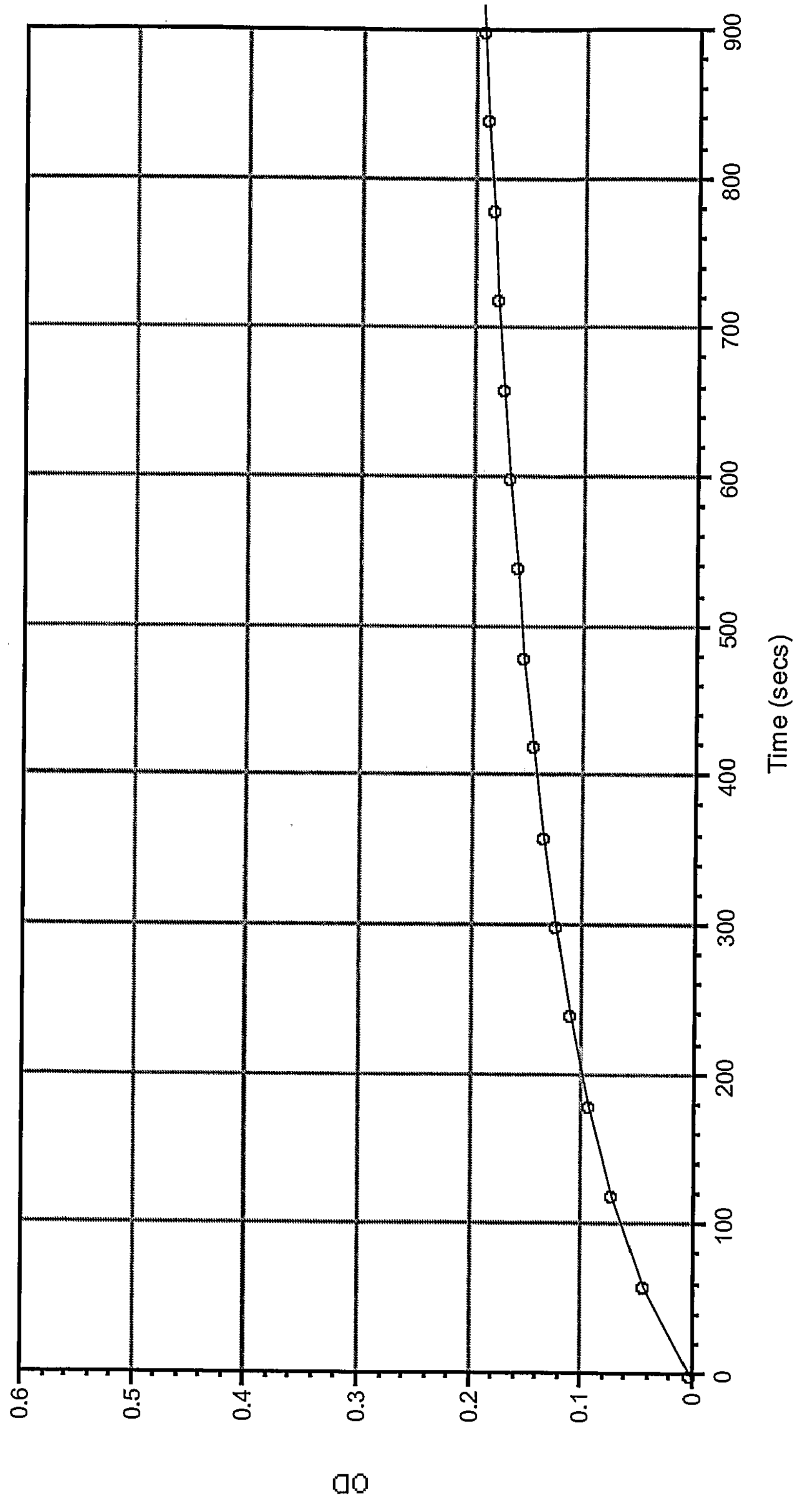


○ = without 2-thio PAF substrate; with DTNB added

FIG. 8: Plasma Lp-PLA2 Activity in Improved ThioPAF Assay

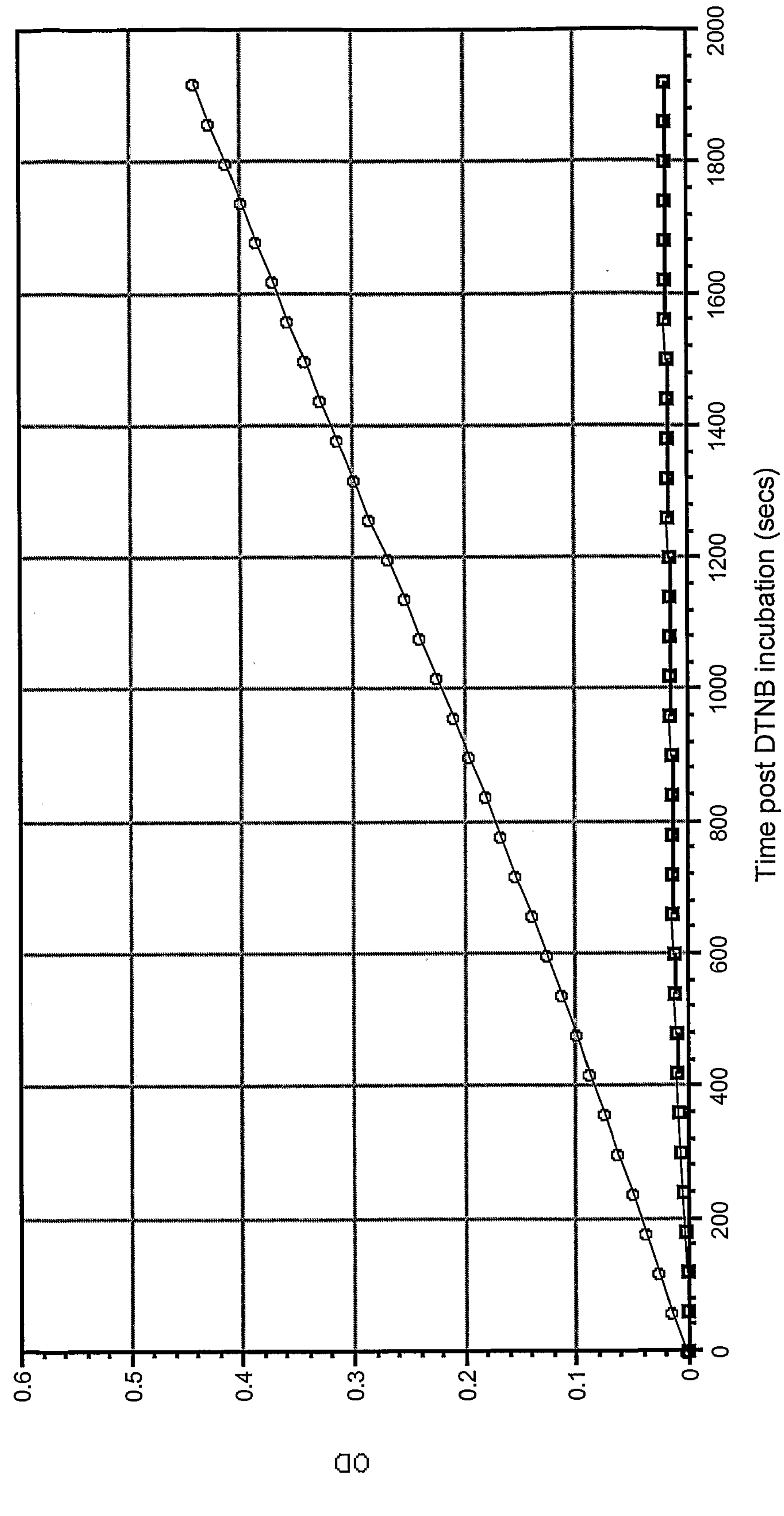


○ = with 2-thio PAF substrate

□ = without 2-thio PAF substrate

Europäisches Patentamt
European Patent Office
Office européen des brevets

# EUROPEAN SEARCH REPORT

Application Number
EP 10 18 5947

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 847 088 A (ICOS CORPORATION) 8 December 1998 (1998-12-08) * column 31 - column 32 * ----- | 1-21 | INV. G01N33/53 G01N33/573 C07K16/00 |
| A | WO 99/42830 A (DIADEXUS) 26 August 1999 (1999-08-26) * claims 1-15; example 1 * ----- | 1-21 | |
| A | BIELICKI J K ET AL: "Relative sensitivities of plasma lecithin:cholesterol acyltransferase, platelet-activating factor acetylhydrolase, and paraoxonase to in vitro gas-phase cigarette smoke exposure.", ATHEROSCLEROSIS MAR 2001, vol. 155, no. 1, March 2001 (2001-03), pages 71-78, XP002465271, ISSN: 0021-9150 * page 72, right-hand column, paragraph 5 - page 73, left-hand column, paragraph 1 * ----- | 1-21 | |
| | | | TECHNICAL FIELDS SEARCHED (IPC) |
| A | PETROVIC N ET AL: "A simple assay for a human serum phospholipase A2 that is associated with high-density lipoproteins", JOURNAL OF LIPID RESEARCH, BETHESDA, MD, US, vol. 42, no. 10, October 2001 (2001-10), pages 1706-1713, XP002377514, ISSN: 0022-2275 * page 1707 * ----- | 1-21 | G01N |

-/--

The present search report has been drawn up for all claims

1

| Place of search | Date of completion of the search | Examiner |
|---|---|---|
| Munich | 9 December 2010 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 18 5947

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DADA NISHA ET AL: "Lp-PLA2: an emerging biomarker of coronary heart disease", EXPERT REVIEW OF MOLECULAR DIAGNOSTICS, FUTURE DRUGS, LONDON, GB, vol. 2, no. 1, January 2002 (2002-01), pages 17-22, XP009086628, ISSN: 1473-7159 * abstract * ----- | 1-21 | |
| Y | KOSAKA T ET AL: "Spectrophotometric assay for serum platelet-activating factor acetylhydrolase activity", CLINICA CHIMICA ACTA, ELSEVIER BV, AMSTERDAM, NL, vol. 296, 1 June 2000 (2000-06-01), pages 151-161, XP002997443, ISSN: 0009-8981, DOI: DOI:10.1016/S0009-8981(00)00216-3 * the whole document * ----- | 1-21 | |
| Y | KUJIRAOKA T ET AL: "Altered distribution of plasma PAF-AH between HDLs and other lipoproteins in hyperlipidemia and diabetes mellitus", JOURNAL OF LIPID RESEARCH, AMERICAN SOCIETY FOR BIOCHEMISTRY AND MOLECULAR BIOLOGY, INC, US, vol. 44, 16 July 2003 (2003-07-16), pages 2006-2014, XP008092123, ISSN: 0022-2275, DOI: DOI:10.1194/JLR.D300021-JLR200 * the whole document * ----- | 1-21 | TECHNICAL FIELDS SEARCHED (IPC) |

The present search report has been drawn up for all claims

1

| Place of search | Date of completion of the search | Examiner |
|---|---|---|
| Munich | 9 December 2010 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 5947

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2010

| Patent document cited in search report | Publication date | Patent family member(s) | Publication date |
|---|---|---|---|
| US 5847088 A | 08-12-1998 | US 5605801 A | 25-02-1997 |
| | | US 5698403 A | 16-12-1997 |
| | | US 5656431 A | 12-08-1997 |
| WO 9942830 A | 26-08-1999 | CA 2320843 A1 | 26-08-1999 |
| | | EP 1057024 A1 | 06-12-2000 |
| | | JP 3524876 B2 | 10-05-2004 |
| | | JP 2002511564 T | 16-04-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 54158304 P
- WO 9500649 A1
- US 5981252 A
- US 5968818 A
- US 6177257 B
- WO 0024910 A1
- US 5532152 A
- US 5605801 A
- US 5641669 A
- US 5656431 A
- US 5698403 A
- US 5977308 A
- US 5847088 A
- WO 0032808 A, Azwell
- WO 03048172 A
- WO 2005001416 A
- WO 0029444 A
- US 5627052 A
- US 5807715 A
- US 6054297 A
- US 5821337 A
- US 5770196 A
- US 5766886 A
- US 5821123 A
- US 5869619 A
- US 6180377 A
- US 6013256 A
- US 5693761 A
- US 6180370 A
- US 5200084 A
- US 5186827 A
- US 5108933 A
- US 4925788 A
- WO 0047998 A
- US 6156504 A
- US 5501963 A
- WO 0142504 A


### Non-patent literature cited in the description


- **TEW et al.** *Arterioscler. Thromb. Vasc. Biol.,* 1996, vol. 16, 591-599
- **TJOELKER et al.** *Nature,* 1995, vol. 374 (6522), 549-53
- **CASLAKE et al.** *Atherosclerosis,* 2000, vol. 150 (2), 413-9
- **HAKKININ.** *Arterioscler Thromb Vasc Biol,* 1999, vol. 19 (12), 2909-17
- **PACKARD.** *N Engl J Med,* 2000, vol. 343 (16), 1148-55
- *Clin Chem Acta,* vol. 296, 151-161
- *JAMA,* 2001, vol. 285 (19), 2486-97
- **EATON.** *J Am Board Fam Pract,* 1998, vol. 11 (3), 180-6
- **LUSIS.** *Atherosclerosis. Nature,* 2000, vol. 407 (6801), 233-41
- **LINDAHL.** *N Engl J Med,* 2000, vol. 343 (16), 1139-47
- **RIDKER.** *N Engl J Med,* 2002, vol. 347 (20), 1557-65
- **BLAKE.** *J Intern Med,* 2002, vol. 252 (4), 283-94
- **GLASS.** *Cell,* 2001, vol. 104 (4), 503-16
- **WITZTUM.** *Lancet,* 1994, vol. 344 (8925), 793-5
- **DAVIE.** *Heart,* 2000, vol. 83, 361-366
- **LIBBY.** *Curr Opin Lipidol,* 1996, vol. 7 (5), 330-5
- **MACPHEE.** *Biochem J,* 1999, vol. 338, 479-87
- **MACPHEE.** *Curr Opin Pharmacol,* 2001, vol. 1 (2), 121-5
- **MACPHEE.** *Expert Opin Ther Targets,* 2002, vol. 6 (3), 309-14
- **BLAKE.** *J Am Coll Cardiol,* 2001, vol. 38 (5), 1302-6
- **BALLANTYNE.** *Circulation,* 2004, vol. 109 (7), 837-42
- **GRISSOM.** *Crit Care Med.,* 2003, vol. 31 (3), 770-5
- **YOON.** *Clin Genet.,* 2002, vol. 62 (2), 128-34
- **LTNNO.** *Surgery,* 2002, vol. 132 (1), 66-71
- **MCMANUS ; PINCKARD.** *Crit Rev Oral Biol Med.,* 2000, vol. II (2), 240-5 8
- **HENDERSON.** *J. Immunol.,* 2000, vol. 64 (6), 3360-7
- **KHOVIDHUNKIT.** *Metabolism,* 1999, vol. 48 (12), 1524-31
- **SATOH.** *Am J Respir Crit Care Med.,* 1999, vol. 159 (3), 974-9
- **TSELEPIS.** *Arthritis Rheum.,* 1999, vol. 42 (2), 373-83

- **TSUJI.** *ORL J Otorhinolaryngol Relat Spec.,* 1998, vol. 60 (1), 25-9
- **BELL.** *Biochem Biophys Res Commun.,* 1997, vol. 241 (3), 630-5 9
- **MUGURUMA.** *Adv Exp Med Biol.,* 1997, vol. 407, 3 79-82
- **FURUKAWA.** *Pediatr Res.,* 1993, vol. 34 (2), 237-41
- **CARPENTER.** *FEBS Lett.,* 2001, vol. 505 (3), 357-63
- **LEACH.** *Farmaco,* 2001, vol. 56 (1-2), 45-50
- **BOYD et al.** *Bioorg Med Chem Lett.,* 2000, vol. 10 (4), 395-8
- **PINTO.** *Bioorg Med Chem Lett.,* 2000, vol. 10 (17), 2015-7
- **THIRKETTLE et al.** *J Antibiot (Tokyo).,* 2000, vol. 53 (7), 664-9
- **BUSBY.** *J Antibiot (Tokyo).,* 2000, vol. 53 (7), 670-6
- **THIRKETTLE.** *J Antibiot (Tokyo,* 2000, vol. 53 (7), 733-5
- **BOYD et al.** *Bioorg Med Chem Lett,* 2000, vol. 10 (22), 2557-61
- **BOYD et al.** *Bioorg Med Chem Lett.,* 2001, vol. 11 (5), 701-4
- **BLOOMER.** *Bioorg Med Chem Lett.,* 2001, vol. 11 (14), 1925-9
- **BOYD et al.** *Bioorg Med Chem Lett.,* 2002, vol. 12 (1), 51-5
- **BLACKIE.** *Bioorg Med Chem Lett.,* 24 March 2003, vol. 13 (6), 1067-70
- **WINKLER.** *J Clin Endocrinol Metab.,* 2004, vol. 89 (3), 1153-1159
- **BLANKENBERG.** *J of Lipid Research,* 2003, vol. 44, 1381-1386
- **SAMBROOK et al.** Molecular Cloning: A Laboratory Manual. Cold Spring Harbor Laboratory Pres, 1989
- **SAMBROOK et al.** Molecular Cloning: A Laboratory Manual. Cold Spring Harbor Press, 2001
- **AUSUBEL et al.** Current Protocols in Molecular Biology. Greene Publishing Associates, 1992
- **AUSUBEL et al.** Short Protocols in Molecular Biology: A Compendium of Methods from Current Protocols in Molecular Biology. Wiley & Sons, 1999
- **HARLOW ; LANE.** Antibodies: A Laboratory Manual. Cold Spring Harbor Laboratory Press, 1990
- **HARLOW ; LANE.** Using Antibodies: A Laboratory Manual. Cold Spring Harbor Laboratory Press, 1999
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER et al.** *Proc. Natl. Acad. Sci. USA,* 1993, vol. 90, 6444-6448
- **POLJAK et al.** *Structure,* 1994, vol. 2, 1121-1123
- **TAM et al.** *Proc. Natl. Acad. Sci. USA,* 1988, vol. 85, 5409-5413
- **POSNETT et al.** *J. Biol. Chem.,* 1988, vol. 263, 1719-1725
- Using Antibodies: A Laboratory Manual. Cold Spring Harbor Laboratory, 1998
- Current Protocols in Immunology. John Wiley & Sons, Inc, 2001
- **ZOLA.** Monoclonal Antibodies: Preparation and Use of Monoclonal Antibodies and Engineered Antibody Derivatives (Basics: From Background to Bench). Springer Verlag, 2000
- **GROSS M, SPECK.** *J.Dtsch. Tierarztl. Wochenschr.,* 1996, vol. 103, 417-422
- **MOSS.** *Semin. Immunol.,* 1990, vol. 2, 317-327
- **VIKINGE et al.** *Biosens. Bioelectron.,* 1998, vol. 13, 1257-1262
- Basic Methods in Antibody Production and Characterization. CRC Press, 2000
- Monoclonal Antibody Protocols. Humana Press, 1995, vol. 45
- Antibody Production: Essential Techniques. John Wiley & Son Ltd, 1997
- **KENNEY.** Antibody Solution: An Antibody Methods Manual. Chapman & Hall, 1997
- **SIDHU.** *Curr. Opin. Biotechnol.,* 2000, vol. 11 (6), 610-6
- **GRIFFITHS et al.** *Curr. Opin. Biotechnol.,* 1998, vol. 9 (1), 102-8
- **HOOGENBOOM et al.** *Immunotechnology,* 1998, vol. 4 (1), 1-20
- **RADER et al.** *Current Opinion in Biotechnology,* 1997, vol. 8, 503-508
- **AUJAME et al.** *Human Antibodies,* 1997, vol. 8, 155-168
- **HOOGENBOOM.** *Trends in Biotechnol.,* 1997, vol. 15, 62-70
- **BARBAS et al.** *Trends in Biotechnol.,* 1996, vol. 14, 230-234
- **WINTER et al.** *Ann. Rev. Immunol.,* 1994, 433-455
- **TAKAHASHI et al.** *Biosci. Biotechnol. Biochem.,* 2000, vol. 64 (10), 2138-44
- **FREYRE et al.** *J. Biotechnol.,* 2000, vol. 76 (2-3), 1 57-63
- **FISCHER et al.** *Biotechnol. Appl. Biochem.,* 1999, vol. 30, 117-20
- **PENNELL et al.** *Res. Immunol.,* 1998, vol. 149 (6), 599-603
- **ELDIN et al.** *J. Immunol. Methods.,* 1997, vol. 201 (1), 67-75
- **FRENKEN et al.** *Res. Immunol.,* 1998, vol. 149 (6), 589-99
- **SHUSTA et al.** *Nature Biotechinol.,* 1998, vol. 16 (8), 773-7
- **LI et al.** *Protein Expr. Purif.,* 2001, vol. 21 (1), 121-8

- **AILOR et al.** *Biotechnol. Bioeng.,* 1998, vol. 58 (2-3), 196-203
- **HSU et al.** *Biotechnol. Prog.,* 1997, vol. 13 (1), 96-104
- **EDELMAN et al.** *Immunology,* 1997, vol. 91 (1), 13-9
- **NESBIT et al.** *J. Immunol. Methods,* 1992, vol. 151 (1-2), 201-8
- **GIDDINGS et al.** *Nature Biotechnol.,* 2000, vol. 18 (11), 1151-5
- **GAVILONDO et al.** *Biotechniques,* 2000, vol. 29 (1), 128-38
- **FISCHER et al.** *J. Biol. Regul. Homeost. Agents,* 2000, vol. 14 (2), 83-92
- **FISCHER et al.** *Biotechnol. Appl. Biochem.,* 1999, vol. 30, 113-6
- **FISCHER et al.** *Biol. Chem.,* 1999, vol. 380 (7-8), 825-39
- **RUSSELL.** *Curr. Top. Microbiol. Immunol.,* 1999, vol. 240, 119-38
- **MA et al.** *Plant Physiol.,* 1995, vol. 109 (2), 341-6
- **POLLOCK et al.** *J. Immunol Methods.,* 1999, vol. 231, 147-57
- **YOUNG et al.** *Res. Immunol.,* 1998, vol. 149, 609-10
- **LIMONTA et al.** *Immunotechnology,* 1995, vol. 1, 107-13
- **VERMA et al.** *J. Immunol. Methods,* 1998, vol. 216 (1-2), 165-81
- **MERK et al.** *J. Biochem. (Tokyo,* 1999, vol. 125 (2), 328-33
- **RYABOVA et al.** *Nature Biotechnol.,* 1997, vol. 15 (1), 79-84
- **POLLOCK et al.** *J. Immunol. Methods,* 1999, vol. 231 (1-2), 147-57
- **HUDSON.** *Curr. Opin. Biotechnol.,* 1998, vol. 9 (4), 395-402
- **MORRISON et al.** *Proc. Natl. Acad. Sci USA,* 1984, vol. 81 (21), 6851-5
- **SHARON et al.** *Nature,* 1984, vol. 309 (5966), 364-7
- **TAKEDA et al.** *Nature,* 1985, vol. 314 (6010), 452-4
- **RIECHMANN et al.** *Nature,* 1988, vol. 332 (6162), 323-7
- **CO et al.** *Nature,* 1991, vol. 351 (6326), 501-2
- **THORPE et al.** *Methods Enzymol.,* 1986, vol. 133, 331-53
- **KRICKA et al.** *J. Immunoassay,* 1996, vol. 17 (1), 67-83
- **LUNDQVIST et al.** *J. Biolumin. Chemilumin.,* 1995, vol. 10 (6), 353-9